# EUROPEAN PATENT SPECIFICATION

(11) **EP 4 267 226 B1**
(45) Date of publication and mention of the grant of the patent: **26.11.2025**
(21) Application number: 21908162.7
(22) Date of filing: 23.12.2021
(51) Int. Cl.: A61M 16/20, A61M 16/08, A61M 16/06

(54) **CONDUIT CONNECTOR WITH FLOW-REGULATING VENT FOR PATIENT INTERFACE**
LEITUNGSVERBINDER MIT DURCHFLUSSREGULIERENDER ENTLÜFTUNG FÜR EINE PATIENTENSCHNITTSTELLE
RACCORD DE CONDUIT AVEC ÉVENT DE RÉGULATION DE DÉBIT POUR INTERFACE PATIENT

(30) Priority: 23.12.2020 US 202063129913 P
(43) Date of publication of application: 01.11.2023
(73) Proprietor: ResMed Pty Ltd, Bella Vista, New South Wales 2153 (AU)
(72) Inventor: SANGADI, Nookarajesh Varma, Bella Vista, New South Wales 2153 (AU); BLANCH, Emily Elizabeth, Bella Vista, New South Wales 2153 (AU); DANTANARAYANA, Muditha Pradeep, Bella Vista, New South Wales 2153 (AU)
(74) Representative: Vossius & Partner Patentanwälte Rechtsanwälte mbB
(86) International application number: PCT/AU2021/051557
(87) International publication number: WO 2022/133550

(56) References cited:
- EP-A2- 2 601 994
- WO-A1-03/066146
- WO-A1-2006/074511
- WO-A2-2014/186584
- GB-A- 1 498 059
- US-A1- 2011 056 495
- US-A1- 2015 034 079
- US-A1- 2016 256 661
- US-A1- 2017 049 988
- US-A1- 2018 326 173
- US-A1- 2019 224 440
- US-A1- 2020 368 478

## Description

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

### 2 BACKGROUND OF THE TECHNOLOGY

### 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2012.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

### 2.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 2.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074513; WO 2010/135785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFTTM nasal pillows mask, SWIFTTM II nasal pillows mask, SWIFTTM LT nasal pillows mask, SWIFTTM FX nasal pillows mask and MIRAGE LIBERTYTM full-face mask. The following patent applications, assigned to ResMed Limited, describe examples of nasal pillows masks: International Patent Application WO2004/073778 (describing amongst other things aspects of the ResMed Limited SWIFTTM nasal pillows), US Patent Application 2009/0044808 (describing amongst other things aspects of the ResMed Limited SWIFTTM LT nasal pillows); International Patent Applications WO 2005/063328 and WO 2006/130903 (describing amongst other things aspects of the ResMed Limited MIRAGE LIBERTYTM full-face mask); International Patent Application WO 2009/052560 (describing amongst other things aspects of the ResMed Limited SWIFTTM FX nasal pillows).

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

### 2.2.3.5 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034665; International Patent Application Publication No. WO 2000/078381; US Patent No. 6,581,594; US Patent Application Publication No. US 2009/0050156; US Patent Application Publication No. 2009/0044808.

**Table of noise of prior masks (ISO 17510-2:2007, 10 cmH2O pressure at 1m)**

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed MirageTM (*) | nasal | 29.5 | 21.5 | 1998 |
| ResMed UltraMirageT M | nasal | 36 (3) | 28 (3) | 2000 |
| ResMed Mirage ActivaTM | nasal | 32 (3) | 24 (3) | 2002 |
| ResMed Mirage MicroTM | nasal | 30 (3) | 22 (3) | 2008 |
| ResMed MirageTM SoftGel | nasal | 29 (3) | 22 (3) | 2008 |
| ResMed MirageTM FX | nasal | 26 (3) | 18 (3) | 2010 |
| ResMed Mirage SwiftTM (*) | nasal pillows | 37 | 29 | 2004 |
| ResMed Mirage SwiftTM II | nasal pillows | 28 (3) | 20 (3) | 2005 |
| ResMed Mirage SwiftTM LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| (* one specimen only, measured using test method specified in ISO 3744 in CPAP mode at 10 cmH2O) | | | | |

Sound pressure values of a variety of objects are listed below

| Object | A-weighted sound pressure dB(A) | Notes |
|---|---|---|
| Vacuum cleaner: Nilfisk Walter Broadly Litter Hog: B+ Grade | 68 | ISO 3744 at 1m distance |
| Conversational speech | 60 | 1m distance |
| Average home | 50 | |
| Quiet library | 40 | |
| Quiet bedroom at night | 30 | |
| Background in TV studio | 20 | |

WO 2006/074511 A1 discloses a one-way valve which comprises a body portion defining a passage through which fluid is able to pass during use, a support member disposed in the passage, a seat member disposed in the passage and provided with at least one aperture, a shaft extending between the support member and the seat member and a membrane disposed on the shaft such that the membrane is rotatable relative to the shaft. The seat member cooperates with the membrane such that a first predetermined pressure differential across the membrane causes the membrane to cover the at least one aperture and thereby prevent fluid flow past the seat member, and such that a second predetermined pressure differential of opposite polarity to the first predetermined pressure differential causes the membrane to not cover the at least one aperture and thereby permit fluid flow past the seat member.

### 3 BRIEF SUMMARY OF THE TECHNOLOGY

The invention is set out in the appended claims. The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

Another aspect of the present technology is directed to a patient interface that may comprise: a plenum chamber; a seal-forming structure; and a positioning and stabilising structure. The patient interface may further comprise a vent structure. The patient interface may further be configured to leave the patient's mouth uncovered, or if the seal-forming structure is configured to seal around the patient's nose and mouth, the patient interface may be further configured to allow the patient to breath from ambient in the absence of a flow of pressurised air through the plenum chamber inlet port.

Another aspect of the present technology is directed to a conduit connector and vent assembly comprising: a conduit connector comprising: an inlet portion having a vent assembly receiving hole; and an outlet portion; and a vent assembly positioned in the vent assembly receiving hole and comprising: a base; a cover; and a flap positioned between the base and the cover and not connected to the flap or the cover so as to be freely movable between the base and the cover during use.

Another aspect of the present technology is directed to a conduit connector and vent assembly for connecting an air circuit to a patient interface to direct pressurized air into the patient interface from the air circuit, the conduit connector and vent assembly comprising: a conduit connector comprising: an inlet portion having an inlet end configured to be connected to the air circuit and having a vent assembly receiving hole that extends through the inlet portion in a radial direction; and an outlet portion having an outlet end configured to be connected to the patient interface; and a vent assembly positioned in the vent assembly receiving hole, constructed and arranged to allow for washout of exhaled gases to ambient, and comprising: a base positioned proximal to an interior of the conduit connector; a cover positioned distal to the interior of the conduit connector; and a flap positioned between the base and the cover and not connected to the flap or the cover so as to be freely movable between the base and the cover during use.

Another aspect of the present technology is directed to a conduit connector and vent assembly comprising: a conduit connector comprising: an inlet portion having a vent assembly receiving hole; and an outlet portion; and a vent assembly positioned in the vent assembly receiving hole and comprising: a base; a cover; and a flap positioned between the base and the cover, the flap being configured to move between the base and the cover during use, and the flap having an annulus shape.

Another aspect of the present technology is directed to a conduit connector and vent assembly for connecting an air circuit to a patient interface to direct pressurized air into the patient interface from the air circuit, the conduit connector and vent assembly comprising: a conduit connector comprising: an inlet portion having an inlet end configured to be connected to the air circuit and having a vent assembly receiving hole that extends through the inlet portion in a radial direction; and an outlet portion having an outlet end configured to be connected to the patient interface; and a vent assembly positioned in the vent assembly receiving hole, constructed and arranged to allow for washout of exhaled gases to ambient, and comprising: a base positioned proximal to an interior of the conduit connector; a cover positioned distal to the interior of the conduit connector; and a flap positioned between the base and the cover, the flap being configured to move between the base and the cover during use, and the flap having an annulus shape.

Another aspect of the present technology is directed to a conduit connector and vent assembly comprising: a conduit connector comprising: an inlet portion; and an outlet portion; and a vent assembly comprising: a base; a cover; and a flap positioned between the base and the cover, the flap being configured to move between the base and the cover during use, and the flap having an annulus shape, wherein the flap is oriented between the base and the cover such that pressurized air travels through the conduit connector in a direction approximately parallel to the flap's diameter.

Another aspect of the present technology is directed to a conduit connector and vent assembly for connecting an air circuit to a patient interface to direct pressurized air into the patient interface from the air circuit, the conduit connector and vent assembly comprising: a conduit connector comprising: an inlet portion having an inlet end configured to be connected to the air circuit; and an outlet portion having an outlet end configured to be connected to the patient interface; and a vent assembly constructed and arranged to allow for washout of exhaled gases to ambient, and comprising: a base positioned proximal to an interior of the conduit connector; a cover positioned distal to the interior of the conduit connector; and a flap positioned between the base and the cover, the flap being configured to move between the base and the cover during use, and the flap having an annulus shape, wherein the flap is oriented between the base and the cover such that pressurized air travels through the conduit connector in a direction approximately parallel to the flap's diameter.

Another aspect of the present technology is directed to a conduit connector and vent assembly comprising: a conduit connector comprising: an inlet portion having a vent assembly receiving hole; and an outlet portion; and a vent assembly positioned in the vent assembly receiving hole and comprising: a base positioned proximal to an interior of the conduit connector; a cover positioned distal to the interior of the conduit connector; and a flap positioned between the base and the cover, the flap being configured to move between the base and the cover during use, and the flap having a flap hole such that all pressurized air that travels through the flap hole exits to atmosphere through the cover.

Another aspect of the present technology is directed to a conduit connector and vent assembly for connecting an air circuit to a patient interface to direct pressurized air into the patient interface from the air circuit, the conduit connector and vent assembly comprising: a conduit connector comprising: an inlet portion having an inlet end configured to be connected to the air circuit and having a vent assembly receiving hole; and an outlet portion having an outlet end configured to be connected to the patient interface; and a vent assembly positioned in the vent assembly receiving hole, constructed and arranged to allow for washout of exhaled gases to ambient, and comprising: a base positioned proximal to an interior of the conduit connector; a cover positioned distal to the interior of the conduit connector; and a flap positioned between the base and the cover, the flap being configured to move between the base and the cover during use, and the flap having a flap hole such that all pressurized air that travels through the flap hole exits to atmosphere through the cover.

In examples of any of the aspects of the preceding paragraphs: (a) a radial protrusion may extend from the inlet portion within the vent assembly receiving hole, (b) the base may include a radial base notch configured to receive the radial protrusion when the base is positioned within the vent assembly receiving hole, (c) the cover may include a radial cover notch configured to receive the radial protrusion when the cover is positioned within the vent assembly receiving hole, (d) the base may include a post configured to contact the cover and maintain a gap between the base and the cover, the flap being positioned within the gap, when the vent assembly is positioned within the vent assembly receiving hole, (e) the base may include an axial protrusion and the cover includes an axial notch, the axial notch being configured to receive the axial protrusion to maintain the gap between the base and the cover when the vent assembly is positioned within the vent assembly receiving hole, (f) the base may include a plurality of spokes forming a plurality of base holes to allow exhaled gases to pass through the base, (g) the cover may include a plurality of cover holes to allow exhaled gases to pass through the cover to ambient, (h) the flap may be configured to rest on the base until sufficient pressure within the conduit connector causes the flap to lift off from the base, (h) the cover may include an annular portion and a central portion, the cover holes being positioned between the annular portion and the central portion, (i) the flap may be configured to completely block the cover holes when the flap contacts the annular portion and the central portion, (j) the flap may be configured to allow exhaled gases to pass through the cover to ambient when the flap contacts only one of the annular portion or the central portion, (k) the annular portion and the central portion may be offset relative to one another in an axial direction of the cover, (1) the flap may be constructed from a flexible material, (m) the flexible material may be silicone, (n) each of the base and the cover may be constructed from a rigid material, (o) the inlet end may include a bayonet connector such that when the air circuit is connected to the conduit connector, the air circuit and the conduit connector are not rotatable relative to one another, and/or (p) the conduit connector may be an elbow.

Another aspect of the present technology is directed to a patient interface comprising: a plenum chamber pressurisable to a therapeutic pressure of at least 4 cmH2O above ambient air pressure by a flow of air at the therapeutic pressure for breathing by a patient, the plenum chamber further comprising two plenum chamber connectors, each of the plenum chamber connectors being positioned on a corresponding lateral side of the plenum chamber and configured to receive the flow of air at the therapeutic pressure; a seal-forming structure connected to the plenum chamber, the seal-forming structure being constructed and arranged to seal with a region of the patient's face that at least partly surrounds an entrance to the patient's airways, the seal-forming structure having a hole therein to deliver the flow of air at the therapeutic pressure to at least the patient's nares during use, the seal-forming structure being constructed and arranged to maintain the therapeutic pressure within the plenum chamber throughout the patient's respiratory cycle in use; a positioning and stabilising structure configured to hold the seal-forming structure in a therapeutically effective position on the patient's head, the positioning and stabilising structure further comprising two conduits and a central section between the conduits, the central section having a conduit connector receiving hole, each of the conduits being configured to be positioned on a corresponding lateral side of the patient's head in use and being configured to be connect to a corresponding one of the plenum chamber connectors; and the conduit connector and vent assembly described in any of the aspects or examples of the preceding paragraphs connected to the central section at the conduit connector receiving hole, wherein the patient interface is configured to leave the patient's mouth uncovered, or the seal-forming structure is configured to seal around the patient's nose and mouth and the patient interface is configured to allow the patient to breath from ambient in the absence of the flow of air at the therapeutic pressure.

In examples of the patient interface aspect of the preceding paragraph, (a) the conduit connector and vent assembly may be rotatably and releasably connected to the central section and/or (b) the plenum chamber may comprise a passive vent configured to allow exhaled gases to pass to ambient independent of therapeutic pressure and throughout the patient's respiratory cycle.

Another aspect of the present technology is directed to a patient interface comprising: a plenum chamber pressurisable to a therapeutic pressure by a flow of air; a seal-forming structure connected to the plenum chamber, the seal-forming structure being constructed and arranged to seal with a region of the patient's face that at least partly surrounds an entrance to the patient's airways; a positioning and stabilising structure configured to hold the seal-forming structure in a therapeutically effective position on the patient's head; a first vent configured to allow exhaled gases to pass to ambient independent of therapeutic pressure and throughout the patient's respiratory cycle; and a second vent configured to reduce a vent flow of exhaled gases therethrough as the therapeutic pressure increases.

Another aspect of the present technology is directed to a patient interface comprising: a plenum chamber pressurisable to a therapeutic pressure of at least 4 cmH2O above ambient air pressure by a flow of air at the therapeutic pressure for breathing by a patient; a seal-forming structure connected to the plenum chamber, the seal-forming structure being constructed and arranged to seal with a region of the patient's face that at least partly surrounds an entrance to the patient's airways, the seal-forming structure having a hole therein to deliver the flow of air at the therapeutic pressure to at least the patient's nares during use, the seal-forming structure being constructed and arranged to maintain the therapeutic pressure within the plenum chamber throughout the patient's respiratory cycle in use; a positioning and stabilising structure configured to hold the seal-forming structure in a therapeutically effective position on the patient's head; a first vent configured to allow exhaled gases to pass to ambient independent of therapeutic pressure and throughout the patient's respiratory cycle; and a second vent configured to reduce a vent flow of exhaled gases therethrough as the therapeutic pressure increases, wherein the patient interface is configured to leave the patient's mouth uncovered, or the seal-forming structure is configured to seal around the patient's nose and mouth and the patient interface is configured to allow the patient to breath from ambient in the absence of the flow of air at the therapeutic pressure.

In examples of any of the aspects of the preceding paragraphs: (a) the plenum chamber may comprise two plenum chamber connectors, each of the plenum chamber connectors being positioned on a corresponding lateral side of the plenum chamber and configured to receive the flow of air at the therapeutic pressure, (b) the positioning and stabilising structure may comprise two conduits and a central section between the conduits, the central section having a conduit connector receiving hole, each of the conduits being configured to be positioned on a corresponding lateral side of the patient's head in use and being configured to be connect to a corresponding one of the plenum chamber connectors, (c) a conduit connector and vent assembly may be connected to the central section at the conduit connector receiving hole, (d) the plenum chamber may include the first vent. (e) the conduit connector and vent assembly includes the second vent, and/or (f) the conduit connector and vent assembly may comprises: a conduit connector comprising: an inlet portion having an inlet end configured to be connected to the air circuit and having a vent assembly receiving hole; and an outlet portion having an outlet end configured to be connected to the patient interface; and a vent assembly positioned in the vent assembly receiving hole, constructed and arranged to allow for washout of exhaled gases to ambient, and comprising: a base positioned proximal to an interior of the conduit connector; a cover positioned distal to the interior of the conduit connector; and a flap positioned between the base and the cover, the flap being configured to move between the base and the cover during use.

Another aspect of the present technology is directed to a conduit connector and vent assembly for connecting an air circuit to a patient interface to direct pressurized air into the patient interface from the air circuit, the conduit connector and vent assembly comprising: a body portion having an inlet end configured to be connected to the air circuit and an outlet end configured to be connected to the patient interface; one or more holes extending through the body portion at a position between the inlet end and the outlet end and configured to allow air flowing through the conduit connector and vent assembly to pass to atmosphere; and a flap connected to the body portion, the flap being structured to cover the one or more holes in a closed position so that air flowing through the conduit connector and vent assembly cannot pass to atmosphere, the flap being structured to not cover the one or more holes in an open position to allow air flowing through the conduit connector and vent assembly to pass to atmosphere, and the flap being freely movable between the closed position and the open position, wherein a bump is positioned between the body portion and the flap such that in the closed position of the flap the flap is flush against the body portion.

In examples of any of the aspects of the preceding paragraphs: (a) the bump may be positioned on the flap and may face the body portion, (b) the bump may be positioned on the body portion and may face the flap, (c) the flap may comprise a plurality of bumps, (d) the flap may comprise a hinge that allows the flap to be freely movable between the closed position and the open position, (e) the hinge may be formed by a reduced material thickness, (f) the flap may comprise a tab fixed to the body portion, (g) the body portion may include a tab receiving hole that receives the tab to fix the flap to the body portion, (g) the hinge may be positioned between the tab and the flap, (h) the flap, the hinge, and the tab may be constructed from single homogeneous piece of material, (i) the hinge may be constructed from a material that is more flexible than the flap and the tab, (j) the tab may be permanently connected to the body portion, (k) the tab may be overmolded to the body portion, (1) the hinge may be biased such that the flap extends into a flow path through the body portion in an undeformed state of the hinge, (m) the hinge may be biased such that the flap is not in the closed position in an undeformed state of the hinge, (n) the hinge may be biased such that the flap does not cover the one or more holes in an undeformed state of the hinge, (o) the flap may be positioned to be impacted by an incoming flow of air through the inlet end, (p) the one or more holes may be positioned on the body portion such that a longitudinal axis of the one or more holes is approximately perpendicular to a longitudinal axis of the inlet end, (q) the one or more holes may be positioned on the body portion such that a longitudinal axis of the one or more holes is approximately parallel to a longitudinal axis of the outlet end, (r) the conduit connector and vent assembly may be an elbow having a bend between the inlet end and the outlet end, (s) the conduit connector and vent assembly may comprise a swivel positioned at the inlet end and configured to removably connect to the air circuit, and/or (t) the body portion may be constructed from a relatively rigid material.

Another aspect of the present technology is directed to a patient interface comprising: a plenum chamber pressurisable to a therapeutic pressure of at least 4 cmH2O above ambient air pressure by a flow of air at the therapeutic pressure for breathing by a patient, the plenum chamber further comprising two plenum chamber connectors, each of the plenum chamber connectors being positioned on a corresponding lateral side of the plenum chamber and configured to receive the flow of air at the therapeutic pressure; a seal-forming structure connected to the plenum chamber, the seal-forming structure being constructed and arranged to seal with a region of the patient's face that at least partly surrounds an entrance to the patient's airways, the seal-forming structure having a hole therein to deliver the flow of air at the therapeutic pressure to at least the patient's nares during use, the seal-forming structure being constructed and arranged to maintain the therapeutic pressure within the plenum chamber throughout the patient's respiratory cycle in use; a positioning and stabilising structure configured to hold the seal-forming structure in a therapeutically effective position on the patient's head, the positioning and stabilising structure further comprising two conduits and a central section between the conduits, the central section having a conduit connector and vent assembly receiving hole, each of the conduits being configured to be positioned on a corresponding lateral side of the patient's head in use and being configured to connect to a corresponding one of the plenum chamber connectors; and the conduit connector and vent assembly described in any of the aspects or examples of the preceding paragraphs connected to the central section at the conduit connector receiving and vent assembly hole, wherein the patient interface is configured to leave the patient's mouth uncovered, or the seal-forming structure is configured to seal around the patient's nose and mouth and the patient interface is configured to allow the patient to breath from ambient in the absence of the flow of air at the therapeutic pressure.

In examples of any of the aspects of the preceding paragraph: (a) the conduit connector and vent assembly may be rotatably and releasably connected to the central section, and/or, (b) the plenum chamber may comprise a passive vent configured to allow exhaled gases to pass to ambient independent of therapeutic pressure and throughout the patient's respiratory cycle.

Another aspect of the present technology is directed to a conduit connector and vent assembly for connecting an air circuit to a patient interface to direct pressurized air into the patient interface from the air circuit, the conduit connector and vent assembly comprising: a body portion having an inlet end configured to be connected to the air circuit and an outlet end configured to be connected to the patient interface and the body portion including a tab receiving hole; a plurality of holes extending through the body portion at a position between the inlet end and the outlet end and configured to allow air flowing through the conduit connector and vent assembly to pass to atmosphere and the plurality of holes surrounding the tab receiving hole; and a flap having a tab connected to the body portion at the tab receiving hole, the flap being structured to cover the plurality of holes in a closed position so that air flowing through the conduit connector and vent assembly cannot pass to atmosphere, the flap being structured to not cover the plurality of holes in an open position to allow air flowing through the conduit connector and vent assembly to pass to atmosphere, and the flap being freely movable between the closed position and the open position.

In examples: (a) the flap and the tab may be constructed from single homogeneous piece of material, (b) the tab may be permanently connected to the body portion, (c) the tab may be overmolded to the body portion, (d) the flap may be biased from the tab such that the flap is not in the closed position in an undeformed state, (e) the flap may be biased from the tab such that the flap does not cover the plurality of holes in an undeformed state, (f) the plurality of holes may be positioned on the body portion such that a longitudinal axis of the plurality holes is approximately perpendicular to a longitudinal axis of the inlet end, (g) the plurality of holes may be positioned on the body portion such that a longitudinal axis of the plurality of holes is approximately parallel to a longitudinal axis of the outlet end, (h) the conduit connector and vent assembly may be an elbow having a bend between the inlet end and the outlet end, (i) the conduit connector and vent assembly may comprise a swivel positioned at the inlet end and configured to removably connect to the air circuit, (j) the body portion may be constructed from a relatively rigid material, (k) the plurality of holes may be arranged in a circle around the tab receiving hole, (1) the flap may have a circular shape, (m) the flap may have a radius such that an outer peripheral edge of the flap extends beyond the plurality of holes in an undeformed state, (n) the flap may have a radius such that an outer peripheral edge of the flap contacts the body portion radially outwardly of the plurality of holes when deformed into contact with the body portion, and/or (o) the flap may be constructed from a relatively flexible material.

Another aspect of the present technology is directed to a patient interface comprising: a plenum chamber pressurisable to a therapeutic pressure of at least 4 cmH2O above ambient air pressure by a flow of air at the therapeutic pressure for breathing by a patient, the plenum chamber further comprising two plenum chamber connectors, each of the plenum chamber connectors being positioned on a corresponding lateral side of the plenum chamber and configured to receive the flow of air at the therapeutic pressure; a seal-forming structure connected to the plenum chamber, the seal-forming structure being constructed and arranged to seal with a region of the patient's face that at least partly surrounds an entrance to the patient's airways, the seal-forming structure having a hole therein to deliver the flow of air at the therapeutic pressure to at least the patient's nares during use, the seal-forming structure being constructed and arranged to maintain the therapeutic pressure within the plenum chamber throughout the patient's respiratory cycle in use; a positioning and stabilising structure configured to hold the seal-forming structure in a therapeutically effective position on the patient's head, the positioning and stabilising structure further comprising two conduits and a central section between the conduits, the central section having a conduit connector and vent assembly receiving hole, each of the conduits being configured to be positioned on a corresponding lateral side of the patient's head in use and being configured to connect to a corresponding one of the plenum chamber connectors; and the conduit connector and vent assembly described in any of the aspects or examples of the preceding paragraphs connected to the central section at the conduit connector and vent assembly receiving hole, wherein the patient interface is configured to leave the patient's mouth uncovered, or the seal-forming structure is configured to seal around the patient's nose and mouth and the patient interface is configured to allow the patient to breath from ambient in the absence of the flow of air at the therapeutic pressure.

In examples of any of the aspects of the preceding paragraph: (a) the conduit connector and vent assembly may be rotatably and releasably connected to the central section, and/or, (b) the plenum chamber may comprise a passive vent configured to allow exhaled gases to pass to ambient independent of therapeutic pressure and throughout the patient's respiratory cycle.

Another aspect of the present technology is directed to a conduit connector and vent assembly for connecting an air circuit to a patient interface to direct pressurized air into the patient interface from the air circuit, the conduit connector and vent assembly comprising: a vent ring having a plurality of vent holes positioned radially on the vent ring and configured to allow air flowing through the conduit connector and vent assembly to pass to atmosphere; a connector ring connected to the vent ring, the connector ring having a cage extending radially inward therefrom, and the connector ring having at least one connector configured to releasably connect the conduit connector and vent assembly to a patient interface or to an intermediate tube; and a membrane positioned between the vent ring and the connector ring to control airflow through the vent holes to atmosphere in response to pressure or flow of pressurized air through the conduit connector and vent assembly.

In examples: (a) the connector ring may comprise two connectors positioned opposite one another, (b) the at least one connector may be flexibly joined to the connector ring, (c) the vent ring may be structured to be releasably connected to an air circuit, (d) an elbow may be connected to the vent ring, the elbow being structured to be releasably connected to an air circuit, (e) the cage may be permanently connected to the connector ring, (f) the vent ring may have a vent ring annular surface and the connector ring may have a connector ring annular surface that faces the vent ring annular surface, the membrane being positioned between the vent ring annular surface and the connector ring annular surface, and/or (g) the cage may be positioned on the connector ring such that the membrane cannot contact the cage.

Another aspect of the present technology is directed to a patient interface comprising: a plenum chamber pressurisable to a therapeutic pressure of at least 4 cmH2O above ambient air pressure by a flow of air at the therapeutic pressure for breathing by a patient, the plenum chamber further comprising a connection port configured to receive the flow of air at the therapeutic pressure; a seal-forming structure connected to the plenum chamber, the seal-forming structure being constructed and arranged to seal with a region of the patient's face that at least partly surrounds an entrance to the patient's airways, the seal-forming structure having a hole therein to deliver the flow of air at the therapeutic pressure to at least the patient's nares during use, the seal-forming structure being constructed and arranged to maintain the therapeutic pressure within the plenum chamber throughout the patient's respiratory cycle in use; a positioning and stabilising structure comprising at least one tie configured to hold the seal-forming structure in a therapeutically effective position on the patient's head; and the conduit connector and vent assembly described in any of the aspects or examples of the preceding paragraphs, wherein the patient interface is configured to leave the patient's mouth uncovered, or the seal-forming structure is configured to seal around the patient's nose and mouth and the patient interface is configured to allow the patient to breath from ambient in the absence of the flow of air at the therapeutic pressure.

In examples of any of the aspects of the preceding paragraph: (a) an intermediate conduit may be releasably connected at a first end to the connector ring by the at least one connector and releasably connected to the plenum chamber at a second end and/or (b) the connector ring of the conduit connector and vent assembly may be releasably connected to the plenum chamber at the connection port by the at least one connector.

Another aspect of the present technology is directed to a vent assembly for discharging air to atmosphere from a patient interface, the vent assembly comprising: a vent body having a first plurality of vent holes and a second plurality of vent holes and having a first side and a second side; a base connected to the vent body on the first side of the vent body; a membrane positioned between the vent body and the base; a cap connected to the vent body on the second side of the vent body such that at least one vent passage is formed between the cap and the vent body to allow air to pass through the vent body to atmosphere; and a diffuser member constructed from a porous material and positioned between the cap and the vent body; wherein the membrane is configured to control airflow through the first plurality of vent holes to atmosphere in response to pressure or flow of pressurized air through the vent assembly, and wherein the second plurality of vent holes are not covered by the membrane.

In examples: (a) the first plurality of vent holes may be positioned radially on the vent body, (b) the second plurality of vent holes may be positioned radially on the vent body, (c) the second plurality of vent holes may be positioned radially outward of the first plurality of vent holes, (d) the membrane may be constructed from a flexible material, (e) the flexible material may be silicone, (f) each of the vent body, the base, and the cap may be constructed from a rigid material, (g) the vent body may include a ring-shaped protrusion extending from the first side of the vent body and between the first plurality of vent holes and the second plurality of vent holes, (h) the membrane may be positioned inside of the ring-shaped protrusion, (i) a diameter of the membrane may be less than an inner diameter of the ring-shaped protrusion to allow air to pass between an outer periphery of the membrane and an inner periphery of the ring-shaped protrusion, (j) a hole may be formed through the membrane to allow air to pass through the membrane, (k) the first plurality of vent holes and the second plurality of vent holes may be positioned on the vent body such that air that flows through either or both of the first plurality of vent holes and the second plurality of vent holes reaches the at least one vent passage before being discharged to atmosphere, (1) the first plurality of vent holes and the second plurality of vent holes may be positioned on the vent body such that air that flows through either or both of the first plurality of vent holes and the second plurality of vent holes is directed at the diffuser member, (m) the first plurality of vent holes and the second plurality of vent holes may be oriented on the vent body such that longitudinal axes through the first plurality of vent holes and the second plurality of vent holes extend to the diffuser member, (n) the diffuser member may be shaped and dimensioned so as not to extend completely across the at least one passage, (o) the diffuser member may be shaped and dimensioned to allow air passing to atmosphere through the at least one vent passage to bypass the diffuser member, (p) the vent body may comprise a groove configured to connect the vent body to a plenum chamber of a patient interface, and/or (q) the vent body may comprise a diffuser member spacer to support the diffuser member against the cap such that a bypass passage for airflow is formed between the vent body and the diffuser member.

Another aspect of the present technology is directed to a patient interface comprising: a plenum chamber pressurisable to a therapeutic pressure of at least 4 cmH2O above ambient air pressure by a flow of air at the therapeutic pressure for breathing by a patient, the plenum chamber further comprising a connection port configured to receive the flow of air at the therapeutic pressure; a seal-forming structure connected to the plenum chamber, the seal-forming structure being constructed and arranged to seal with a region of the patient's face that at least partly surrounds an entrance to the patient's airways, the seal-forming structure having a hole therein to deliver the flow of air at the therapeutic pressure to at least the patient's nares during use, the seal-forming structure being constructed and arranged to maintain the therapeutic pressure within the plenum chamber throughout the patient's respiratory cycle in use; a positioning and stabilising structure comprising at least one tie configured to hold the seal-forming structure in a therapeutically effective position on the patient's head; and the vent assembly described in any of the aspects or examples of the preceding paragraphs connected to the plenum chamber to allow air from within the plenum chamber to pass to atmosphere, wherein the patient interface is configured to leave the patient's mouth uncovered, or the seal-forming structure is configured to seal around the patient's nose and mouth and the patient interface is configured to allow the patient to breath from ambient in the absence of the flow of air at the therapeutic pressure.

In examples: (a) the vent assembly may be removably attached to the plenum chamber.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 4 BRIEF DESCRIPTION OF THE DRAWINGS

The present technology is illustrated by way of example, and not by way of limitation, in the figures of the accompanying drawings, in which like reference numerals refer to similar elements including:

### 4.1 RESPIRATORY THERAPY SYSTEMS

Fig. 1 shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.
Fig. 2 shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.
Fig. 3 shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

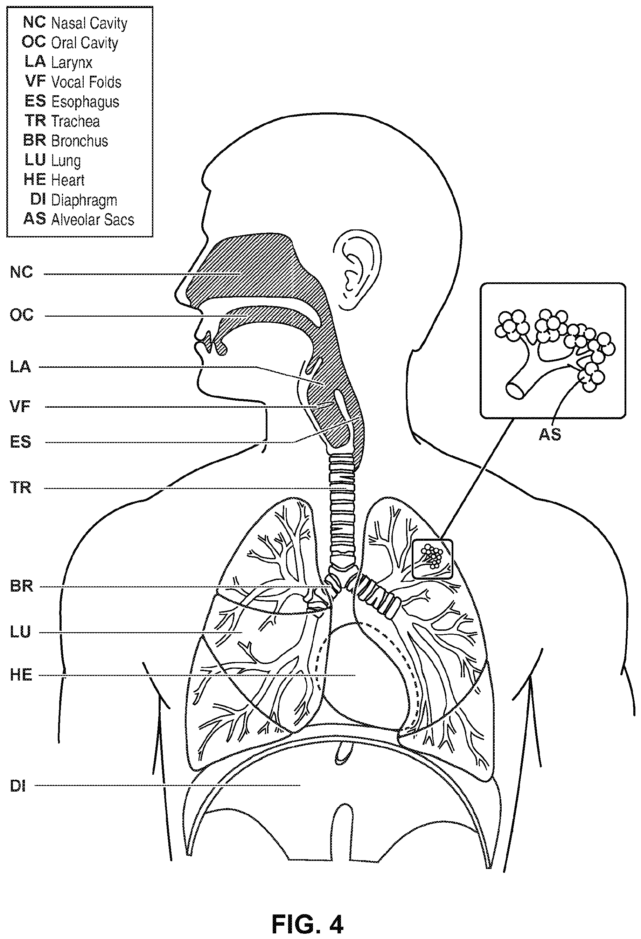

Fig. 4 shows an overview of a human respiratory system including the nasal and oral cavities, the larynx, vocal folds, oesophagus, trachea, bronchus, lung, alveolar sacs, heart and diaphragm.

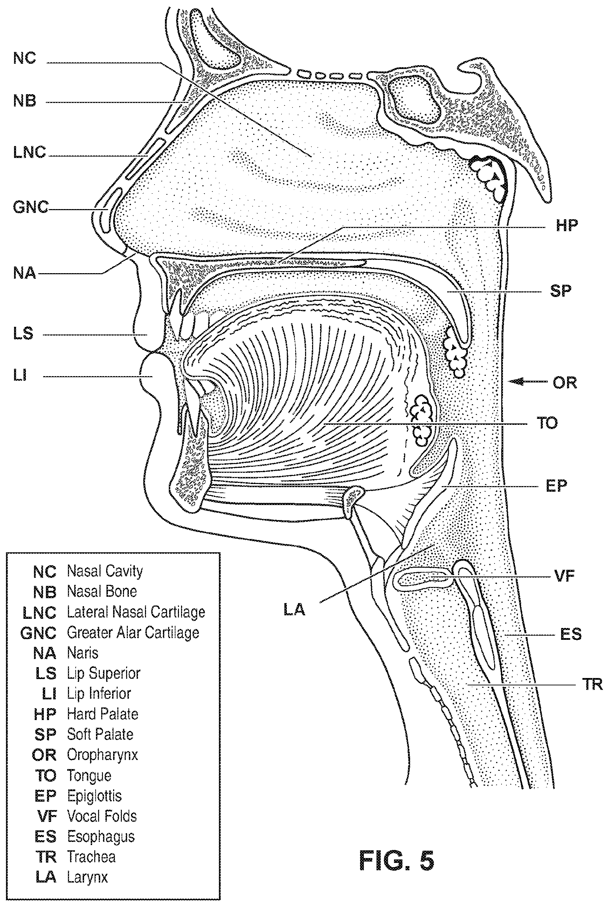

Fig. 5 shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

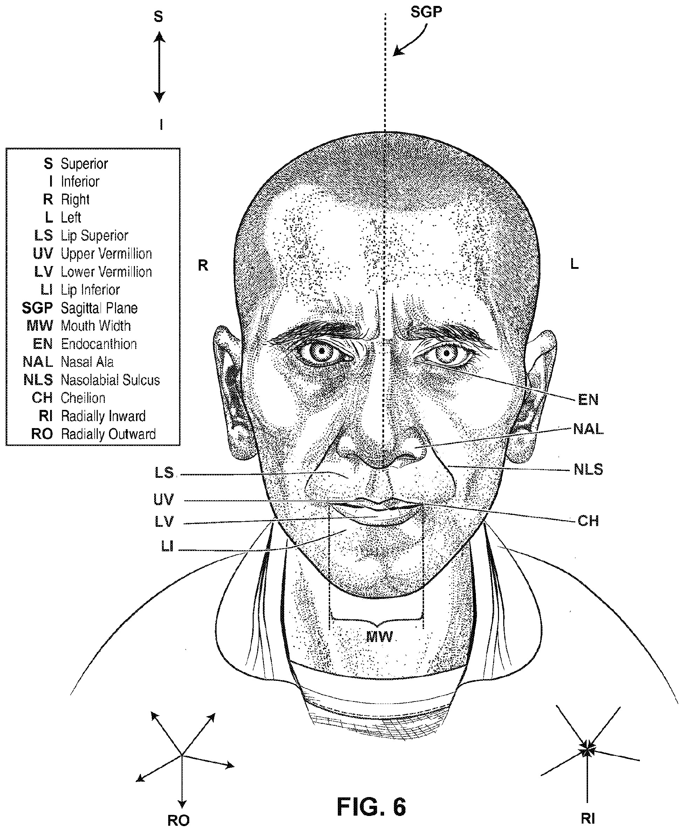

Fig. 6 is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

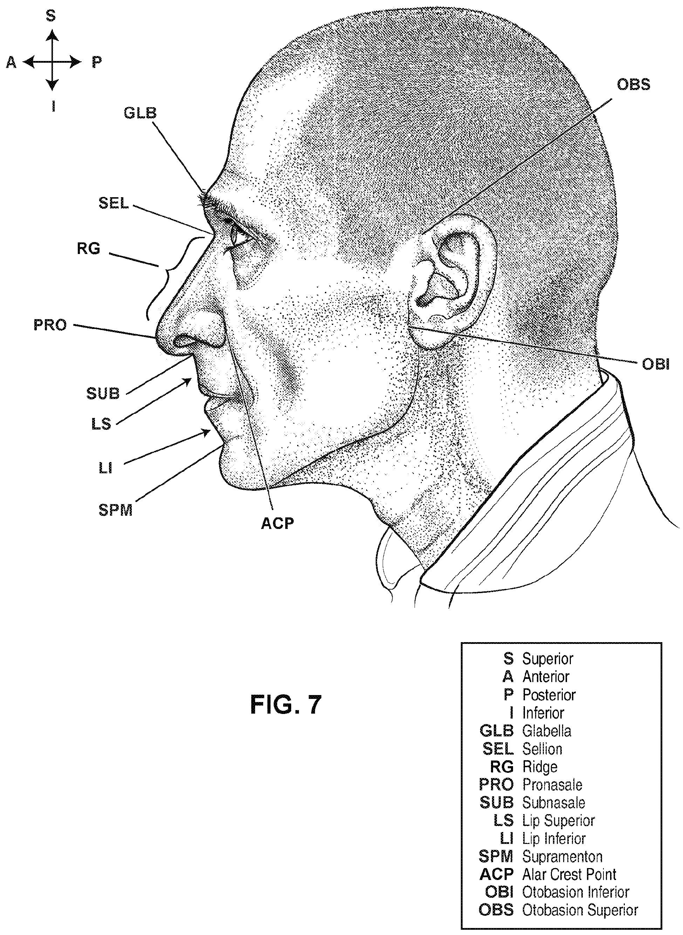

Fig. 7 is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

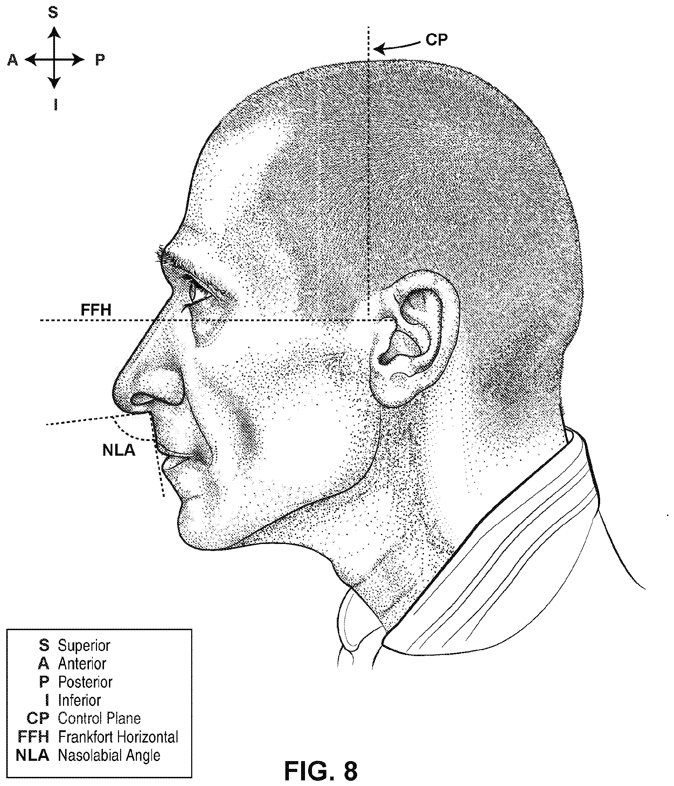

Fig. 8 is a further side view of a head. The approximate locations of the Frankfort horizontal and nasolabial angle are indicated. The coronal plane is also indicated.

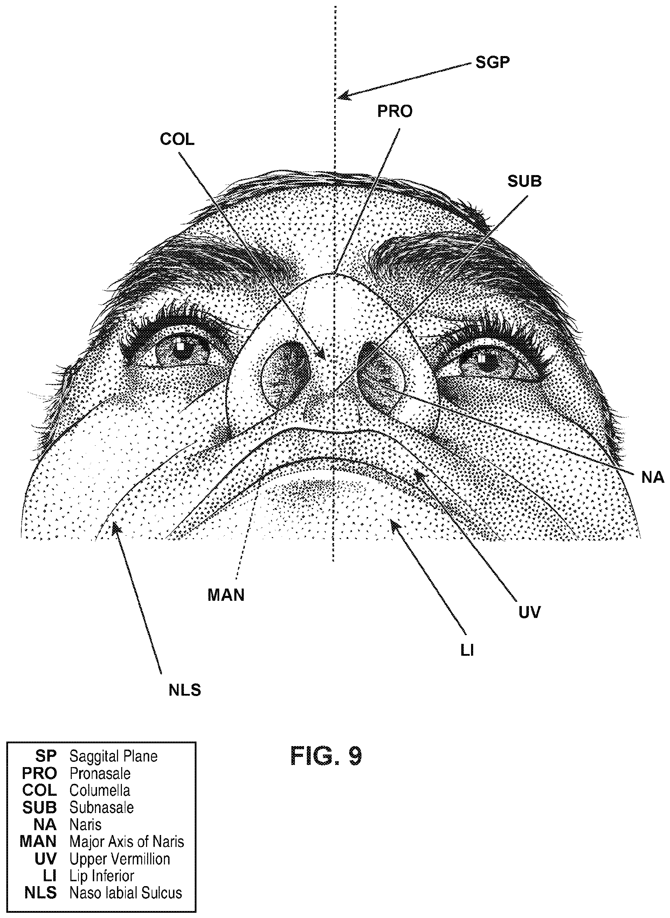

Fig. 9 shows a base view of a nose with several features identified including naso-labial sulcus, lip inferior, upper Vermilion, naris, subnasale, columella, pronasale, the major axis of a naris and the midsagittal plane.

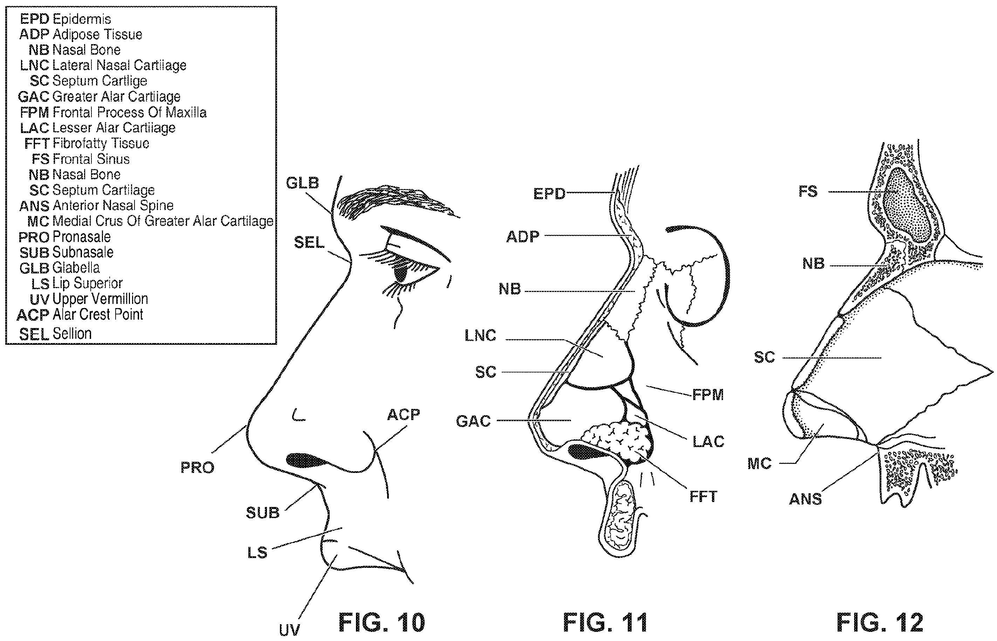

Fig. 10 shows a side view of the superficial features of a nose.
Fig. 11 shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 12 shows a medial dissection of a nose, approximately several millimeters from the midsagittal plane, amongst other things showing the septum cartilage and medial crus of greater alar cartilage.

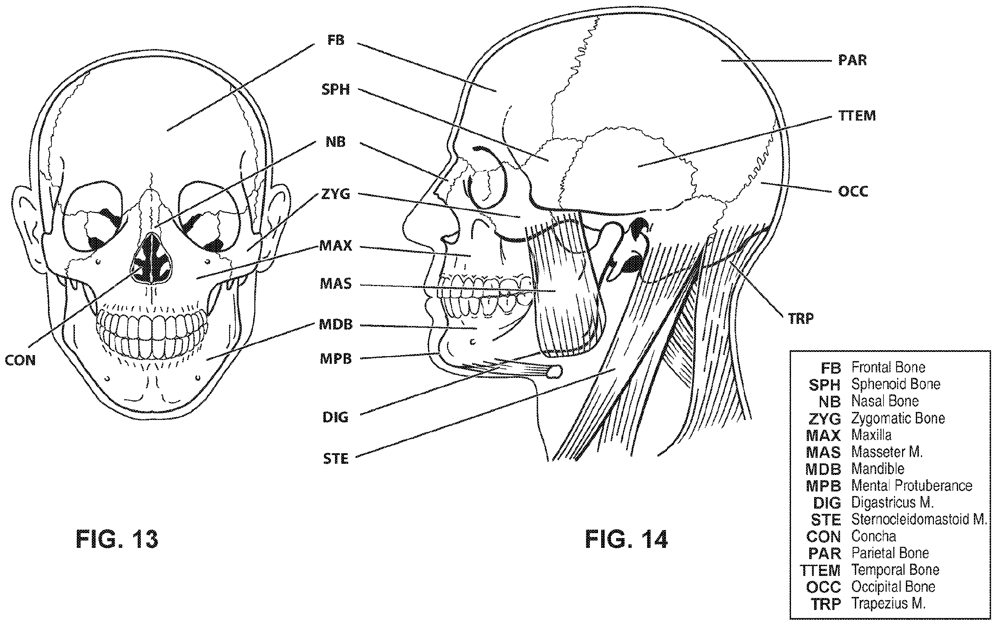

Fig. 13 shows a front view of the bones of a skull including the frontal, nasal and zygomatic bones. Nasal concha are indicated, as are the maxilla, and mandible.
Fig. 14 shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

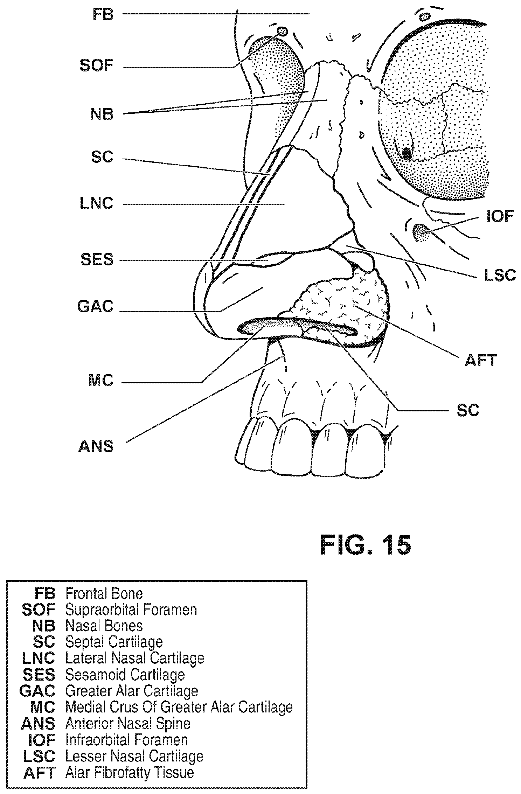

Fig. 15 shows an anterolateral view of a nose.

### 4.3 PATIENT INTERFACE

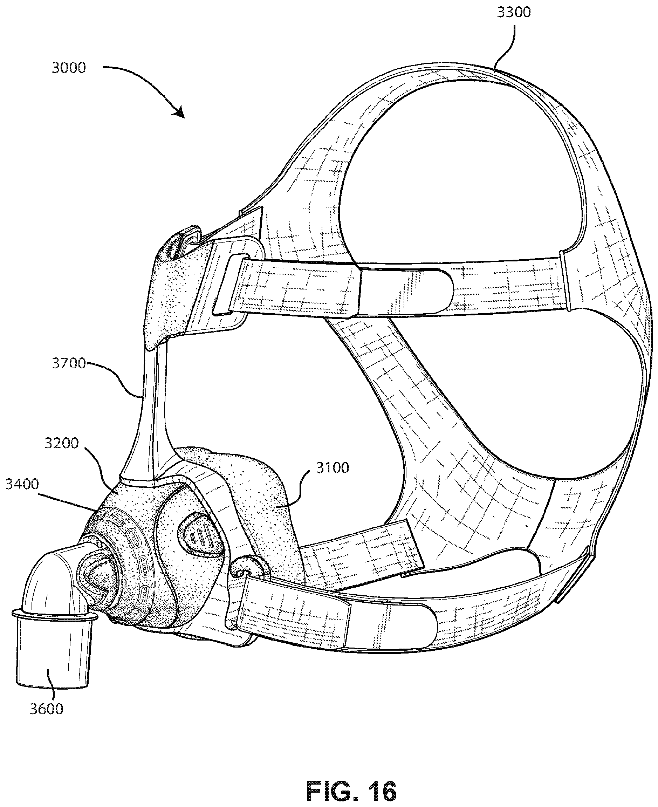

Fig. 16 shows a patient interface in the form of a nasal mask in accordance with one form of the present technology.

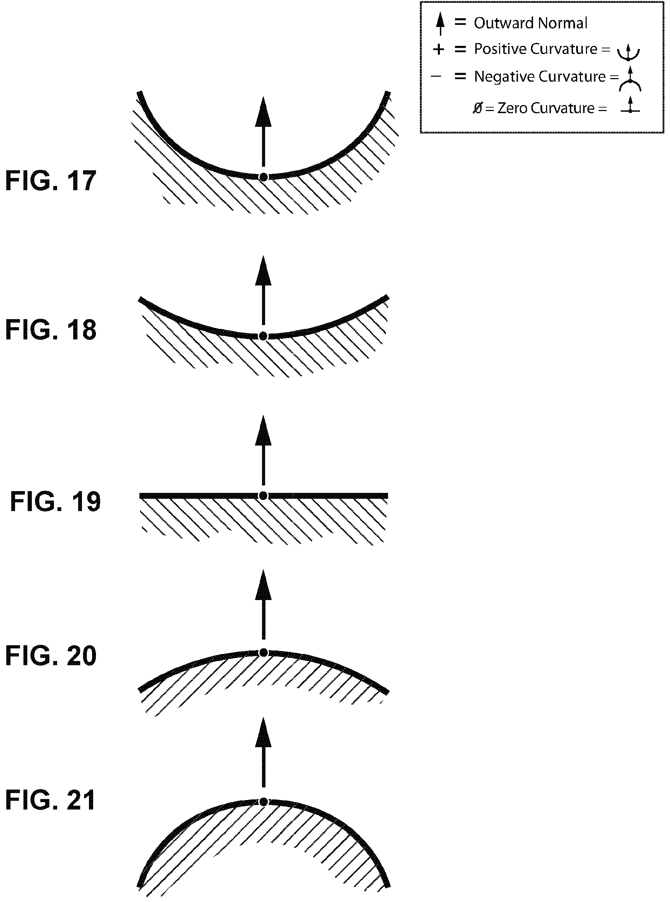

Fig. 17 shows a schematic of a cross-section through a structure at a point. An outward normal at the point is indicated. The curvature at the point has a positive sign, and a relatively large magnitude when compared to the magnitude of the curvature shown in Fig. 18.
Fig. 18 shows a schematic of a cross-section through a structure at a point. An outward normal at the point is indicated. The curvature at the point has a positive sign, and a relatively small magnitude when compared to the magnitude of the curvature shown in Fig. 17.
Fig. 19 shows a schematic of a cross-section through a structure at a point. An outward normal at the point is indicated. The curvature at the point has a value of zero.
Fig. 20 shows a schematic of a cross-section through a structure at a point. An outward normal at the point is indicated. The curvature at the point has a negative sign, and a relatively small magnitude when compared to the magnitude of the curvature shown in Fig. 21.
Fig. 21 shows a schematic of a cross-section through a structure at a point. An outward normal at the point is indicated. The curvature at the point has a negative sign, and a relatively large magnitude when compared to the magnitude of the curvature shown in Fig. 20.

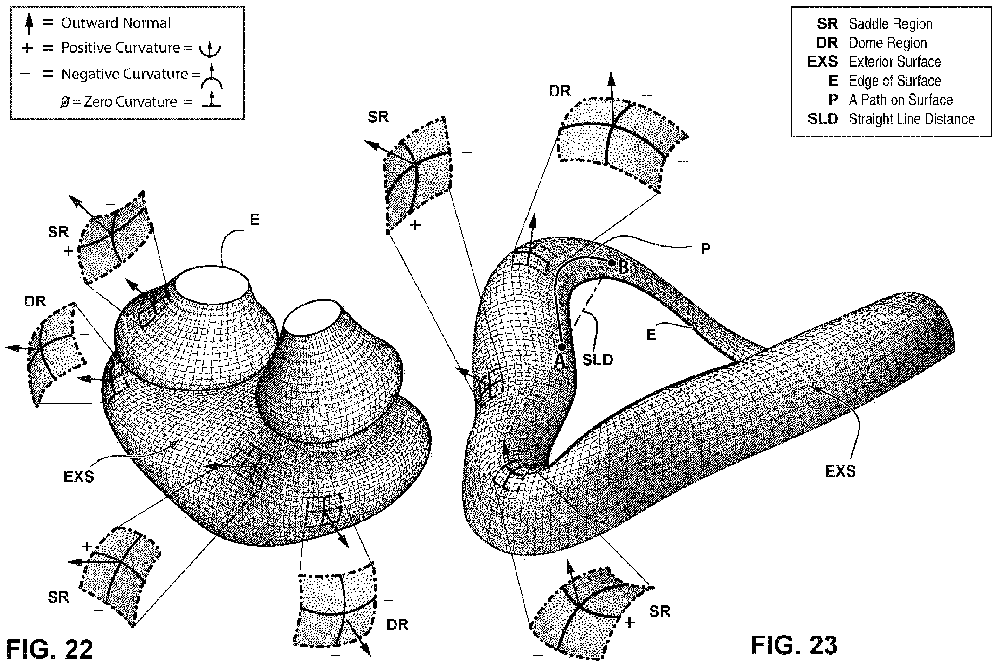

Fig. 22 shows a cushion for a mask that includes two pillows. An exterior surface of the cushion is indicated. An edge of the surface is indicated. Dome and saddle regions are indicated.
Fig. 23 shows a cushion for a mask. An exterior surface of the cushion is indicated. An edge of the surface is indicated. A path on the surface between points A and B is indicated. A straight line distance between A and B is indicated. Two saddle regions and a dome region are indicated.

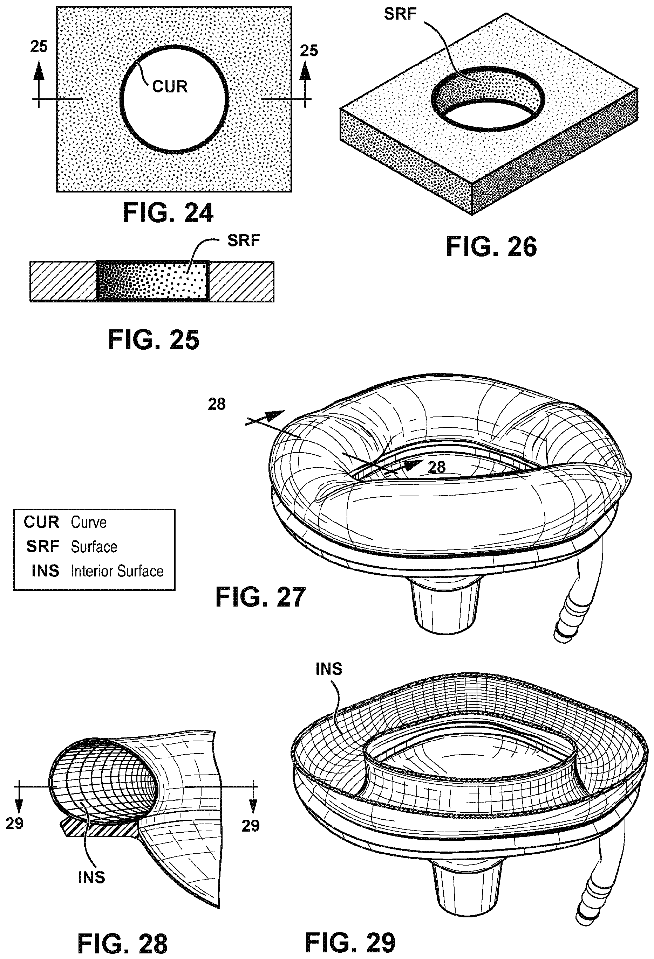

Fig. 24 shows the surface of a structure, with a one dimensional hole in the surface. The illustrated plane curve forms the boundary of a one dimensional hole.
Fig. 25 shows a cross-section through the structure of Fig.24. The illustrated surface bounds a two dimensional hole in the structure of Fig. 24.
Fig. 26 shows a perspective view of the structure of Fig. 24, including the two dimensional hole and the one dimensional hole. Also shown is the surface that bounds a two dimensional hole in the structure of Fig. 24.
Fig. 27 shows a mask having an inflatable bladder as a cushion.
Fig. 28 shows a cross-section through the mask of Fig. 27, and shows the interior surface of the bladder. The interior surface bounds the two dimensional hole in the mask.
Fig. 29 shows a further cross-section through the mask of Fig. 27. The interior surface is also indicated.

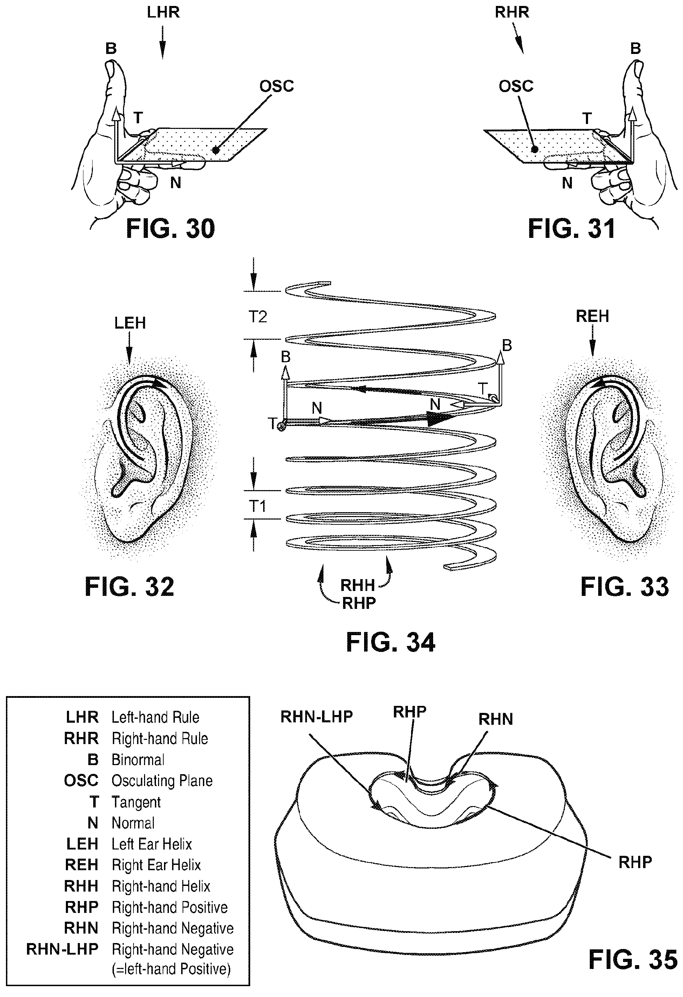

Fig. 30 illustrates a left-hand rule.
Fig. 31 illustrates a right-hand rule.
Fig. 32 shows a left ear, including the left ear helix.
Fig. 33 shows a right ear, including the right ear helix.
Fig. 34 shows a right-hand helix.
Fig. 35 shows a view of a mask, including the sign of the torsion of the space curve defined by the edge of the sealing membrane in different regions of the mask.
Fig. 36 shows a view of a plenum chamber 3200 showing a sagittal plane and a mid-contact plane.
Fig. 37 shows a view of a posterior of the plenum chamber of Fig. 36. The direction of the view is normal to the mid-contact plane. The sagittal plane in Fig. 37 bisects the plenum chamber into left-hand and right-hand sides.

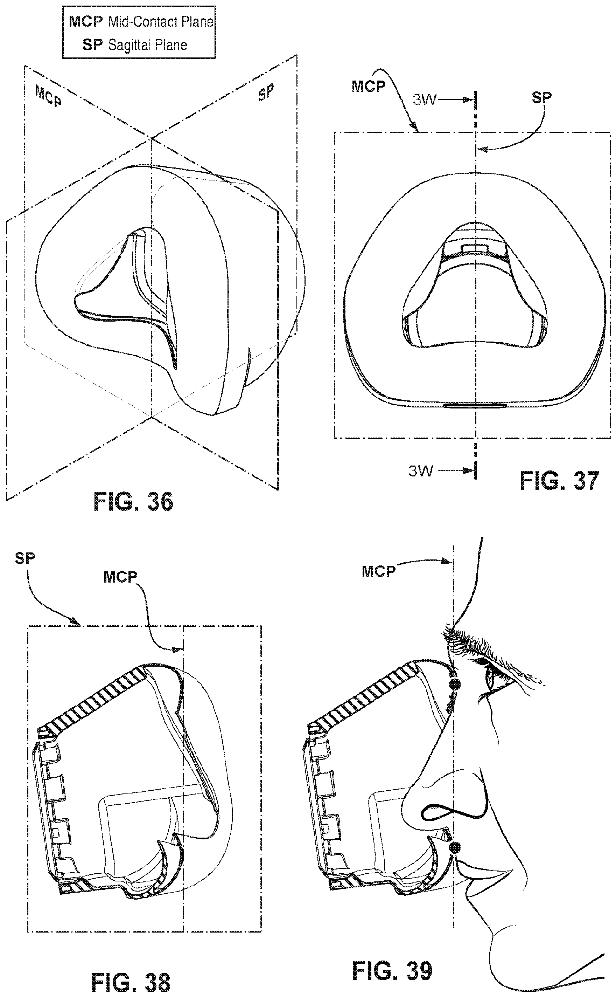

Fig. 38 shows a cross-section through the plenum chamber of Fig. 37, the cross-section being taken at the sagittal plane shown in Fig. 37. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.
Fig. 38 shows the plenum chamber 3200 of Fig. 36 in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 38 the plenum chamber 3200 is that of a nasal mask, and the superior point 3220 sits approximately on the sellion, while the inferior point 3230 sits on the lip superior.

### 4.4 RPT DEVICE

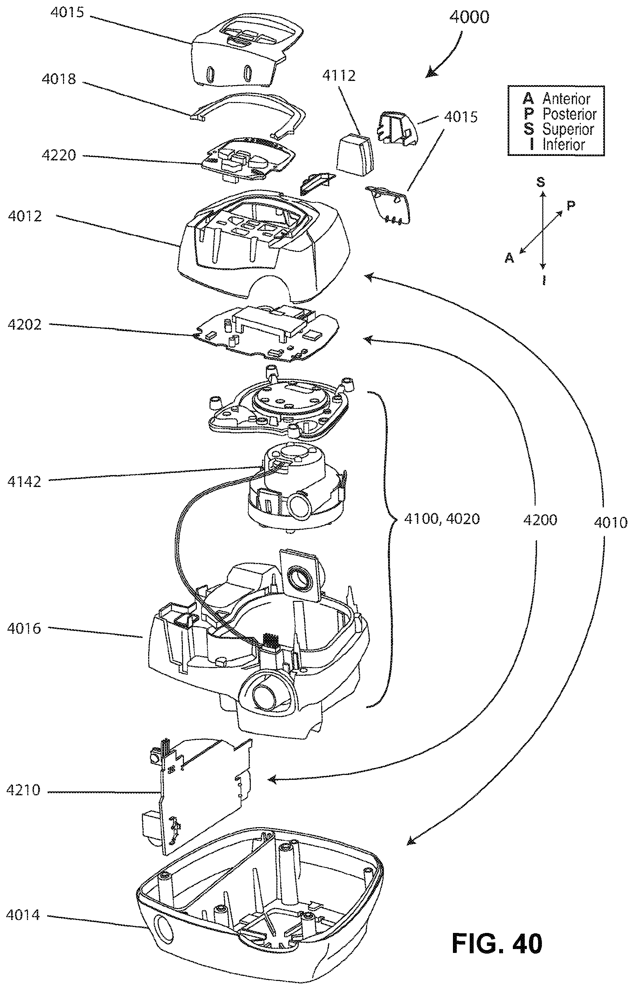

Fig. 40 shows an RPT device in accordance with one form of the present technology.

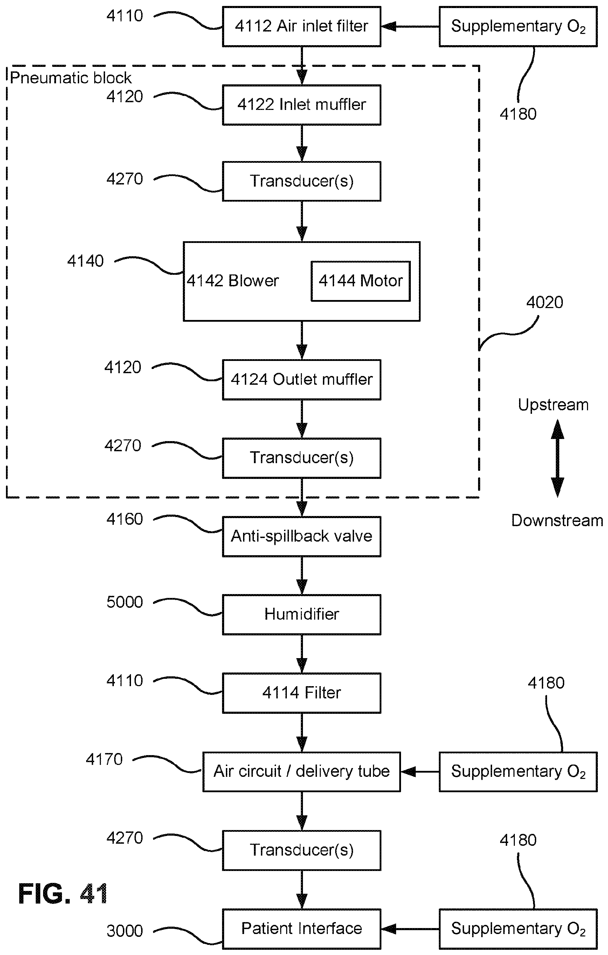

Fig. 41 is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 4.5 BREATHING WAVEFORMS

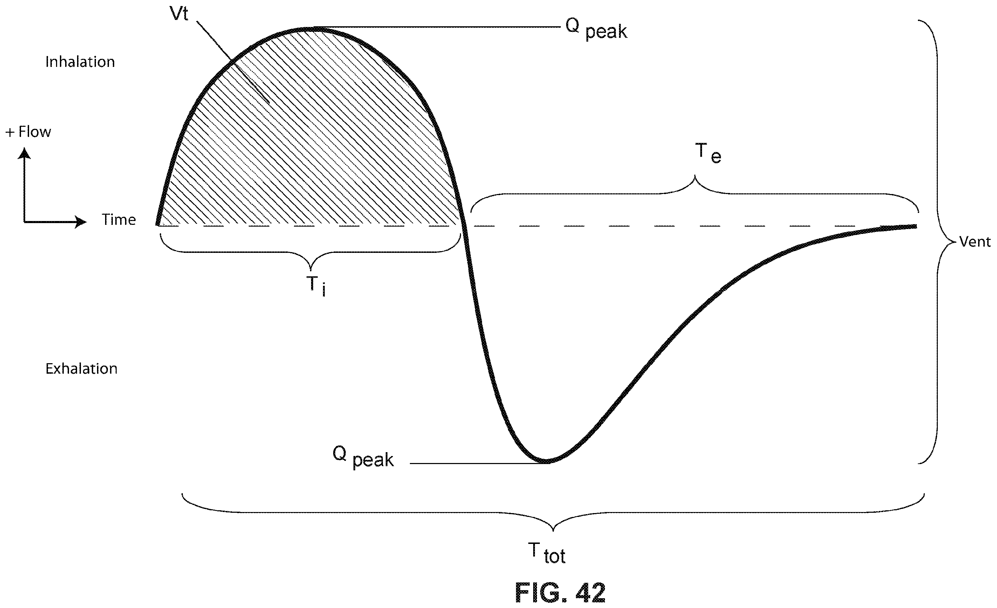

Fig. 42 shows a model typical breath waveform of a person while sleeping.

### 4.6 PATIENT INTERFACE

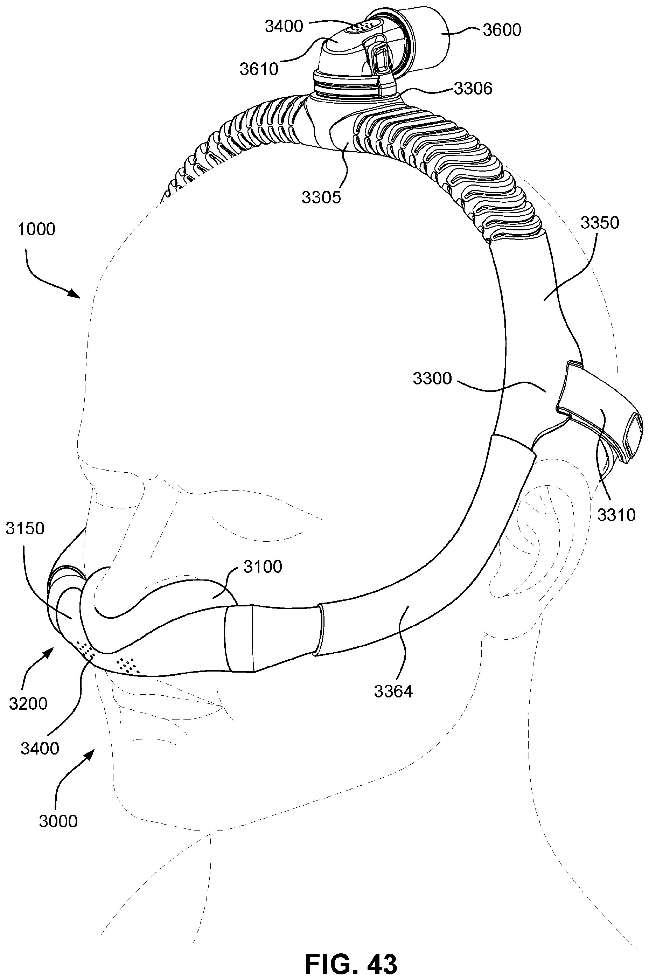

Fig. 43 shows a perspective view of a patient interface according to an example of the present invention on a patient.

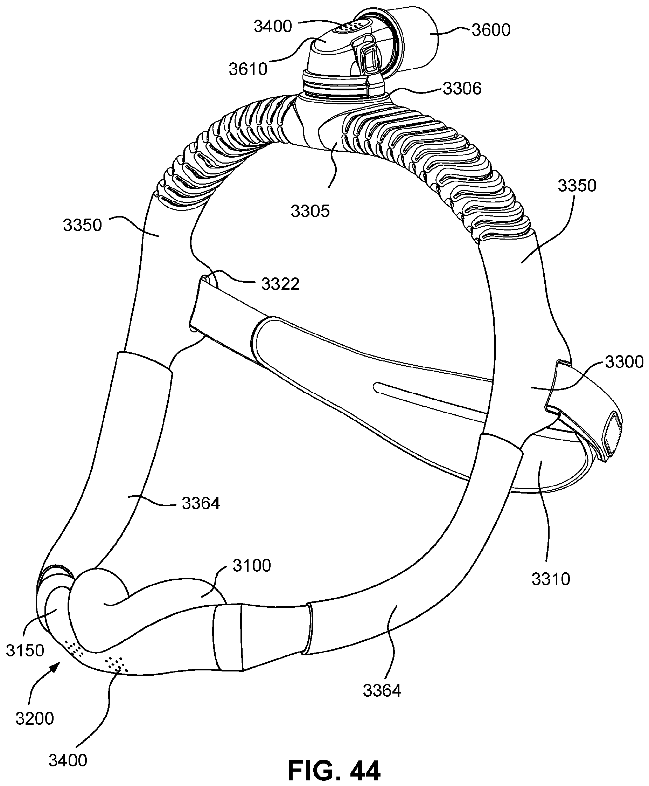

Fig. 44 shows a perspective view of a patient interface according to an example of the present invention.

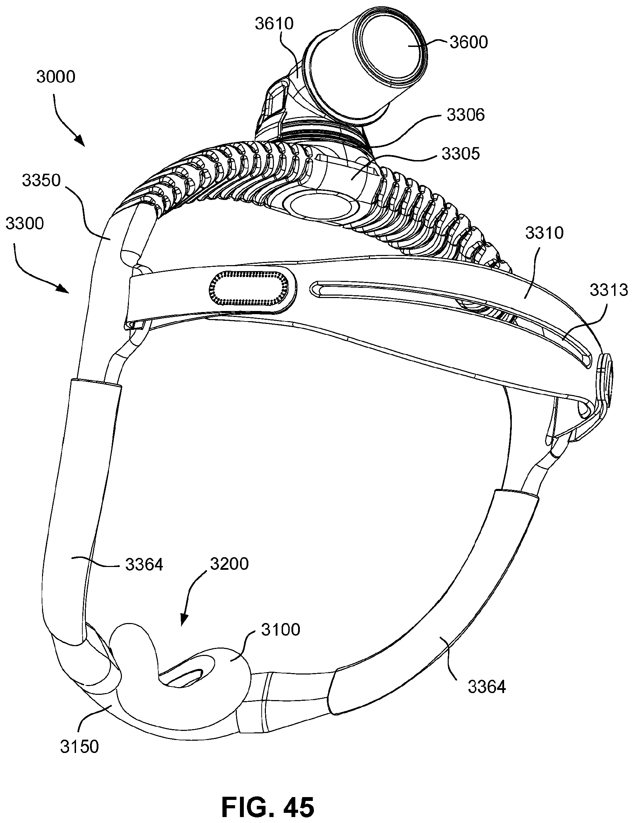

Fig. 45 shows a rear perspective view of a patient interface according to an example of the present invention.

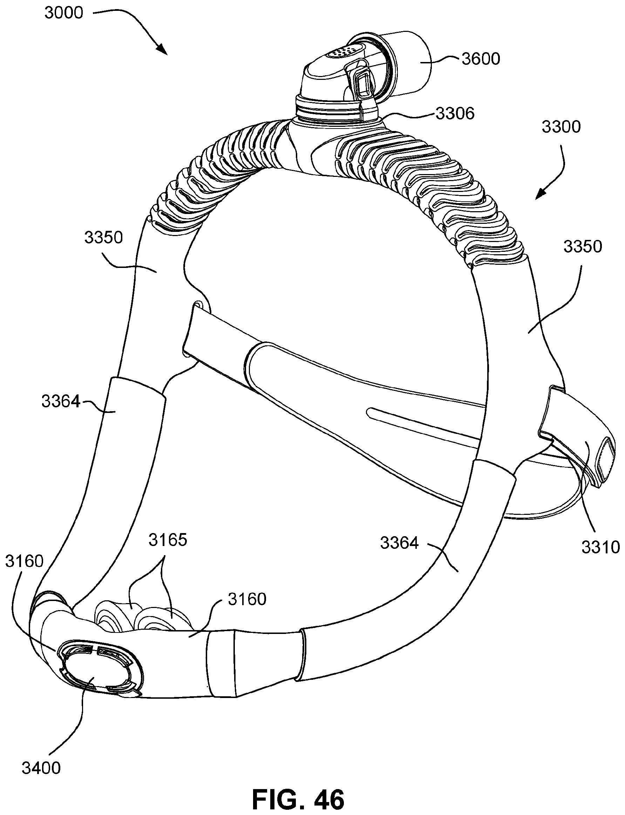

Fig. 46 shows a perspective view of a patient interface according to another example of the present invention.

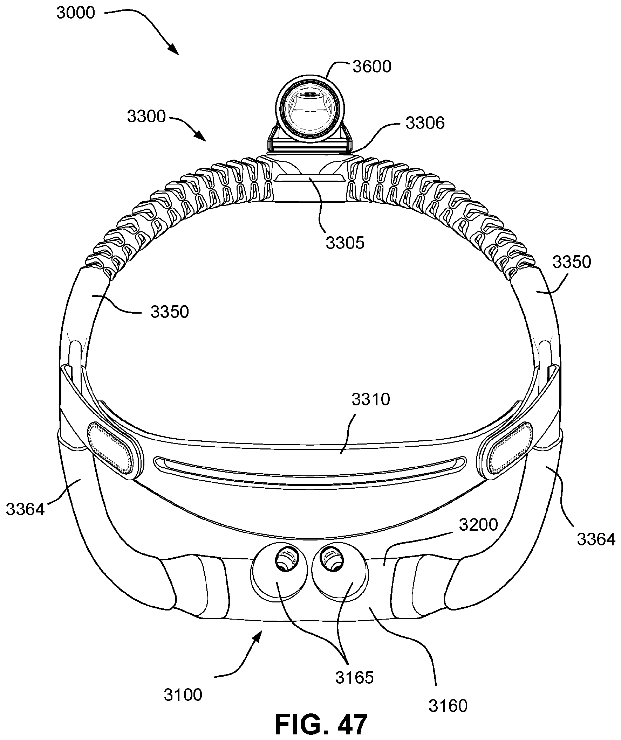

Fig. 47 shows a rear view of a patient interface according to another example of the present invention.

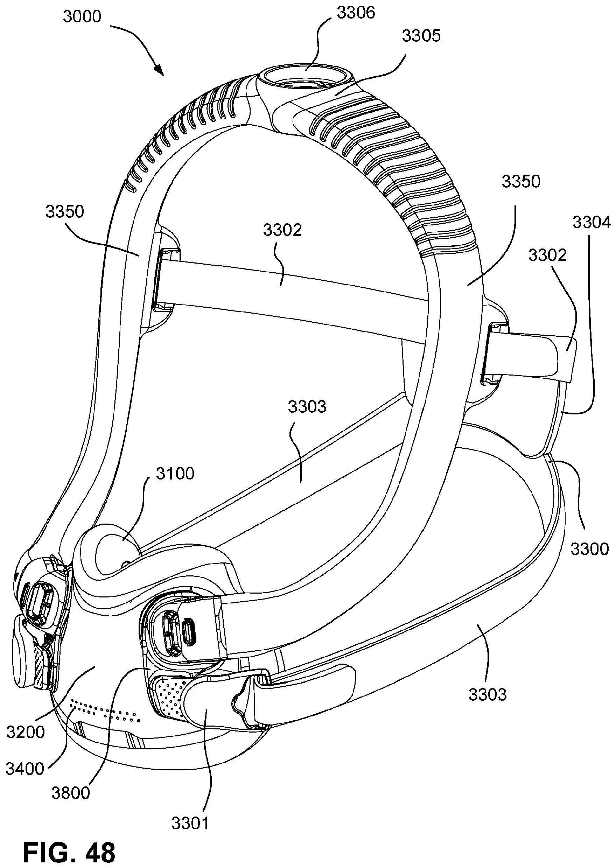

Fig. 48 shows a perspective view of a patient interface according to another example of the present invention on a patient.

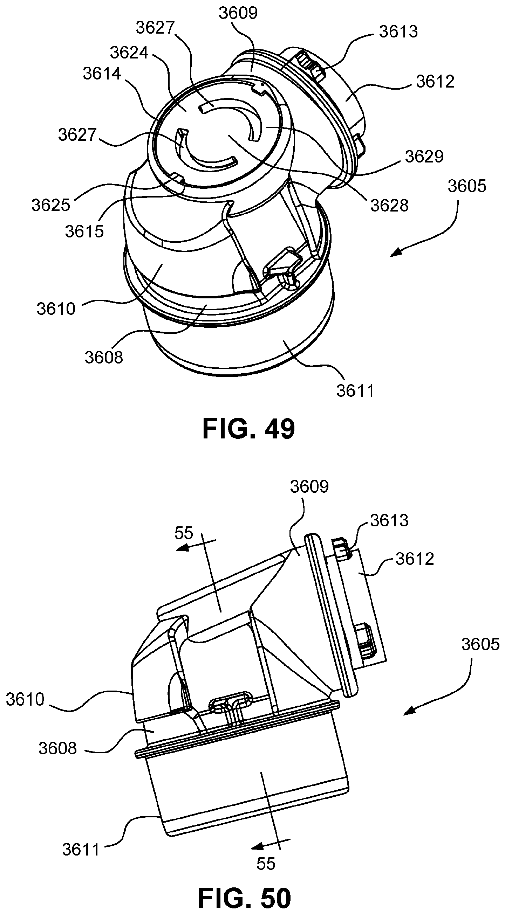

Fig. 49 shows a top perspective view of a conduit connector according to an example of the present invention.
Fig. 50 shows a side view of a conduit connector according to an example of the present invention.

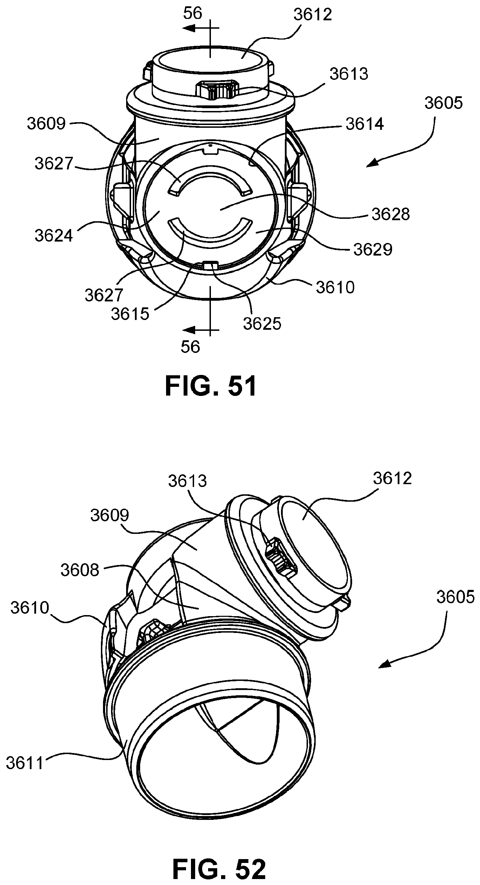

Fig. 51 shows a top view of a conduit connector according to an example of the present invention.
Fig. 52 shows a bottom perspective view of a conduit connector according to an example of the present invention.

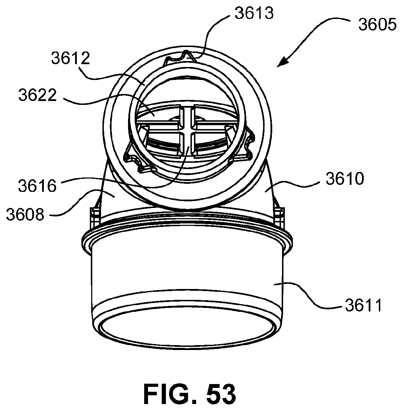

Fig. 53 shows another side view of a conduit connector according to an example of the present invention.

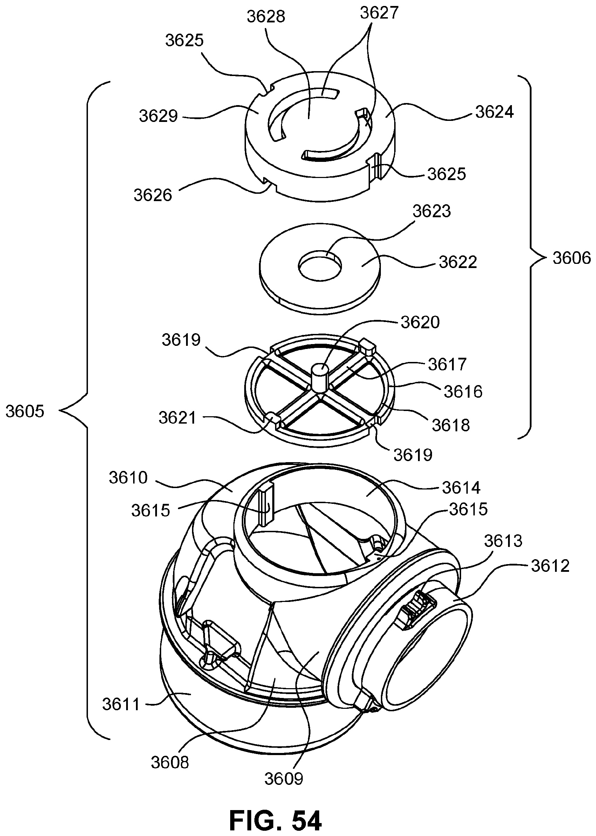

Fig. 54 shows an exploded view of a conduit connector according to an example of the present invention.

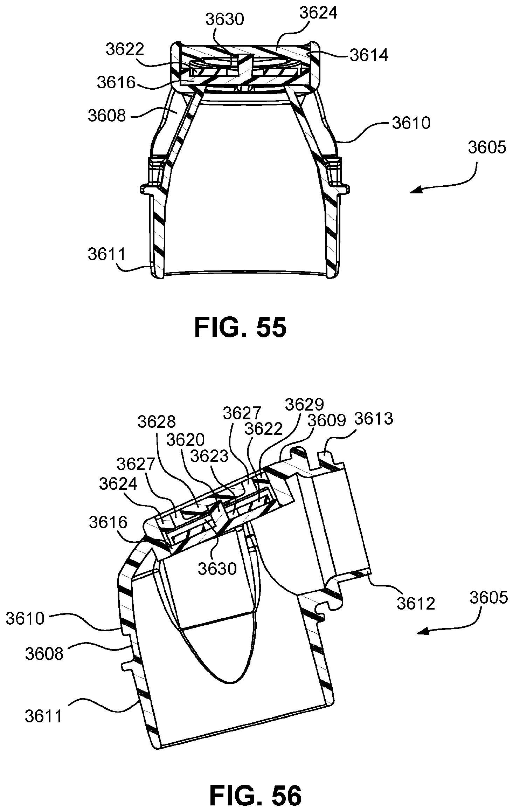

Fig. 55 shows a cross-sectional view of a conduit connector according to an example of the present invention taken through line 55-55 of Fig. 50.
Fig. 56 shows a cross-sectional view of a conduit connector according to an example of the present invention taken through line 56-56 of Fig. 51.

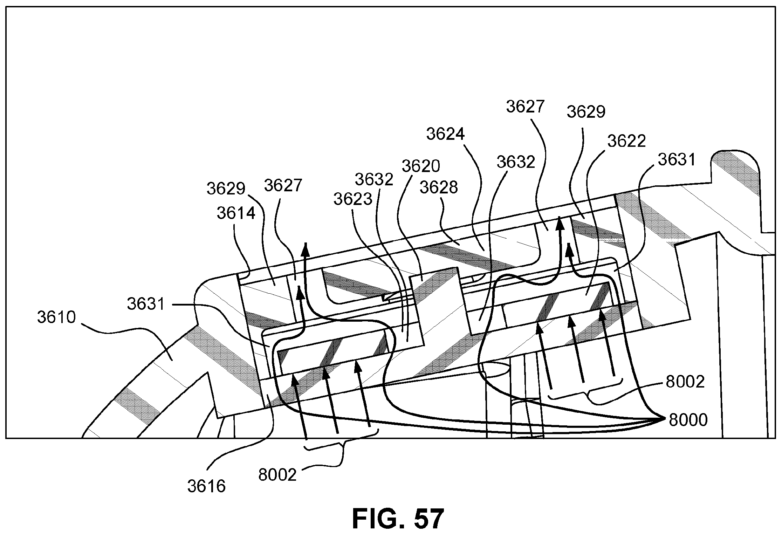

Fig. 57 shows a detailed cross-sectional view of a conduit connector according to an example of the present invention based on Fig. 56 with a flap in an undeformed position.

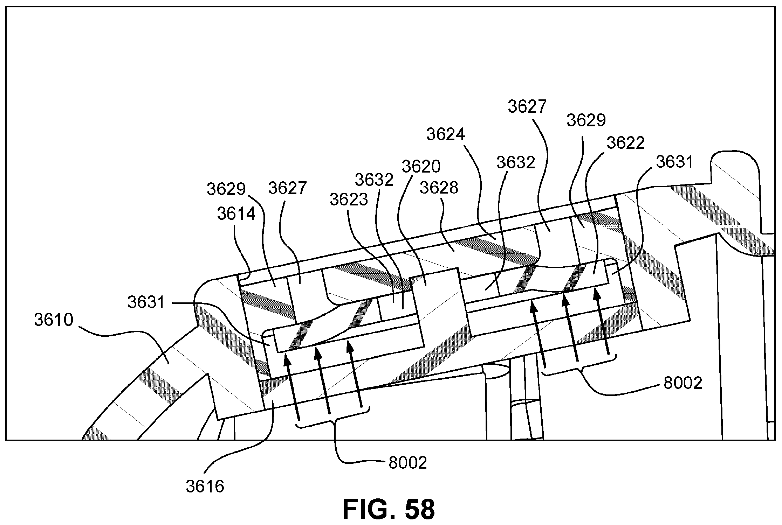

Fig. 58 shows a detailed cross-sectional view of a conduit connector according to an example of the present invention based on Fig. 56 with a flap in an undeformed position.

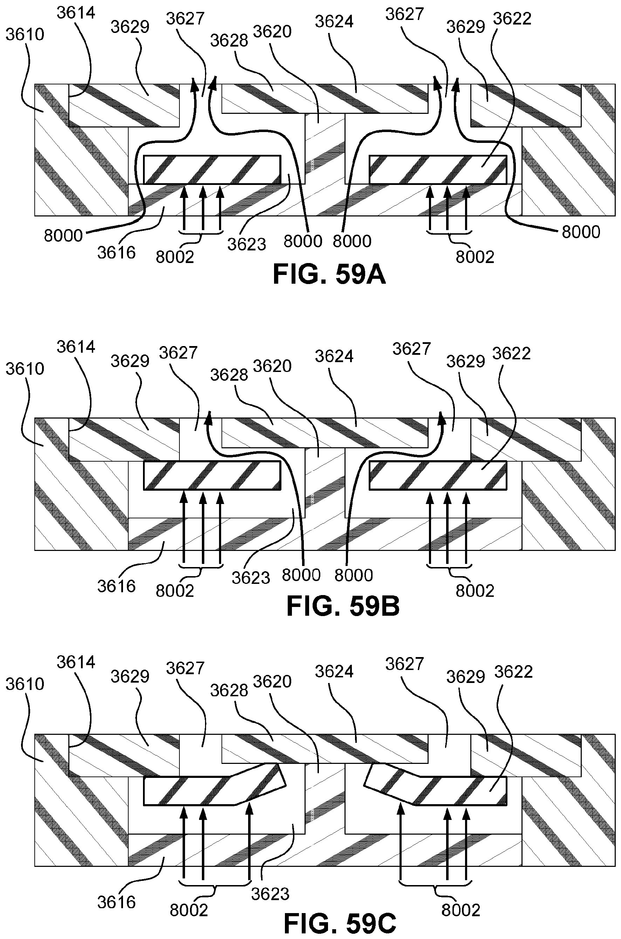

Figs. 59A-59C show a schematic of a conduit connector vent according to an example of the present invention with a flap in different positions.

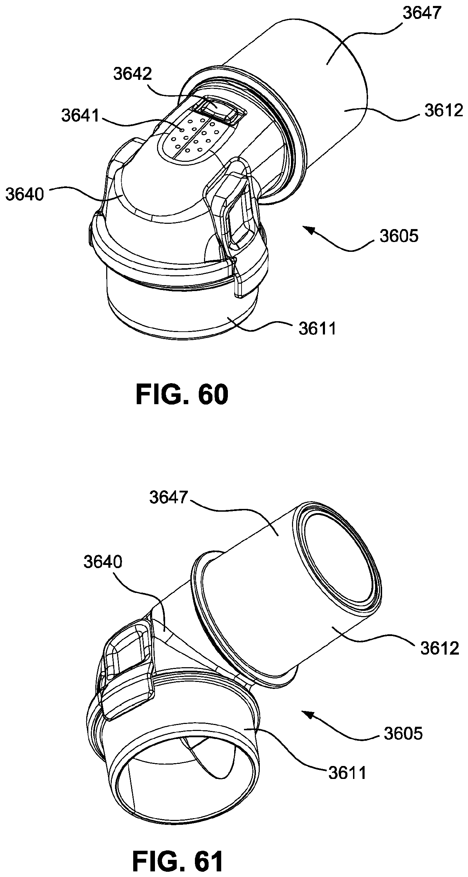

Fig. 60 shows a perspective view of a conduit connector according to an example of the present technology.
Fig. 61 shows another perspective view of a conduit connector according to an example of the present technology.

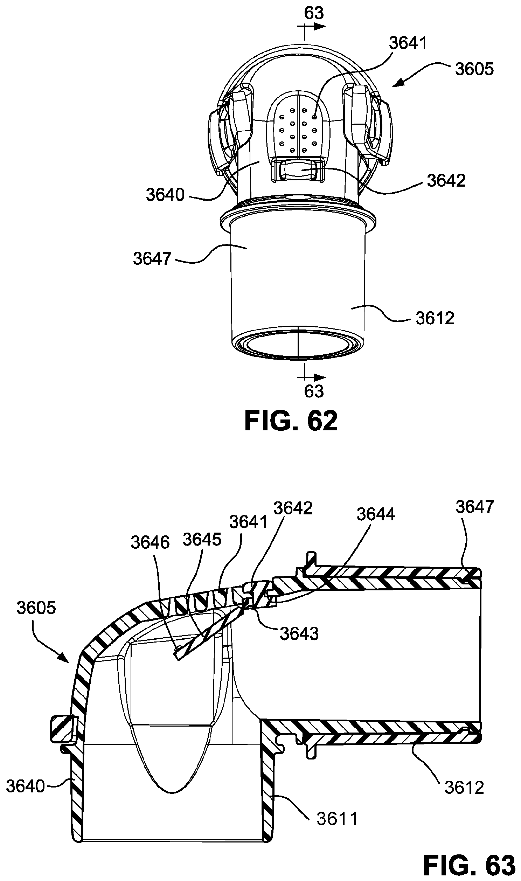

Fig. 62 shows a top view of a conduit connector according to an example of the present technology.
Fig. 63 shows a cross-sectional view of a conduit connector according to an example of the present technology taken through line 63-63 of Fig. 62.

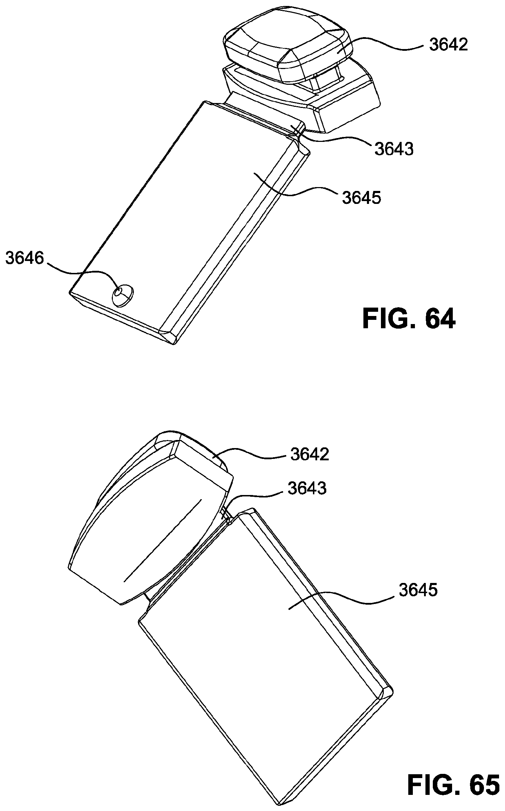

Fig. 64 shows a perspective view of a flap for a conduit connector according to an example of the present technology.
Fig. 65 shows another perspective view of a flap for a conduit connector according to an example of the present technology.

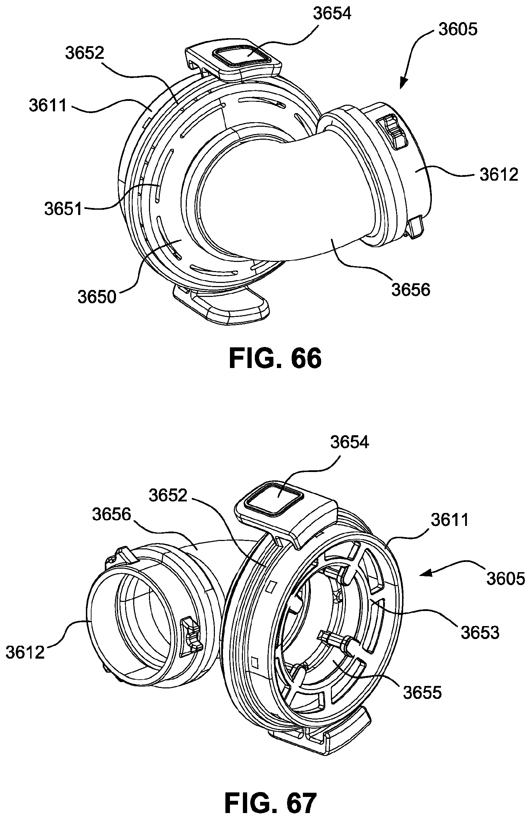

Fig. 66 shows a perspective view of a conduit connector according to an example of the present technology.
Fig. 67 shows another perspective view of a conduit connector according to an example of the present technology.

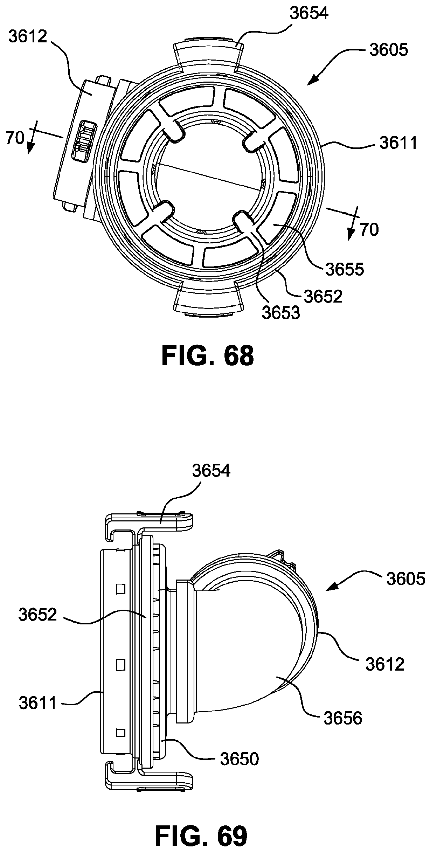

Fig. 68 shows a side view of a conduit connector according to an example of the present technology.
Fig. 69 shows a top view of a conduit connector according to an example of the present technology.

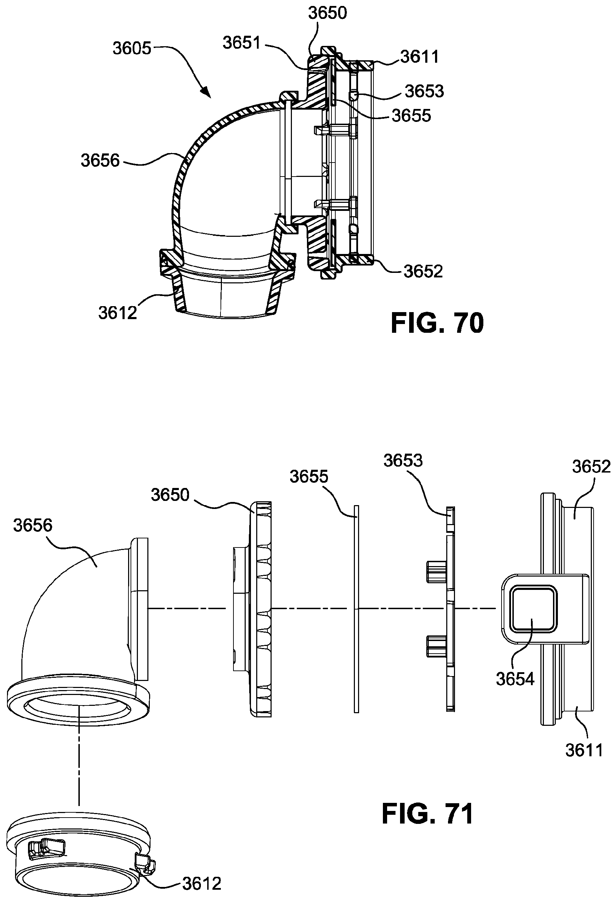

Fig. 70 shows a cross-sectional view of a conduit connector according to an example of the present technology taken through line 70-70 of Fig. 68.
Fig. 71 shows an exploded view of a conduit connector according to an example of the present technology.

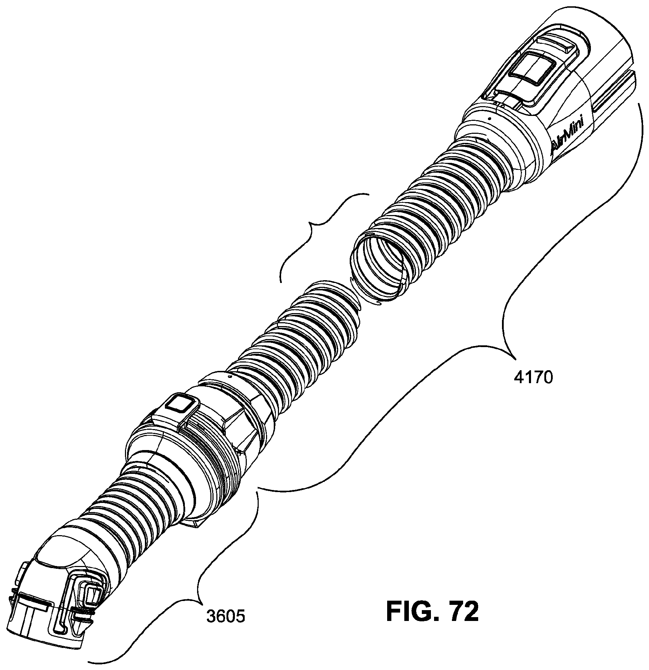

Fig. 72 shows a perspective view of a conduit connector and a conduit according to an example of the present technology.

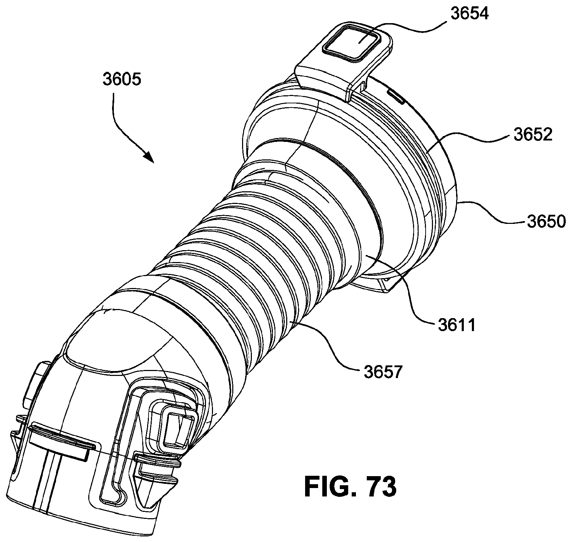

Fig. 73 shows a perspective view of a conduit connector according to an example of the present technology.

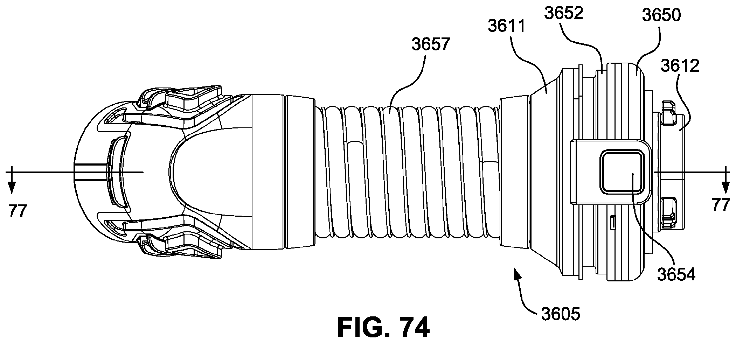

Fig. 74 shows a top view of a conduit connector according to an example of the present technology.

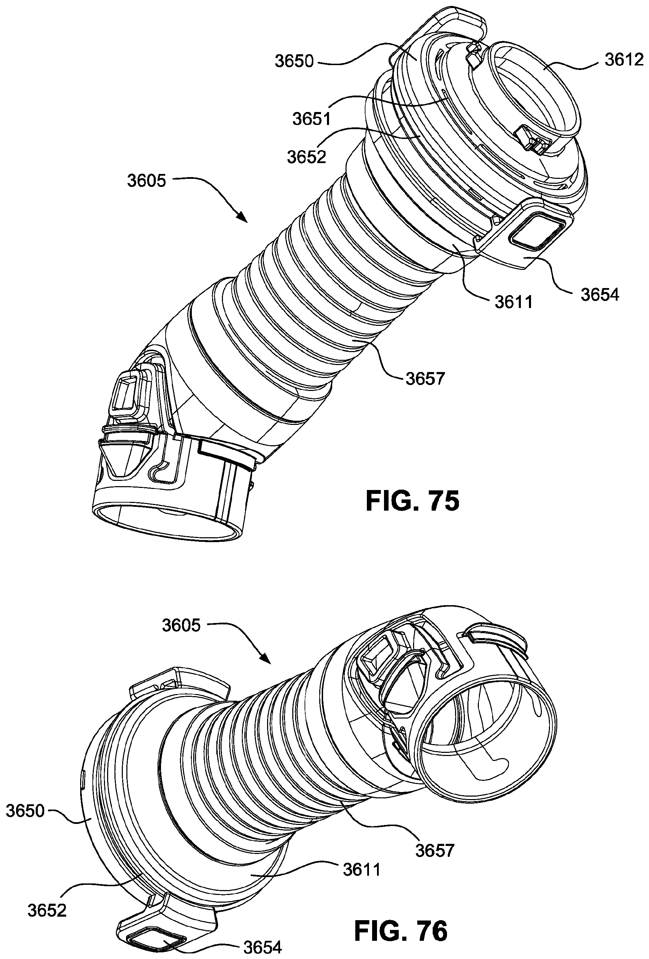

Fig. 75 shows another perspective view of a conduit connector according to an example of the present technology.
Fig. 76 shows another perspective view of a conduit connector according to an example of the present technology.

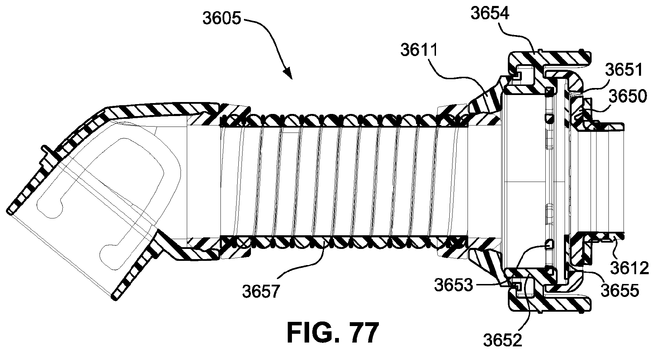

Fig. 77 shows a cross-sectional view of a conduit connector according to an example of the present technology taken through line 77-77 of Fig. 74.

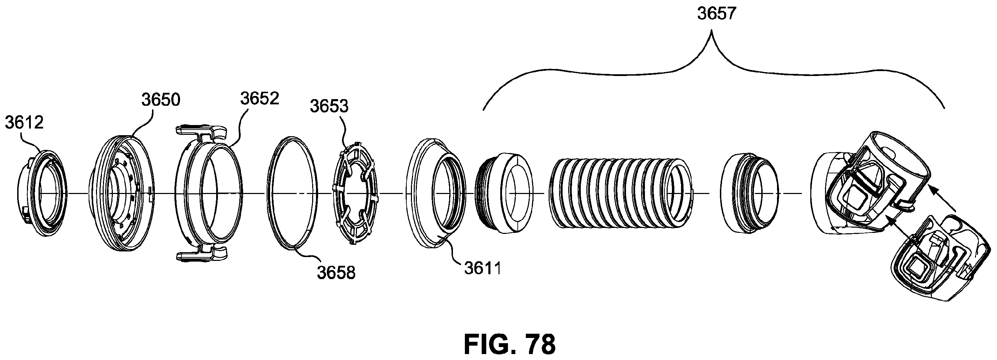

Fig. 78 shows an exploded view of a conduit connector according to an example of the present technology.

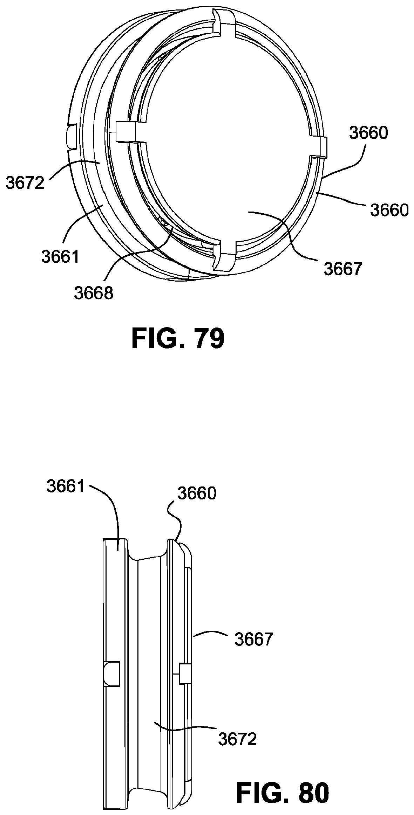

Fig. 79 shows a perspective view of a vent system according to an example of the present technology.
Fig. 80 shows a side view of a vent system according to an example of the present technology.

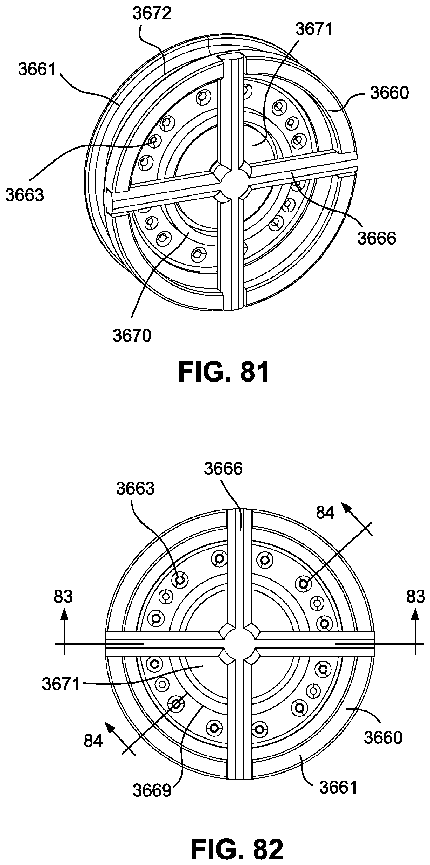

Fig. 81 shows another perspective view of a vent system according to an example of the present technology.
Fig. 82 shows a rear view of a vent system according to an example of the present technology.

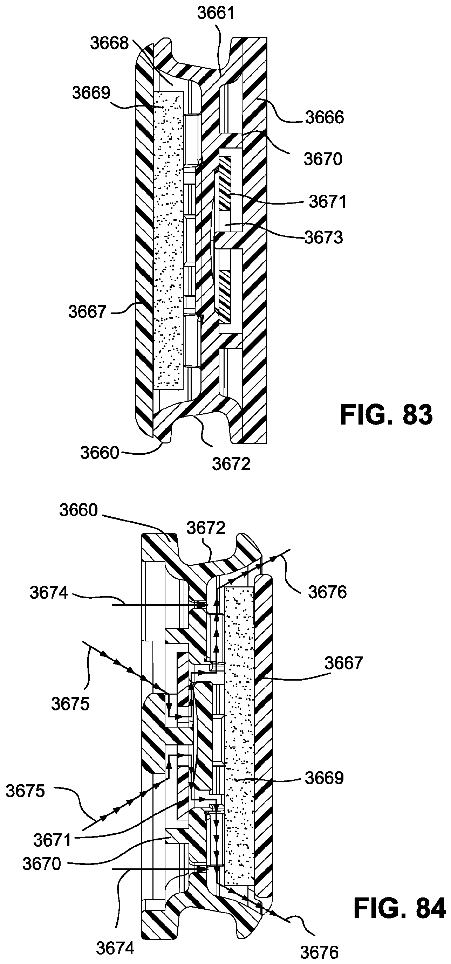

Fig. 83 shows a cross-sectional view of a vent system according to an example of the present technology taken through line 83-83 of Fig. 82.
Fig. 84 shows a cross-sectional view of a vent system according to an example of the present technology taken through line 84-84 of Fig. 82.

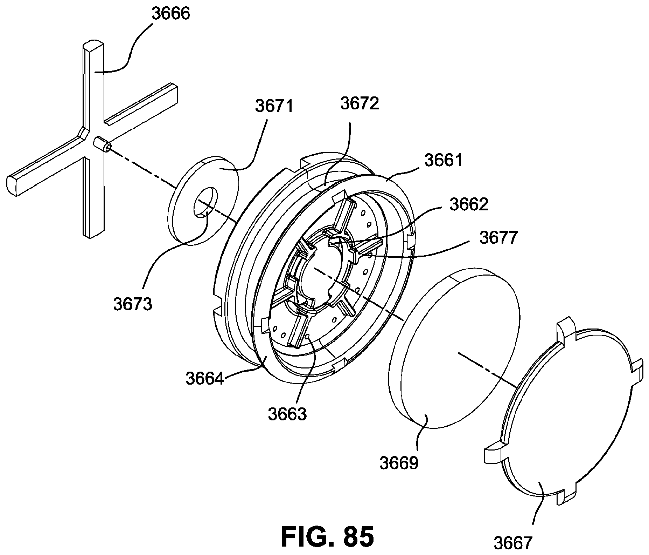

Fig. 85 shows an exploded view of a vent system according to an example of the present technology.

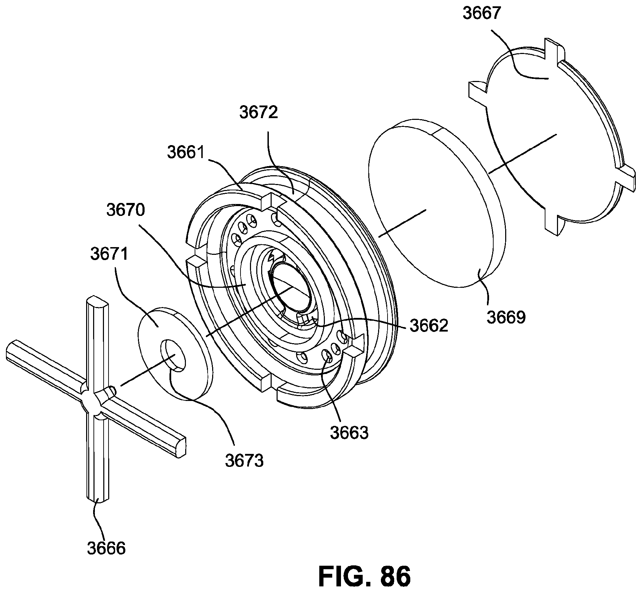

Fig. 86 shows another exploded view of a vent system according to an example of the present technology.

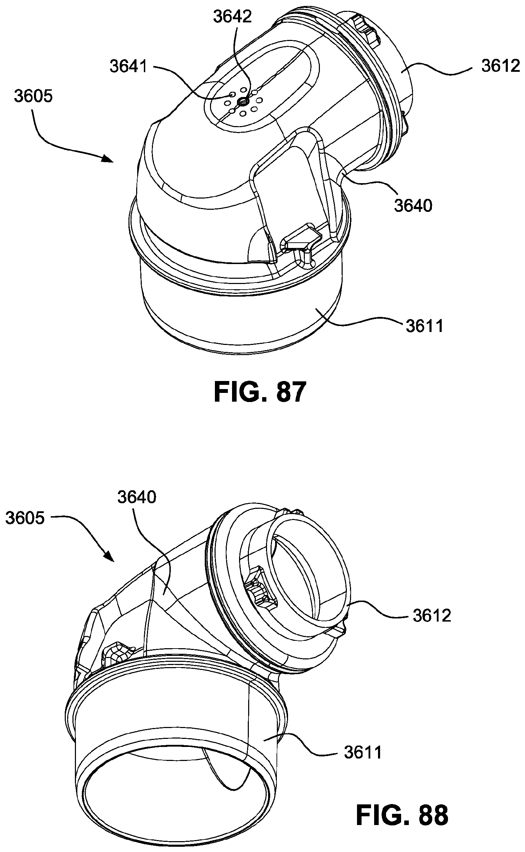

Fig. 87 shows a perspective view of a conduit connector according to an example of the present technology.
Fig. 88 shows another perspective view of a conduit connector according to an example of the present technology.

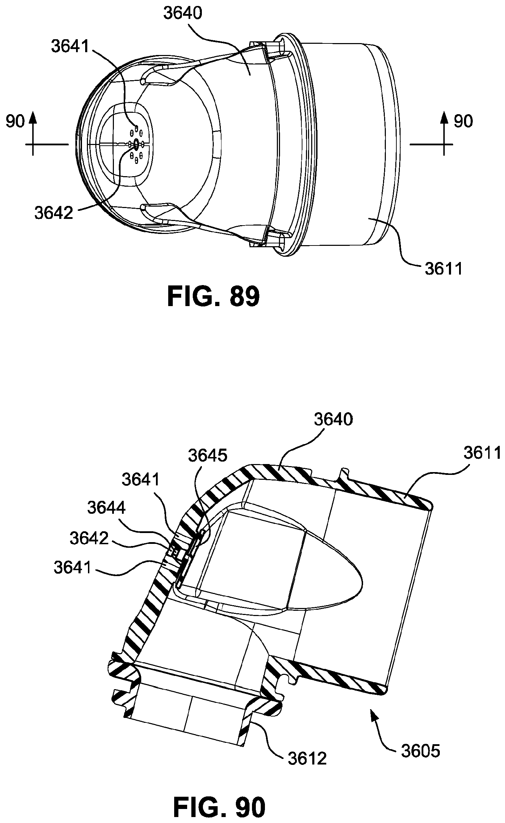

Fig. 89 shows a top view of a conduit connector according to an example of the present technology.
Fig. 90 shows a cross-sectional view of a conduit connector according to an example of the present technology taken through line 90-90 of Fig. 89.

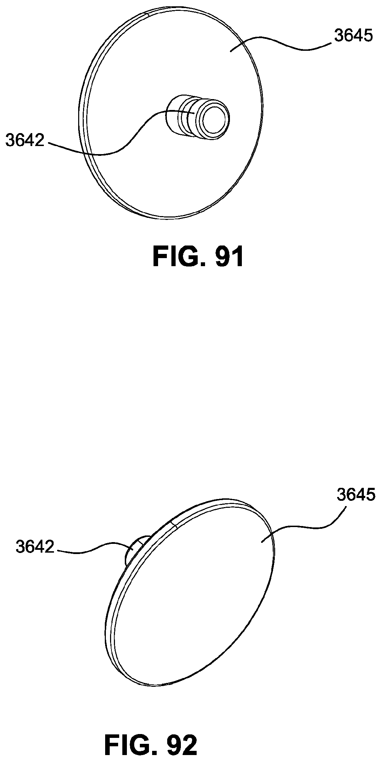

Fig. 91 shows a perspective view of a flap of a conduit connector according to an example of the present technology.
Fig. 92 shows another perspective view of a flap of a conduit connector according to an example of the present technology.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000.

### 5.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH2O with respect to ambient.

The patient interface 3000 in accordance with one form of the present technology is constructed and arranged to be able to provide a supply of air at a positive pressure of at least 10 cmH2O with respect to ambient.

The patient interface 3000 in accordance with one form of the present technology is constructed and arranged to be able to provide a supply of air at a positive pressure of at least 20 cmH2O with respect to ambient.

### 5.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 5.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 5.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 5.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 5.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 5.3.1.5 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

In one form the seal-forming structure 3100 of the non-invasive patient interface 3000 comprises a pair of nasal puffs, or nasal pillows, each nasal puff or nasal pillow being constructed and arranged to form a seal with a respective naris of the nose of a patient. Figs. 46 and 47 show a patient interface 3000 having a seal-forming structure 3100 provided by a pillows cushion module 3160. The pillows cushion module 3160 comprises a pair of nasal pillows 3165. In this example, the same positioning structure 3300 as shown in Figs. 43-45 is used to hold the pillows cushion module 3160 in sealing contact with the patient's nose. The same concepts and features of the positioning and stabilising structure 3300 described with reference to the cradle cushion module 3150 may be applied to a positioning and stabilising structure 3300 configured to be used with the pillows cushion module 3160 (or another type of cushion module such as a full face cushion module, oro-nasal cushion module, ultra-compact full face cushion module, nasal cushion module and the like).

Nasal pillows 3165 in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 5.3.1.1 Nasal Cradle

In one form, for example as shown in Figs. 43-45, the seal-forming structure 3100 is configured to form a seal in use with the underside of the nose around the nares and optionally with the lip superior of the patient 1000. This type of seal-forming structure may be referred to as a "cradle cushion" or "sub-nasal mask". The shape of the seal-forming structure may be configured to match or closely follow the underside of the patient's nose, i.e. the profile and angle of the seal-forming structure may be substantially parallel to the patient's naso-labial angle. In one form of nasal cradle cushion, the seal-forming structure comprises a bridge portion defining two orifices, each of which, in use, supplies air or breathable gas to a different one of the patient's nares. The bridge portion may be configured to contact or seal against the patient's columella in use. In some forms of the technology, the seal-forming structure 3100 is configured to form a seal on an underside of the patient's nose without contacting a nasal bridge region of the patient's nose. In some examples, patient interface may comprise a seal-forming structure 3100 in the form of a cradle cushion as described in U.S. Publication No. US 2020/0054850 A1.

### 5.3.1.2 Nasal Mask Cushion

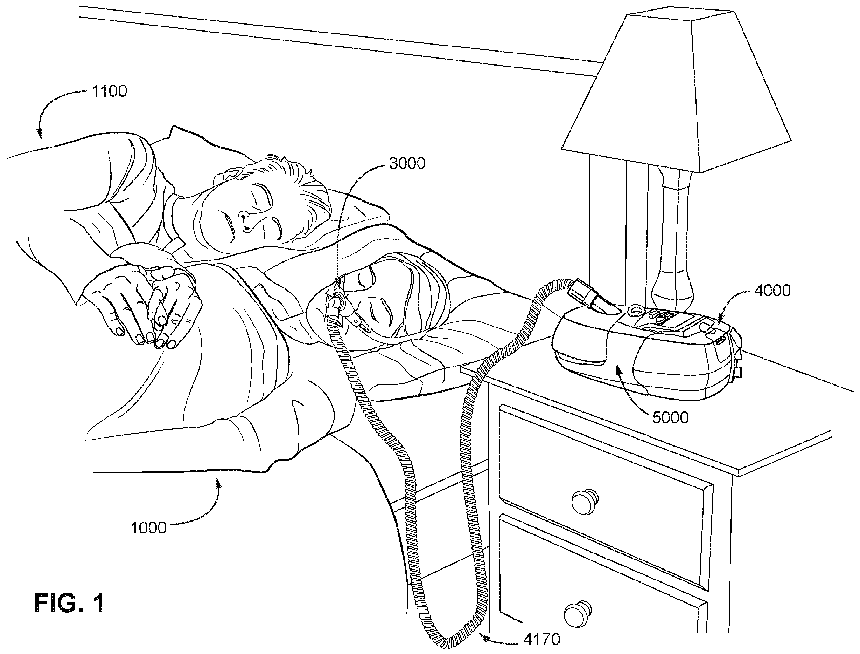

In one form, the non-invasive patient interface 3000 comprises a seal-forming portion that forms a seal in use on an upper lip region (that is, the *lip superior*), a nasal bridge region and a cheek region of the patient's face. This is the case, for example, with the patient interface 3000 shown in Fig. 1B. This seal-forming portion delivers a supply of air or breathable gas to both nares of patient 1000 through a single orifice. This type of seal-forming structure may be referred to as a "nasal cushion" or "nasal mask". In some examples of the present technology, the positioning and stabilising structure 3300 shown in Figs. 43-47 may be utilised to hold a nasal cushion in sealing position on a patient's face.

### 5.3.1.3 Full-face Mask Cushion

In one form the patient interface 3000 comprises a seal-forming portion that forms a seal in use on a chin-region, a nasal bridge region and a cheek region of the patient's face. This is the case, for example, with the patient interface 3000 shown in Fig. 1C. This seal-forming portion delivers a supply of air or breathable gas to both nares and mouth of patient 1000 through a single orifice. This type of seal-forming structure may be referred to as a "full-face mask". In some examples of the present technology, the positioning and stabilising structure 3300 shown in Figs. 43-47 may be utilised to hold a full-face cushion in sealing position on a patient's face.

### 5.3.1.4 Oronasal Mask Cushion

In another form the patient interface 3000 comprises a nasal seal-forming structure in the manner of a nasal cushion or nasal cradle cushion and an oral seal-forming structure that is configured to form a seal in use around the mouth of a patient (which may be referred to as a "mouth cushion" or "oral mask"). In such a mask air or breathable gas is supplied in use through separate orifices to the patient's nares and the patient's mouth. This type of seal-forming structure 3100 may be referred to as an "oronasal cushion" or "ultra-compact full face cushion". In one form, the nasal seal-forming structure and oral seal-forming structure are integrally formed as a single component, an example of which is shown in Fig. 48. In some examples, patient interface may comprise a seal-forming structure 3100 in the form of a cradle cushion as described in International Publication No. WO 2019/183680 A1.

### 5.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

The plenum chamber 3200 in examples of the present technology may be constructed from a relatively flexible material such as silicone rubber. In further examples, the seal-forming structure 3100 and the plenum chamber may be constructed from a single piece of homogenous material, such as relatively flexible material, which may be silicone rubber. In further examples, the seal-forming structure 3100 and the plenum chamber 3200 may be formed in one piece but the material of each may have a different mechanical characteristic, e.g., both may be made from silicone rubber with the seal-forming structure 3100 having a lower durometer than the plenum chamber 3200. The plenum chamber 3200 may also include tube connectors 3202 to connect to corresponding tubes, as shown in Figs. 43-47, to receive pressurised air.

### 5.3.3 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone. The first tie may be provided, for example, as part of a patient interface that comprises a cradle cushion, nasal pillows, nasal cushion, full-face cushion or an oronasal cushion. For example, as shown in Figs. 43-48, the positioning and stabilising structure 3300 comprises a first tie in the form of tubes 3350 which lie over the top of the patient's head.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping. As shown in Figs. 43-47, the positioning and stabilising structure 3300 comprises a strap 3310 that is bendable. The strap 3310 may be considered a backstrap. The strap 3310 is sufficiently flexible to pass around the back of the patient's head and lie comfortably against the patient's head, even when under tension in use.

Fig. 48 also has straps, but instead includes upper straps 3302 and lower straps 3303 that extend from a rear portion 3304. The upper straps 3302 connect releasably to corresponding tubes 3350 and clips 3301 connect the lower straps 3303 to corresponding conduit connectors 3800 that connect to the plenum chamber 3200.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

### 5.3.3.1 Headgear tubing

In some forms of the present technology, the positioning and stabilising structure 3300 comprises one or more tubes 3350 that deliver pressurised air received from a conduit forming part of the air circuit 4170 from the RPT device to the patient's airways, for example through the plenum chamber 3200 and seal-forming structure 3100. In the form of the present technology illustrated in Figs. 43-48, the positioning and stabilising structure 3300 comprises two tubes 3350 that deliver air to the seal-forming structure 3100 from the air circuit 4170. The tubes 3350 are an integral part of the positioning and stabilising structure 3300 of patient interface 3000 to position and stabilise the seal-forming structure 3100 of the patient interface to the appropriate part of the patient's face (for example, the nose and/or mouth). This allows the conduit of air circuit 4170 providing the flow of pressurised air to connect to a connection port 3600 of the patient interface in a position other than in front of the patient's face which may be unsightly to some people. While a pair of tubes 3350 have some advantages (described below), in some examples, the positioning and stabilising structure 3300 comprises only a single tube 3350 configured to overlie the patient's head on one side. A strap or other stabilising component may be provided to the other side of the patient's head between the top end of the single tube 3350 and the seal-forming structure 3100, to provide balanced forces on the seal-forming structure 3100.

Since air can be contained and passed through headgear tubing 3350 in order to deliver pressurised air from the air circuit 4170 to the patient's airways, the positioning and stabilising structure 3300 may be described as being inflatable. It will be understood that an inflatable positioning and stabilising structure 3300 does not require all components of the positioning and stabilising structure 3300 to be inflatable. For example, in the example shown in Figs. 43-48, the positioning and stabilising structure 3300 comprises the headgear tubing 3350, which is inflatable, and the strap 3310, which is not inflatable.

In certain forms of the present technology, the patient interface 3000 may comprise a connection port 3600 located proximal a top, side or rear portion of a patient's head. For example, in the form of the present technology illustrated in Figs. 43-48, the connection port 3600 is located on top of the patient's head. In this example the patient interface 3000 comprises a conduit connector 3610 to which the connection port 3600 is provided. The conduit connector 3610 may swivel with respect to the positioning and stabilising structure 3300 and order to decouple movement of a conduit connected to the connection port 3600 from the positioning and stabilising structure 3300. Additionally, or alternatively, a conduit connected to the connection port 3600 may swivel with respect to the conduit connector 3610. In the illustrated example, conduit connector 3610 comprises a swivelling conduit connector to which a conduit of the air circuit 4170 is able to connect such that the conduit can rotate about its longitudinal axis with respect to the conduit connector 3610.

Patient interfaces in which the connection port is not positioned in front of the patient's face may be advantageous as some patients find a conduit that connects to a patient interface in front of the face to be unsightly and obtrusive. For example, a conduit connecting to a patient interface in front of the face may be prone to being tangled up in bedclothes or bed linen, particularly if the conduit extends downwardly from the patient interface in use. Forms of the technology with a patient interface with a connection port positioned proximate the top of the patient's head in use may make it easier or more comfortable for a patient to lie or sleep in one or more of the following positions: in a side or lateral position; in a supine position (i.e. on their back, facing generally upwards); and in a prone position (i.e. on their front, facing generally downwards). Moreover, connecting a conduit to the front of a patient interface may exacerbate a problem known as tube drag, wherein the conduit may provide an undesired drag force upon the patient interface thereby causing dislodgement away from the face.

In the form of the present technology illustrated in Figs. 43-48, the positioning and stabilising structure 3300 comprises two tubes 3350, each tube 3350 being positioned in use on a different side of the patient's head and extending across the respective cheek region, above the respective ear (superior to the otobasion superior on the patient's head) to the conduit connector 3610 on top of the head of the patient 1000. This form of technology may be advantageous because, if a patient sleeps with their head on its side and one of the tubes is compressed to block or partially block the flow of gas along the tube, the other tube remains open to supply pressurised gas to the patient. In other examples of the technology, the patient interface 3000 may comprise a different number of tubes, for example one tube, or three or more tubes. In one example in which the patient interface has one tube 3350, the single tube 3350 is positioned on one side of the patient's head in use (e.g. across one cheek region) and a strap forms part of the positioning and stabilising structure 3300 and is positioned on the other side of the patient's head in use (e.g. across the other region) to assist in securing the patient interface 3000 on the patient's head.

In the form of the technology shown in Figs. 43-48, the two tubes 3350 are fluidly connected at their upper ends to each other and to connection port 3600. In one embodiment, the two tubes are integrally formed while in other embodiments the tubes are separate components that are connected together in use and may be disconnected, for example for cleaning or storage. Where separate tubes are used they may be indirectly connected together, for example each may be connected to a T-shaped conduit having two conduit arms each fluidly connectable to the tubes 3350 and a third conduit arm or opening acting as the connection port 3600 and connectable in use to the air circuit 4170. The connection port 3600 may comprise a conduit connector 3610 received in fluid connection opening 3390 at the centre of two integrally formed tubes 3350. The conduit connector 3610 may be received in a ring in the fluid connection opening 3390 and may be configured to swivel within the ring. The fluid connection opening 3390 may be also considered a connection port 3600 itself.

The tubes 3350 may be formed of a semi-rigid material such as an elastomeric material, e.g. silicone. The tubes may have a natural, preformed shape and be able to be bent or moved into another shape if a force is applied to the tubes. For example, the tubes may be generally arcuate or curved in a shape approximating the contours of a patient's head between the top of the head and the nasal or oral region.

The positioning and stabilising structure 3300 in some examples may comprise sleeves 3364 around the tubes 3350. For example, as shown in 43-47, sleeves 3364 are provided to the non-extendable tube sections. In some examples, the patient interface 3000 may not comprise sleeves 3364 and in other examples the patient interface 3000 may comprise sleeves 3364 that cover more, or all, of the tubes 3350. The sleeves 3364 may be formed to fit to the curved shape of the tubes 3350. In some examples, the sleeves 3364 are formed from a smooth fabric. The sleeves 3364 may be more comfortable against the patient's face than the tube 3350 without any covering.

As described in US Patent No. 6,044,844, the tubes 3350 may be crush resistant to avoid the flow of breathable gas through the tubes if either is crushed during use, for example if it is squashed between a patient's face and pillow. Crush resistant tubes may not be necessary in all cases as the pressurised gas in the tubes may act as a splint to prevent or at least restrict crushing of the tubes 3350 during use. A crush resistant tube may be advantageous where only a single tube 3350 is present as if the single tube becomes blocked during use the flow of gas would be restricted and therapy will stop or reduce in efficacy.

In certain forms of the technology, one or more portions of the tubes 3350 may be rigidised by one or more rigidising or stiffening elements. Examples of rigidising elements include: sections of the tubes 3350 that are comparatively thicker than other sections; sections of the tubes 3350 that are formed from a material that is comparatively more rigid that the material forming other sections; and a rigid member attached to the inside, outside or embedded in a section of tube. The use of such rigidising elements helps to control how the positioning and stabilising structure 3300 will function in use, for example where the tubes 3350 is more likely to deform if forces are applied to them and where the shape of the tubes 3350 is more likely to be maintained if forces are applied. The selection of where such rigidising elements are positioned in the tubes 3350 can therefore help to promote comfort when the patient interface 3000 is worn and can help to maintain a good seal at the seal-forming structure 3100 during use. Rigidising or stiffening elements may be in positioning and stabilising structures 3300 which are configured to support relatively heavy seal-forming structures such as full face or oro-nasal cushion assemblies.

The tubes 3350 in the form of the technology shown in Figs. 43-48 have a length of between 15 and 30cm each, for example between 20 and 27cm each. In one example each of the tubes are around 26cm long. In another example each of the tubes is around 23cm long. The length of the tubes is selected to be appropriate for the dimensions of the heads of typical patients, for example the distance between the region proximate the top of the head where the upper end of the tubes 3350 are situated, and the region proximate the openings to the patient's airways at which the lower end of the tubes 3350 connect to the cradle cushion module 3150 (or pillows cushion module 3160) when following a generally arcuate path down the sides of the heads and across the patient's cheek region such as is shown in Figs. 43-48. As described in more detail below, the patient interface 3000 is configured so that the length of the tubes 3350 can be varied in some forms of the technology and the above lengths may apply to the tube in a contracted, stretched or neutral state. It will be appreciated that the length of the tubes 3350 will depend on the length of other components in the patient interface 3000, for example the length of arms of a T-shaped conduit to which the upper ends of tubes 3350 connect and/or the size of the plenum chamber 3200.

### 5.3.3.1.1 Positioning of Headgear Components

Each tube 3350 may be configured to receive a flow of air from the connection port 3600 on top of the patient's head and to deliver the flow of air to the seal-forming structure at the entrance of the patient's airways. In the example of Figs. 43-48, the at least one tube 3350 extends between the seal-forming structure 3100 and the connection port 3600 across the patient's cheek region and above the patient's ear, i.e. a portion of tube 3350 that connects to the cushion module overlays a maxilla region of the patient's head in use and a portion of tube 3350 overlays a region of the patient's head superior to the otobasion superior on the patient's head. Each of the one or more tubes 3350 may also lie over the patient's sphenoid bone and/or temporal bone and either or both of the patient's frontal bone and parietal bone. The connection port 3600 and conduit connector 3610 may be located in use over the patient's parietal bone, frontal bone or the junction therebetween.

The exemplary form of the technology illustrated in Figs. 43-48 has tubes 3350 which curve around the upper part of the patient's head from the upper end of the tubes 3350 that connect to conduit connector 3610 on top of the head to the point at which the strap 3310 connects to the tubes 3350 with relatively little curvature in the sagittal plane. In between the point at which the rear headgear strap 3310 connects to the tubes 3350 and the lower ends of the tubes 3350 at which they connect with the cradle cushion module 3150 in front of the patient's airways under the nose, the tubes 3350 curve forwards between the patient's ears and eyes and across the cheek region.

The degree to which the patient interface 3000 fits an individual patient can be altered by varying the length of the tubes 3350 and, alternatively or additionally, by altering the position of the patient interface 3000 or portions thereof on the patient's head. For example, a patient interface 3000 having tubes 3350 of a certain length can be adjusted to better fit a patient by moving portions of the positioning and stabilising structure 3300 in the posterior or anterior direction on the patient's head. For example, positioning the junction of the tubes 3350 above the patient's head further forward (i.e. in the anterior direction) enables a patient interface 3000 having tubes 3350 of a certain length to fit a larger head than if the junction of the tubes 3350 is positioned further backward (i.e. in the posterior direction). In most patient, if the junction of the tubes 3350 is positioned forwardly, the superior portions of the tubes 3350 lie over a smaller portion of the patient's head than if the junction of the tube 3350 is positioned rearwardly.

In certain forms of the present technology the patient interface 3000 is configured such that the connection port 3600 can be positioned in a range of positions across the top of the patient's head so that the patient interface 3000 can be positioned as appropriate for the comfort or fit of an individual patient. One way this can be achieved so that the seal-forming structure 3100 forms an effective seal with the patient's face irrespective of the position of the connection port 3600 on the patient's head is to de-couple movement of the upper portion of the patient interface 3000 from the lower portion of the patient interface 3000. Such de-coupling can be achieved using, for example, mechanisms that allow parts of the headgear tubes 3350 to easily move or flex relative to other parts of the patient interface 3000. Such mechanisms will be described below.

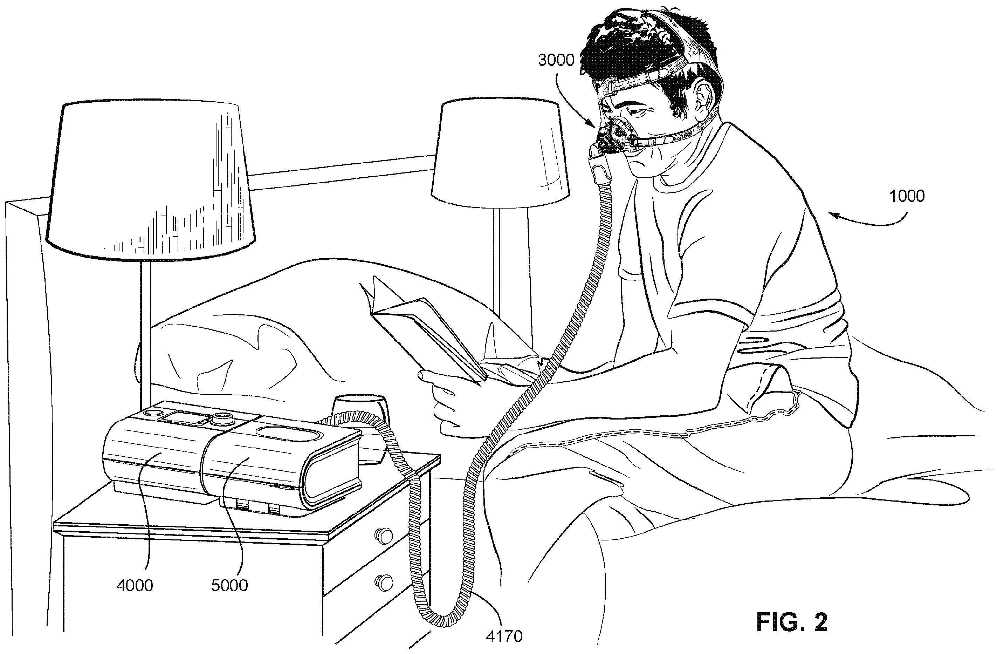

In a certain form of the present technology, the patient interface 3000 is configured such that the connection port 3600 is positioned approximately at a top point of the patient's head. The connection port 3600 may be positioned in the sagittal plane and aligned with the otobasion superior points in a plane parallel to the coronal plane. The otobasion superior points are identified in Fig. 2D. As will be described below, in some forms of the technology, the positioning and stabilising structure 3300 is configured to be worn in different positions, with the effect that the connection port 3600 may be positioned proximate the top of the patient's head in the sagittal plane up to around 20mm forward or 20mm rearward of the otobasion superior points.

In some examples of the present technology, the connection port 3600 may be positioned in the sagittal plane and aligned with a junction between the frontal bone and the parietal bones. The connection port 3600 may be positioned approximately over the junction of the coronal suture and the sagittal suture. In this configuration, the superior portions of the tubes 3350 may lie over and/or along a portion of the coronal suture. However, as mentioned above the patient has the ability to move the connection port 3600 anteriorly or posteriorly in order to adjust the fit of the patient interface 3000.

An advantage provided by the tubes 3350 overlying the patient's head slightly anterior to the superior-most point (e.g. at or proximate the coronal suture) is that the risk of the tubes 3350 riding in a posterior direction in use may be reduced. In many patients there may be a recess or "divot" where the coronal suture meets the sagittal suture. The positioning and stabilising structure 3300 may be particularly stable when tubes 3350 lie within this divot. Accordingly, in some examples the tubes 3350 are configured with appropriate curvature and/or ability to curve in order to lie over the coronal suture.

As described above, in some examples of the present technology the patient interface 3000 comprises a seal-forming structure 3100 in the form of a cradle cushion which lies generally under the nose and seals to an inferior periphery of the nose. The positioning and stabilising structure 3300 may be structured and arranged to pull the seal-forming structure 3100 into the patient's face under the nose with a sealing force vector that has a posterior and superior direction (e.g. a posterosuperior direction). A sealing force vector with a posterosuperior direction may facilitate the seal-forming structure 3100 forming a good seal to both the inferior periphery of the patient's nose and the anterior-facing surfaces of the patient's face on either side of the patient's nose and the upper lip.

For example, in many examples the positioning and stabilising structure 3300 may be configured such that the superior portions of the tubes 3350 lie across the patient's head slightly anterior to a superior-most point. For some patients this may result in the tubes 3350 being angled slightly anteriorly rather than aligned vertically (e.g. in the coronal plane) in order to lie within a slight recess at or proximate the coronal suture of the skull. In such an example, the tension in the strap 3310 could be adjusted by the patient to balance the forces and achieve the optimal sealing force vector.

In certain examples of the present technology, the tubes 3350 are configured to receive the strap 3310 at the locations superior to and proximate the patient's ears. If the strap 3310 connects to the tubes 3350 to high with respect to the patient's head, the strap 3310 may have a tendency to ride up the back of the patient's head. Additionally, the strap 3310 could form too large of an angle with respect to the superior portions of the headgear tubes 3350, resulting in the necessity for the patient to tighten the strap 3310 excessively, which could result in both excessive tension in the positioning and stabilising structure 3300 and make the strap 3310 more likely to ride up the back of the patient's head. Accordingly, it is advantageous for the connection between the strap 3310 and the tubes 3350 to be provided as low as possible but spaced from the top of the patient's ear sufficiently that upon tightening of the strap 3310, the tubes 3350 are not pulled into contact with the patient's ears.

### 5.3.3.1.2 Headgear Tube Fluid Connections

The two tubes 3350 are fluidly connected at their inferior ends to the plenum chamber 3200. In the examples of Figs. 43-48, the tubes 3350 form a fluid connection with the cradle cushion module 3150 and seal-forming structure 3100. In certain forms of the technology, the connection between the tubes 3350 and the cradle cushion module 3150 is achieved by connection of two rigid components so that the patient can easily connect the two components together in a reliable manner. The tactile feedback of a 're-assuring click' or like sound may be easy for a patient to use or for a patient to know that the tube has been correctly connected to the cradle cushion module 3150. In one form, the tubes 3350 are formed from silicone and the lower end of each of the silicone tubes 3350 is overmolded to a rigid connector made, for example, from polypropylene, polycarbonate, nylon or the like. The rigid connector may comprise a male mating feature configured to connect to a female mating feature on the cradle cushion module 3150. Alternatively, the rigid connector may comprise a female mating feature configured to connect to a male mating feature on the cradle cushion module 3150. The same manner of connection by which the tubes 3350 are connected to the cradle cushion module 3150 may also be applied to the connection between the tubes 3350 and the nasal cradle cushion module 3150, or another plenum chamber 3200 or seal-forming structure 3100.

In another embodiment a compression seal is used to connect each tube 3350 to the cradle cushion module 3150. For example, a resiliently flexible (e.g. silicone) tube 3350 without the rigid connector may need to be squeezed slightly to reduce its diameter so that it can be jammed into a port in the plenum chamber 3200 and the inherent resilience of the silicone pushes the tube 3350 outwards to seal the tube 3350 in the port in an air-tight manner. In a hard-to-hard type engagement between the tube 3350 and port, a pressure activated seal such as a peripheral sealing flange may be used. When pressurised gas is supplied through the tubes 3350 the sealing flange is urged against the join between the tubes and the inner circumferential surface of the port of the plenum chamber 3200 to enhance the seal between them. If the port is soft and a rigid connector is provided to the tube 3350, the pressure activated seal as described earlier may also be used to ensure the connection is air-tight. In another example, each tube 3350 is formed from a resiliently flexible (e.g. silicone) material which is over moulded to a rigid connector such that the resiliently flexible material fits over the rigid connector and itself functions as a gasket to seal the connection between the tube 3350 and the cradle cushion module 3150 around a periphery of an air flow passage from the tube 3350 into the plenum chamber 3200 of the cradle cushion module 3150.

Similar connection mechanisms may be used to fluidly connect the tubes 3350 with a T-shaped top member defining the connection port 3600 or connectable to the connection port 3600 in some forms of the technology. In one embodiment, a swivel conduit connector connected at the connection port 3600 is rotatable in order to drive a port size adjustment mechanism that decreases or increases the size of the ports into which tubes 3350 are inserted in order to improve the fit of the tubes through an increase or decrease of compressive forces and to reduce unintended leakage.

### 5.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO2 by the patient while maintaining the therapeutic pressure in the plenum chamber in use.

One form of vent 3400 in accordance with the present technology comprises a plurality of holes, for example. about 20 to about 80 holes, or about 40 to about 60 holes, or about 45 to about 55 holes.

The vent 3400 may be located in the plenum chamber 3200, as shown in Figs. 43-48.

### 5.3.4.1 Conduit Connector with Flow-Regulating Vent

Figs. 49-59C show examples of a conduit connector and vent assembly 3605 according to exemplary embodiments of the present invention, that may be connected to a conduit connector receiving hole 3306 in a central section 3305 between the tubes 3350 of the positioning and stabilising structure 3300 of any of the examples of patient interface 3000 shown in Figs. 43-48. In the example shown in Figs. 43-45 and in the example shown in Figs. 46 and 47, the seal-forming structure 3100 seals around and directs pressurized air into only the patient's nares during therapy, and therefore the mouth is left uncovered. In the example shown in Fig. 48, the seal-forming structure 3100 seals around and directs pressurized air into the patient's nose and mouth such that an anti-asphyxia valve may also be included to allow the patient to breathe through the mouth in the absence of pressurized air. In each of these examples of Figs. 43-48, the plenum chamber 3200 may include the vent 3400, e.g., on the plenum chamber, that functions in conjunction with the conduit connector and vent assembly 3605 to vent exhaled gas to atmosphere.

According to the invention, the conduit connector and vent assembly 3605 includes a conduit connector 3610 and a vent assembly 3606. The conduit connector 3610 in these examples may be an elbow, and in further alternative examples the conduit connector 3610 may be straight from end to end. The conduit connector 3610 may have an outlet end 3611 that is formed on an outlet portion 3608 and is inserted into the conduit connector receiving hole 3306 to removably connect the conduit connector and vent assembly 3605 to the central section 3305. The conduit connector and vent assembly 3605 may be rotatable 360° when connected to the central section 3305. The conduit connector and vent assembly 3605 may connect to the central section 3305 with a snap-fit. The conduit connector and vent assembly 3605 may be removably connected to the central section 3305. The conduit connector 3610 may also include an inlet end 3612 that is formed on an inlet portion 3609 and that may be connected to an air circuit 4170. The inlet end 3612 may include one or more bayonet connectors 3613 to form a bayonet connection with the air circuit 4170, which may further be non-rotatable. Alternatively, the inlet end 3612 may include a swivel that is rotatable 360°. In either example, the air circuit 4170 may be removably connected to the inlet end 3612.

The conduit connector 3610 includes a vent assembly receiving hole 3614 and a vent assembly 3606 is positioned within the vent assembly receiving hole 3614. The vent assembly receiving hole 3614 may extend through the inlet portion 3609 of the conduit connector 3610 in a radial direction. The vent assembly 3606 includes a base 3616, a flap 3622, and a cover 3624. The base 3616 may be positioned at or approximately at the bottom of the vent assembly receiving hole 3614. The base 3616 supports the flap 3622 when, as will be explained below, pressure within the conduit connector and vent assembly 3605 is less than, equal to, or just slightly (e.g., up to approximately 4 cmH₂O) above ambient air pressure. The cover 3624 may be positioned at or approximately at the top of the vent assembly receiving hole 3614. As pressure is increased within the conduit connector and vent assembly 3605, the flap 3622 is forced upward towards and against the cover 3624.

The conduit connector 3610, the base 3616, and the cover 3624 may each be constructed from a relatively rigid material, such as a plastic material. The conduit connector 3610, the base 3616, and the cover 3624 may each be constructed from a material having at least property different from the others. The conduit connector 3610, the base 3616, and the cover 3624 may each be constructed as separate components that are assembled together. Alternatively, the conduit connector 3610 may be formed in one piece with one of the base 3616 and the cover 3624 and then the other of the base 3616 and the cover 3624 may be attached to the conduit connector 3610. The base 3616 and/or the cover 3624 may be attached to the conduit connector 3610, when one or both are separate components, with one or more of a friction fit, a snap-fit, and an adhesive. When the vent assembly 3606 is assembled in the vent assembly receiving hole 3614, the flap 3622 may be positioned between the base 3616 and the cover 3624 such that the base 3616 and the cover 3624 retain the flap 3622 between them. The flap 3622 may be freely movable between the base 3616 and the cover 3624, e.g., due to gravity and/or pressure changes within the conduit connector and vent assembly 3605.

The conduit connector 3610 may have one or more radial protrusions 3615 extending radially inward into the vent assembly receiving hole 3614. Each of the base 3616 and the cover 3624 may have a corresponding number of radial base notches 3619 and radial cover notches 3625, respectively, to engage the radial protrusions 3615. Engagement of the radial protrusions 3615, the radial base notches 3619, and the radial cover notches 3625 may prevent rotation of the base 3616 and the cover 3624 relative to the conduit connector 3610 when assembled. In an alternative example, the protrusions and notches may be reversed such that the conduit connector 3610 has one or more notches and the base 3616 and the cover 3624 each have a corresponding number of protrusions. The radial protrusions 3615, the radial base notches 3619, and the radial cover notches 3625, or vice versa, may all extend axially relative to the vent assembly receiving hole 3614 so that the base 3616 and the cover 3624 can be inserted axially into the vent assembly receiving hole 3614. The vent assembly receiving hole 3614 may have a slight taper into the interior of the conduit connector 3610 and the base 3616 may be shaped and dimensioned so that it cannot be inserted past the bottom of the vent assembly receiving hole 3614. The cover 3624 may then be prevented from being inserted further by contact with the base 3616.

The base 3616 may have a plurality of base holes 3618 to allow vent flow to pass therethrough, as will be explained below. The base holes 3618 may be formed between a plurality of spokes 3617. The spokes 3617 may support the flap 3622 when it is in contact with the base 3616. The base 3616 may also have a post 3620 to contact the cover 3624 and maintain an axial gap 3630 between the cover 3624 and the base 3616. Alternatively, the cover 3624 may have the post 3620 extending to contact the base 3616. The base 3616 may also have one or more axial protrusions 3621 to contact the cover 3624 and maintain the axial gap 3630 between the cover 3624 and the base 3616. The flap 3622 may be located within the axial gap 3630. The cover 3624 may have one or more axial notches 3626 to receive corresponding axial protrusions 3621. Alternatively, the cover 3624 may have one or more axial protrusions 3621 extending to contact the base 3616, and the base 3616 may have one or more axial notches 3626 to receive corresponding axial protrusions 3621.

The flap 3622 may be constructed of a relatively flexible material such as rubber or silicone rubber. The flap 3622 may have a flap hole 3623. The flap 3622 may have an annulus shape. The flap 3622 may have a circular perimeter and a circular inner perimeter. The flap 3622 may have a constant thickness throughout its diameter. Alternatively, the flap 3622 may increase or decrease in thickness between its inner and outer perimeter. The outer diameter of the flap 3622 may be less than the inner diameter of radially adjacent portions of the cover 3624 or radially adjacent portions of the conduit connector 3610 surrounding the vent assembly receiving hole 3614 such that there is an outer radial gap 3631 therebetween. Also, the diameter of the flap hole 3623 may be less than the diameter of the post 3620 such that there is an inner radial gap 3632 therebetween. The flap 3622 may also be able to move radially within the vent assembly 3606, and as the flap 3622 moves in one radial direction the outer radial gap 3631 decreases on that side of the flap 3622 while increasing by an equal amount on the opposite side of the flap 3622 such that the overall area of the outer radial gap 3631 remains the same regardless of the flap's 3622 radial position within the vent assembly 3606.

The cover 3624 may have one or more cover holes 3627 formed between a central portion 3628 and an annular portion 3629. In the depicted examples, the cover holes 3627 are shaped as two semi-circular slots. The cover holes 3627 may also be formed as a plurality of circular holes. The vent assembly 3606, e.g., the cover 3624 in particular, may include a porous, diffuser material to diffuse the vent flow traveling through the cover holes 3627 to further reduce noise and flow jetting, if desired.

### 5.3.4.2 Operation of Conduit Connector with Flow-Regulating Vent

When the conduit connector and vent assembly 3605 is attached to the central section 3305 of the positioning and stabilising structure 3300, as described above, the vent assembly 3606 may allow a vent flow of pressurized air to exit to atmosphere. As is also explained above, the patient interface 3000 may include another vent 3400, on the plenum chamber 3200 for example. Thus, the vent 3400 and the vent assembly 3606 may provide two separate paths for pressurized air to be vented to atmosphere. The vent 3400, as described above, may be passive in that it has one or more holes that are always open such that pressurized air can continuously vent to atmosphere through the vent 3400 during all phases of respiration and without regard to the pressure within the patient interface 3000. The vent assembly 3606, as will be explained below, may be active or the vent flow therethrough may be variable depending on the pressure within the conduit connector 3610.

Figs. 57 and 58 show the flap 3622 in two different positions depending on pressure within the conduit connector. In Fig. 57, the flap 3622 is positioned against the base 3616, e.g., due to gravity, and the arrows 8002 indicate pressure within the conduit connector 3610 acting against the flap 3622. In Fig. 57, the pressure is too low to lift the flap 3622 from the base 3616, but it should be understood that the flap 3622 would be in this position if there is no pressure, e.g., when there is no incoming flow of pressurized air from the air circuit 4170. The pressure at which the flap 3622 begins to be lifted off the base 3616 can be tuned by various factors described below, and in one example the flap 3622 may be constructed such that it remains against the base 3616 at least until the pressure within the conduit connector 3610 is at 4 cmH₂O. In other examples, the pressure may be any value from 1 cmH₂O to 8 cmH₂O at which the flap 3622 begins to be displaced from the base 3616. Additionally, when the flap 3622 is in the position shown in Fig. 57, the vent flow of pressurized air, indicated by arrows 8000, can pass through the base holes 3618, through the flap hole 3623, around the outer perimeter of the flap 3622, through the cover holes 3627, and then to atmosphere.

Fig. 58 shows the flap 3622 when the pressure (arrows 8002) within the conduit connector 3610 has reached a predetermined level to urge the flap 3622 against the cover 3624, thereby occluding the cover holes 3627 and preventing any vent flow from passing to atmosphere through the vent assembly 3606. Accordingly, no vent flow arrows 8000 are shown in Fig. 58. Additionally, because the flap 3622 may be flexible, as described above, it may be deformed against the central portion 3628 and the annular portion 3629 of the cover 3624. As can be seen in Fig. 58, the annular portion 3629 extends deeper into the vent assembly receiving hole 3614 than the central portion 3628 such that central portion 3628 and the annular portion 3629 are offset. The opposite offset may be formed as well such that the central portion 3628 extends deeper into the vent assembly receiving hole 3614 than the annular portion 3629. In either arrangement, when the flap 3622 is contacting one of the central portion 3628 and the annular portion 3629, but not the other, vent flow may still be allowed to pass through to the cover holes 3627 and on to atmosphere.

The effect of the offset can be seen by comparing Figs. 59A-59C. Fig. 59A shows the flap 3622 in a fully open position that corresponds to Fig. 58A, and Fig. 59C shows the flap 3622 in a fully closed position that corresponds to Fig. 58C. Fig. 58B shows the flap 3622 in an intermediate position engaged with the annular portion 3629 but not contacting the central portion 3628 of the cover 3624. In the Fig. 59B position, the flap 3622 has not completely closed off the cover holes 3627 - vent flow (arrows 8000) is not passing around the perimeter of the flap 3622 to reach the cover holes 3627, but vent flow (arrows 8000) is still able to pass through the flap hole 3623 to reach the cover holes 3627 and then exit to atmosphere. The vent assembly 3606 may be understood to operate so that all pressurized air that travels through the flap hole 3623 exits to atmosphere through the cover 3624 because even when the flap 3622 occupies the fully open position of Fig. 59A or the intermediate position of Fig. 59B, all of the vent flow (arrows 8000) passing through the flap hole 3623 then passes through the cover holes 3627 to atmosphere. However, when the flap 3622 occupies the fully closed position of Fig. 59C, no vent flow passes through the flap hole 3623, and therefore no vent flow can reach the cover holes 3627. In the fully closed position of Fig. 59C, the flap 3622 may prevent any vent flow from passing through the vent assembly 3606 when the flap 3622 is in contact with both the central portion 3628 and the annular portion 3629.

In one example, when the pressure is between approximately 0 cmH₂O and approximately 4 cmH₂O, the flap 3622 is positioned as shown in Fig. 59A, as the pressure increases from approximately 4 cmH₂O to approximately 10 cmH₂O, the flap 3622 moves into the position shown in Fig. 59B, and as the pressure increases from approximately 10 cmH₂O to approximately 20 cmH₂O, the flap 3622 moves into the position shown in Fig. 59C. These ranges may be adjusted depending on the desired amount of supplemental vent flow provided by the vent assembly 3606 at different therapy pressures. The vent assembly 3606 may designed so that the flap 3622 does not completely occlude the cover holes 3627 until the pressure reaches approximately 20 cmH₂O. The vent assembly 3606 may also designed so that the flap 3622 does not cause any reduction in vent flow through the cover holes 3627 until the pressure reaches approximately 4 cmH₂O.

The vent flow rate that the vent assembly 3606 permits may be affected by the dimensions of its components, as well as by the flexibility of the flap 3622. The area of the base holes 3618 may affect the flow rate that is allowed to enter the vent assembly 3606. The outer radial gap 3631 and the inner radial gap 3632 may each affect the amount of flow that can pass around the flap 3622. The outer radial gap 3631 can be changed by varying one or both of the flap's 3622 outer diameter and the vent assembly receiving hole's 3614 diameter. The inner radial gap 3632 can be changed by varying one or both of the flap hole's 3623 diameter and the post's 3620 diameter. The flap's 3622 flexibility can be changed by varying one or both of its thickness and its material composition (i.e.. more or less elastic material, low or high durometer). The size and number of the cover holes 3627 can also affect the vent flow rate that can escape to atmosphere through the vent assembly 3606 when not occluded by the flap 3622. These factors can be tuned to change the pressure at which the flap 3622 moves off its fully open position (e.g., Fig. 59A) into a partially open/closed or intermediate position (e.g., Fig. 59B) and then into the fully closed position (e.g., Fig. 59C), as well as when the flap 3622 moves in the opposite order. These factors can also be tuned to vary the vent flow rate through the vent assembly 3606 when the flap 3622 is in each of these different positions.

When coupled with the vent 3400, e.g., in the plenum chamber 3200, that is always open, the vent assembly 3606 may allow the total or combined vent flow of both vents to increase less drastically with pressure, as compared with having a vent in the conduit connector 3610 that is always open in combination with the vent 3400, e.g., in the plenum chamber 3200, that is always open as well. As the pressure within the conduit connector 3610 increases, the flap 3622 progressively occludes the cover holes 3627 thereby reducing the portion of the total vent flow exiting through the vent assembly 3606 while the passive vent 3400 functions to vent pressurized air to atmosphere independent of the pressure within the patient interface 3000. Although the vent flow rate through the vent 3400 may increase due to increased pressure, the fully open state of its holes remains unchanged, but the progressive occlusion of the cover holes 3627 by the flap 3622 and the associated reduction in vent flow through the vent assembly 3606 offsets the increased vent flow through the vent 3400. This may result in total or combined vent flow curve that is relatively flat as pressure increases.

As explained above, the vent assembly receiving hole 3614 may be positioned on the inlet portion 3609 of the conduit connector 3610. This arrangement may allow pressurized air to travel through the conduit connector 3610 from the air circuit 4170 in a direction that is approximately parallel to the flap's 3622 diameter. By orienting, the axes of the vent assembly 3606 and the vent assembly receiving hole 3614 approximately perpendicular to the axis of the inlet portion 3609, the incoming flow of pressurized air is not directed straight at the flap 3622. If the incoming flow of pressurized air is directed straight at the flap 3622, the incoming flow of pressurized air could cause the flap 3622 to move into the closed position prematurely, i.e., when before the pressure in the conduit connector 3610 reaches the intended threshold. By orienting the flap 3622 in this manner, the flap 3622 is pressure-activated, rather than flow-activated.

Additionally, while the examples described above include a passive vent 3400 on the patient interface 3000, e.g., the plenum chamber 3200, and the active vent, in the form of the vent assembly 3606, is located on the conduit connector 3610, the active and passive vents may be reversed with the vent assembly 3606 located on the plenum chamber 3200 and the vent 3400 located on the conduit connector 3610, for example. In still further examples, both the active vent and the passive vent may be positioned on the plenum chamber 3200. In still further examples, the active vent may be located on the conduit connector 3610 or the plenum chamber 3200 and the passive vent 3400 may be positioned on each of the gas delivery tubes 3350. In still further examples of the Fig. 48 arrangement, the passive vent or the active vent may be located on each of the conduit connectors 3800 and the other of the passive vent or the active vent may be located on one or more of the plenum chamber 3200, the gas delivery tubes 3350, and the conduit connector 3610. The operational aspects described above would be unchanged in all of these alternatives.

### 5.3.4.3 Conduit Connector Vent With Flap And Bump

Figs. 60-65 show an example of a conduit connector 3605 for connecting the air circuit 4170 to the patient interface 3000 to direct pressurized air into the patient interface 3000 from the air circuit 4170. The conduit connector 3605 includes a body portion 3640 having an inlet end 3612 that may be connected (e.g., removably) to the air circuit 4170 and an outlet end 3611 that may be connected (e.g., removably) to the patient interface 3000. The conduit connector 3605 includes one or more holes 3641 extending through the body portion 3640 at a position between the inlet end 3612 and the outlet end 3611 to allow air flowing through the conduit connector 3605 to pass to atmosphere. The conduit connector 3605 includes a flap 3645 connected to the body portion 3640. The flap 3645 is structured to cover the holes 3641 in a closed position so that air flowing through the conduit connector 3605 cannot pass to atmosphere. The flap 3645 is also structured to not cover the holes 3641 in an open position to allow air flowing through the conduit connector 3605 to pass to atmosphere. The flap 3645 is also freely movable between the closed position and the open position. The flap 3645 also includes a bump 3646 positioned between the body portion 3605 and the flap 3645 such that in the closed position of the flap 3645 the flap 3645 is flush against the body portion 3640.

The bump 3646 may be positioned on the flap 3645 and may face the body portion 3640. Although the depicted example shows the bump 3646 formed on the flap 3645, the bump 3646 may also be formed on the body portion 3640, in an alternative example, the bump 3646 may be positioned on the body portion 3640 at a location so that it contacts the flap 3645 in the closed position and may face the flap 3645. The flap 3645 or the body portion 3640 may include a plurality of bumps 3646. The bump 3646 may also be positioned proximal to a free end of the flap 3645.

The flap 3645 may also include a hinge 3643 that allows the flap 3645 to be freely movable between the closed position and the open position. The hinge 3643 may be formed by a reduced material thickness. The bump 3646 may be positioned on the flap 3645 opposite the hinge 3643.

The flap 3645 may also include a tab 3642 fixed to the body portion 3640. The body portion 3640 may include a tab receiving hole 3644 that receives the tab 3642 to fix the flap 3645 to the body portion 3640. The hinge 3643 may be positioned between the tab 3642 and the flap 3645. The flap 3645, the hinge 3643, and the tab 3642 may be constructed from single homogeneous piece of material. The hinge 3643 may be constructed from a material that is more flexible than the flap 3645 and the tab 3642. The tab 3642 may be permanently connected to the body portion 3640. The tab 3642 may be overmolded to the body portion 3640. The body portion 3640 may be constructed from a relatively rigid material.

The hinge 3643 may be biased such that the flap 3645 extends into a flow path through the body portion 3640 in an undeformed state of the hinge 3643. The hinge 3643 may be biased such that the flap 3645 is not in the closed position in an undeformed state of the hinge 3643. The hinge 3643 may be biased such that the flap 3645 does not cover the holes 3641 in an undeformed state of the hinge 3643. The flap 3645 may be positioned to be impacted by an incoming flow of air through the inlet end 3612.

The holes 3641 may be positioned on the body portion 3640 such that a longitudinal axis of each of the holes 3641 is approximately perpendicular to a longitudinal axis of the inlet end 3612. The holes 3641 may be positioned on the body portion 3640 such that a longitudinal axis of the holes 3641 is approximately parallel to a longitudinal axis of the outlet end 3611.

The conduit connector 3605 may be an elbow having a bend between the inlet end 3612 and the outlet end 3611.

The conduit connector 3605 may also include a swivel 3647 positioned at the inlet end 3612 and may be removably connected to the air circuit 4170.

The conduit connector 3605 may be used with any of the patient interfaces 3000 shown in Figs. 43-48, as described above. The conduit connector 3605 may be rotatably and releasably connected to the central section 3305. The plenum chamber 3200 may also include a passive vent 3400 to allow exhaled gases to pass to ambient independent of therapeutic pressure and throughout the patient's respiratory cycle.

### 5.3.4.4 Conduit Connector Vent With Flap And Surrounded By Holes

Figs. 87-92 show examples of a conduit connector 3605 for connecting the air circuit 4170 to a patient interface 3000 to direct pressurized air into the patient interface 3000 from the air circuit 4170. The conduit connector 3605 includes a body portion 3640 has an inlet end 3612 connected (e.g., removably) to the air circuit 4170 and an outlet end 3611 connected (e.g., removably) to the patient interface 3000. The body portion 3640 includes a tab receiving hole 3644. A plurality of holes 3641 extend through the body portion 3640 at a position between the inlet end 3612 and the outlet end 3611 to allow air flowing through the conduit connector 3605 to pass to atmosphere. The plurality of holes 3641 surround the tab receiving hole 3644. A flap 3645 has a tab 3642 connected to the body portion 3640 at the tab receiving hole 3644. The flap 3645 is structured to cover the holes 3641 in a closed position so that air flowing through the conduit connector 3605 cannot pass to atmosphere. The flap 3645 is structured to not cover the holes 3641 in an open position to allow air flowing through the conduit connector 3605 to pass to atmosphere. The flap 3645 is freely movable between the closed position and the open position.

The flap 3645 and the tab 3642 may be constructed from single homogeneous piece of material. The tab 3642 may be permanently connected to the hinge 3643. The tab 3642 may be overmolded to the body portion 3640.

The flap 3645 may be biased from the tab 3642 such that the flap 3645 is not in the closed position in an undeformed state. The flap 3645 may be biased from the tab 3642 such that the flap 3645 does not cover the holes 3641 in an undeformed state.

The holes 3641 may be positioned on the body portion 3640 such that a longitudinal axis of the holes 3641 is approximately perpendicular to a longitudinal axis of the inlet end 3612. The holes 3641 may be positioned on the body portion 3640 such that a longitudinal axis of the holes 3641 is approximately parallel to a longitudinal axis of the outlet end 3611. The holes 3641 may be arranged in a circle around the tab receiving hole 3644.

The conduit connector 3605 may be an elbow having a bend between the inlet end 3612 and the outlet end 3611. The conduit connector 3605 may also include a swivel 3647 positioned at the inlet end 3612 to removably connect to the air circuit 4170.

The body portion 3640 may be constructed from a relatively rigid material. The flap 3645 may be constructed from a relatively flexible material.

The flap 3645 may have a circular shape. The flap 3645 may have a radius such that an outer peripheral edge of the flap 3645 extends beyond the holes 3641 in an undeformed state. The flap 3645 may have a radius such that an outer peripheral edge of the flap 3645 contacts the body portion 3640 radially outwardly of the holes 3641 when deformed into contact with the body portion 3640.

The conduit connector 3605 may be used with any of the patient interfaces 3000 shown in Figs. 43-48, as described above. The conduit connector 3605 may be rotatably and releasably connected to the central section 3305. The plenum chamber 3200 may also include a passive vent 3400 to allow exhaled gases to pass to ambient independent of therapeutic pressure and throughout the patient's respiratory cycle.

### 5.3.4.5 Conduit Connector Vent With Membrane And Cage

Figs. 66-71 and 72-78 shows examples of a conduit connector and vent assembly 3605 for connecting the air circuit 4170 to a patient interface 3000 to direct pressurized air into the patient interface 3000 from the air circuit 4170. The conduit connector and vent assembly 3605 includes a vent ring 3650 having a plurality of vent holes 3651 positioned radially on the vent ring 3650 to allow air flowing through the conduit connector and vent assembly 3605 to pass to atmosphere. The conduit connector and vent assembly 3605 includes a connector ring 3652 connected to the vent ring 3650, the connector ring 3652 has a cage 3653 extending radially inward from the connector ring 3652. The connector ring 3652 has at least one connector 3654 to releasably connect the conduit connector and vent assembly 3605 to a patient interface 3000 or to an intermediate tube 3657. A membrane 3655 is positioned between the vent ring 3650 and the connector ring 3652 to control airflow through the vent holes 3651 to atmosphere in response to pressure or flow of pressurized air through the conduit connector and vent assembly 3605.

The connector ring 3652 may include two connectors 3654 positioned opposite one another. The connector 3654 may be flexibly joined to the connector ring 3652. The cage 3653 may be permanently connected to the connector ring 3652. The cage 3653 may be positioned on the connector ring 3652 such that the membrane 3655 cannot contact the cage 3653.

Fig. 66 shows that the conduit connector and vent assembly 3605 includes an elbow connected to the vent ring 3650. The elbow may be releasably connected to the air circuit 4170. Fig. 72 shows that the vent ring 3650 may be releasably connected to the air circuit 4170.

The vent ring 3650 may have a vent ring annular surface and the connector ring 3652 may have a connector ring annular surface that faces the vent ring annular surface with the membrane 3655 being positioned between the vent ring annular surface and the connector ring annular surface.

The conduit connector and vent assembly 3605 examples described above may be used with a patient interface 3000 having a connection port 3600 on the plenum chamber 3200. In the example of Figs. 72-78, an intermediate conduit 3657 may be releasably connected at a first end to the connector ring 3652 by the connector 3654 and releasably connected to the connection port 3600 of the plenum chamber 3200 at a second end. In the example of Figs. 66-71, the connector ring 3652 of the conduit connector and vent assembly 3605 may be releasably connected to the connection port 3600 of the plenum chamber 3200 the connector 3654. Further examples of such connection arrangements and relevant patient interfaces 3000 are disclosed in US Publication No. 2020/0368481 A1.

### 5.3.4.6 Vent With Sandwiched Membrane And Diffuser Member

Figs. 79-86 show examples of a vent assembly 3660 for discharging air to atmosphere from a patient interface 3000. The vent assembly 3660 may include a vent body 3661 having first vent holes 3662 and second vent holes 3663 and having a first side 3664 and a second side 3665. The vent assembly 3660 may include a base 3666 connected to the vent body 3661 on the first side 3664 of the vent body 3661. A membrane 3671 is positioned between the vent body 3661 and the base 3666. A cap 3667 is connected to the vent body 3661 on the second side 3665 of the vent body 3661 such that at least one vent passage 3668 is formed between the cap 3667 and the vent body 3661 to allow air to pass through the vent body 3661 to atmosphere. A diffuser member 3669 may be constructed from a porous material and positioned between the cap 3667 and the vent body 3661. The membrane 3671 may control airflow through the first vent holes 3662 to atmosphere in response to pressure or flow of pressurized air through the vent assembly 3660. The second vent holes 3663 are not covered by the membrane 3671 .

The first vent holes 3662 are positioned radially on the vent body 3661. The second vent holes 3663 are positioned radially on the vent body 3661. The second vent holes 3663 are positioned radially outward of the first vent holes 3662.

The membrane 3671 may be constructed from a flexible material. The flexible material may be silicone. Each of the vent body 3661, the base 3666, and the cap 3667 is constructed from a rigid material.

The vent body 3661 may include a ring-shaped protrusion 3670 extending from the first side 3664 of the vent body 3661 and between the first vent holes 3662 and the second vent holes 3663. The membrane 3671 may be positioned inside of the ring-shaped protrusion 3670. A diameter of the membrane 3671 may be less than an inner diameter of the ring-shaped protrusion 3670 to allow air to pass between an outer periphery of the membrane 3671 and an inner periphery of the ring-shaped protrusion 3670. A membrane hole 3673 may be formed through the membrane 3671 to allow air to pass through the membrane 3671.

The first vent holes 3662 and the second vent holes 3663 may be positioned on the vent body 3661 such that air that flows through either or both of the first vent holes 3662 and the second vent holes 3663 reaches the vent passage 3668 before being discharged to atmosphere. The first vent holes 3662 and the second vent holes 3663 may be positioned on the vent body 3661 such that air that flows through either or both of the first vent holes 3662 and the second vent holes 3663 is directed at the diffuser member 3669. The first vent holes 3662 and the second vent holes 3663 may be oriented on the vent body 3661 such that longitudinal axes through the first vent holes 3662 and the second vent holes 3663 extend to the diffuser member 3669 .

The diffuser member 3669 may be shaped and dimensioned so as not to extend completely across the vent passage 3668. The diffuser member 3669 may be shaped and dimensioned to allow air passing to atmosphere through the vent passage 3668 to bypass the diffuser member 3669. The vent body 3661 may include a diffuser member spacer 3677 to support the diffuser member 3669 against the cap 3667 such that a bypass passage for airflow is formed between the vent body 3661 and the diffuser member 3669.

Fig. 84 also depicts how air may be vented to atmosphere through the vent assembly 3660. A passive vent flow 3674 may pass through the second vent holes 3663 and into the vent passage 3668 to travel on to atmosphere as the atmospheric vent flow 3676, either first passing through the porous material of the diffuser member 3669 or bypassing the diffuser member 3669. An active vent flow 3675 may also pass through the first vent holes 3662 and into the vent passage 3668 to travel on to atmosphere as the atmospheric vent flow 3676, either first passing through the porous material of the diffuser member 3669 or bypassing the diffuser member 3669. The magnitude of the active vent flow 3675, however, may be regulated by the position of the membrane 3671 relative to the vent body 3661 and the extent to which the first vent holes 3662 are covered by the membrane 3671. The membrane 3671 may not be connected to the vent body 3661 so as to be freely movable between open and closed positions in response to flow into the vent assembly 3660 and/or pressure within the vent assembly 3660. Thus, the atmospheric vent flow 3676 may be a combination of the passive vent flow and the active vent flow 3675 at any given time during operation.

The vent body 3661 may include a groove 3672 to connect the vent body 3661 to the plenum chamber 3200 of a patient interface 3000.

### 5.3.5 Decoupling structure(s)

In one form the patient interface 3000 includes at least one decoupling structure, for example, a swivel or a ball and socket. For example, the patient interface 3000 shown in Figs. 43-47 comprises a conduit connector 3610 configured to swivel or rotate with respect to the positioning and stabilising structure 3300. The conduit connector 3610 in these examples may be an elbow, and in further examples the conduit connector 3610 may be straight from end to end. In this example the conduit connector 3610 is configured to swivel about an axis concentric with a circular opening in the positioning and stabilising structure 3300. In some examples of the present technology, the conduit connector 3610 may form part of a ball and socket joint to the positioning and stabilising structure 3300. For example, a ring having a partially spherical inner surface may be provided to the positioning and stabilising structure 3300 and may be configured to receive the conduit connector 3610. The conduit connector 3610 may have partially spherical outer surface complimentary to the partially spherical inner surface of the ring, thereby enabling the conduit connector 3610 to swivel with respect to the ring in a plurality of axes.

### 5.3.6 Connection port

Connection port 3600 allows for connection to the air circuit 4170. In the exemplary patient interface 3000 shown in Figs. 43-47, the conduit connector 3610 forms part of the connection port 3600. The conduit connector 3610, as a decoupling structure, decouples movement of the air circuit 4170 from the positioning and stabilising structure 3300 in order to reduce tube drag on the positioning and stabilising structure 3300.

### 5.3.7 Forehead support

In one form, the patient interface 3000 includes a forehead support 3700. Advantageously, the exemplary patient interface 3000 shown in Figs. 43-48 comprises a positioning and stabilising structure 3300 that is able to hold the seal-forming structure 3100 in sealing position without connection to a forehead support or any frame or strap members that lie in front of the patient's face at eye level.

### 5.3.8 Anti-asphyxia valve

In one form, the patient interface 3000 includes an anti-asphyxia valve. In the Fig. 48 example, each of the conduit connectors 3800 may include an anti-asphyxia valve.

### 5.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH2O, or at least 10cmH2O, or at least 20 cmH2O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors and flow rate sensors.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller, a therapy device controller, a pressure generator 4140, one or more protection circuits, memory, transducers 4270, data communication interface and one or more output devices. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

### 5.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000.

### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

### 5.4.1.3 Pressure generator

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH2O to about 20 cmH₂O, or in other forms up to about 30 cmH2O when delivering respiratory pressure therapy. The blower may be as described in any one of the following patents or patent applications: U.S. Patent No. 7,866,944; U.S. Patent No. 8,638,014; U.S. Patent No. 8,636,479; and PCT Patent Application Publication No. WO 2013/020167.

The pressure generator 4140 may be under the control of the therapy device controller 4240.

In other forms, a pressure generator 4140 may be a piston-driven pump, a pressure regulator connected to a high pressure source (e.g. compressed air reservoir), or a bellows.

### 5.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000.

In one form, a signal from a transducer 4270 may be filtered, such as by low-pass, high-pass or band-pass filtering.

### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device electrical components

### 5.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

### 5.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller.

In one form, the input device 4220 may be constructed and arranged to allow a person to select a value and/or a menu option.

### 5.4.2.3 Central controller

In one form of the present technology, the central controller is one or a plurality of processors suitable to control an RPT device 4000.

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349.

### 5.6 HUMIDIFIER

### 5.6.1 Humidifier overview

In one form of the present technology there is provided a humidifier 5000 (e.g. as shown in Fig. 41) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

### 5.7 BREATHING WAVEFORMS

Fig. 42 shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak -0.5* L/s. The total duration of the breath, *Trot,* is about 4s. The person typically breathes at a rate of about 15 breaths per minute (BPM), with Ventilation *Vent* about 7.5 L/min. A typical duty cycle, the ratio of *Ti* to *Ttot,* is about 40%.

### 5.8 GLOSSARY

For the purposes of the present technology disclosure, in certain forms of the present technology, one or more of the following definitions may apply. In other forms of the present technology, alternative definitions may apply.

### 5.8.1 General

*Air:* In certain forms of the present technology, air may be taken to mean atmospheric air, and in other forms of the present technology air may be taken to mean some other combination of breathable gases, e.g. oxygen enriched air.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Oxygen enriched air:* Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

*Medical Oxygen:* Medical oxygen is defined as oxygen enriched air with an oxygen concentration of 80% or greater.

*Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy:* The application of a supply of air to an entrance to the airways at a treatment pressure that is typically positive with respect to atmosphere.

*Ventilator:* A mechanical device that provides pressure support to a patient to perform some or all of the work of breathing.

### 5.8.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 5.8.1.2 Mechanical properties

Resilience: Ability of a material to absorb energy when deformed elastically and to release the energy upon unloading.

Resilient: Will release substantially all of the energy when unloaded. Includes e.g. certain silicones, and thermoplastic elastomers.

Hardness: The ability of a material per se to resist deformation (e.g. described by a Young's Modulus, or an indentation hardness scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

Stiffness (or rigidity) of a structure or component: The ability of the structure or component to resist deformation in response to an applied load. The load may be a force or a moment, e.g. compression, tension, bending or torsion. The structure or component may offer different resistances in different directions. The inverse of stiffness is flexibility.

Floppy structure or component: A structure or component that will change shape, e.g. bend, when caused to support its own weight, within a relatively short period of time such as 1 second.

Rigid structure or component: A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH2O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.8.2 Respiratory cycle

Apnea: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

Breathing rate: The rate of spontaneous respiration of a patient, usually measured in breaths per minute.

Duty cycle: The ratio of inhalation time, Ti to total breath time, Ttot.

Effort (breathing): The work done by a spontaneously breathing person attempting to breathe.

Expiratory portion of a breathing cycle: The period from the start of expiratory flow to the start of inspiratory flow.

Flow limitation: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) Flattened: Having a rise followed by a relatively flat portion, followed by a fall.
(ii) M-shaped: Having two local peaks, one at the leading edge, and one at the trailing edge, and a relatively flat portion between the two peaks.
(iii) Chair-shaped: Having a single local peak, the peak being at the leading edge, followed by a relatively flat portion.
(iv) Reverse-chair shaped: Having a relatively flat portion followed by single local peak, the peak being at the trailing edge.

Hypopnea: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

Hyperpnea: An increase in flow to a level higher than normal.

Inspiratory portion of a breathing cycle: The period from the start of inspiratory flow to the start of expiratory flow will be taken to be the inspiratory portion of a breathing cycle.

Patency (airway): The degree of the airway being open, or the extent to which the airway is open. A patent airway is open. Airway patency may be quantified, for example with a value of one (1) being patent, and a value of zero (0), being closed (obstructed).

Positive End-Expiratory Pressure (PEEP): The pressure above atmosphere in the lungs that exists at the end of expiration.

Peak flow rate (Qpeak): The maximum value of flow rate during the inspiratory portion of the respiratory flow waveform.

Respiratory flow rate, patient airflow rate, respiratory airflow rate (Qr): These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

Tidal volume (Vt): The volume of air inhaled or exhaled during normal breathing, when extra effort is not applied. In principle the inspiratory volume Vi (the volume of air inhaled) is equal to the expiratory volume Ve (the volume of air exhaled), and therefore a single tidal volume Vt may be defined as equal to either quantity. In practice the tidal volume Vt is estimated as some combination, e.g. the mean, of the inspiratory volume Vi and the expiratory volume Ve.

Inhalation Time (Ti): The duration of the inspiratory portion of the respiratory flow rate waveform.

Exhalation Time (Te): The duration of the expiratory portion of the respiratory flow rate waveform.

Total Time (Ttot): The total duration between the start of one inspiratory portion of a respiratory flow rate waveform and the start of the following inspiratory portion of the respiratory flow rate waveform.

Typical recent ventilation: The value of ventilation around which recent values of ventilation Vent over some predetermined timescale tend to cluster, that is, a measure of the central tendency of the recent values of ventilation.

Upper airway obstruction (UAO): includes both partial and total upper airway obstruction. This may be associated with a state of flow limitation, in which the flow rate increases only slightly or may even decrease as the pressure difference across the upper airway increases (Starling resistor behaviour).

Ventilation (Vent): A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.8.3 Ventilation

Adaptive Servo-Ventilator (ASV): A servo-ventilator that has a changeable, rather than fixed target ventilation. The changeable target ventilation may be learned from some characteristic of the patient, for example, a respiratory characteristic of the patient.

Backup rate: A parameter of a ventilator that establishes the minimum breathing rate (typically in number of breaths per minute) that the ventilator will deliver to the patient, if not triggered by spontaneous respiratory effort.

Cycled: The termination of a ventilator's inspiratory phase. When a ventilator delivers a breath to a spontaneously breathing patient, at the end of the inspiratory portion of the breathing cycle, the ventilator is said to be cycled to stop delivering the breath.

Expiratory positive airway pressure (EPAP): a base pressure, to which a pressure varying within the breath is added to produce the desired interface pressure which the ventilator will attempt to achieve at a given time.

End expiratory pressure (EEP): Desired interface pressure which the ventilator will attempt to achieve at the end of the expiratory portion of the breath. If the pressure waveform template □(□) is zero-valued at the end of expiration, i.e. □(□) = 0 when □ = 1, the EEP is equal to the EPAP.

Inspiratory positive airway pressure (IPAP): Maximum desired interface pressure which the ventilator will attempt to achieve during the inspiratory portion of the breath.

Pressure support: A number that is indicative of the increase in pressure during ventilator inspiration over that during ventilator expiration, and generally means the difference in pressure between the maximum value during inspiration and the base pressure (e.g., PS = IPAP - EPAP). In some contexts, pressure support means the difference which the ventilator aims to achieve, rather than what it actually achieves.

Servo-ventilator: A ventilator that measures patient ventilation, has a target ventilation, and which adjusts the level of pressure support to bring the patient ventilation towards the target ventilation.

Spontaneous/Timed (S/T): A mode of a ventilator or other device that attempts to detect the initiation of a breath of a spontaneously breathing patient. If however, the device is unable to detect a breath within a predetermined period of time, the device will automatically initiate delivery of the breath.

Swing: Equivalent term to pressure support.

Triggered: When a ventilator, or other respiratory therapy device such as an RPT device or portable oxygen concentrator, delivers a volume of breathable gas to a spontaneously breathing patient, it is said to be triggered to do so. Triggering usually takes place at or near the initiation of the respiratory portion of the breathing cycle by the patient's efforts.

### 5.8.4 Anatomy

### 5.8.4.1 Anatomy of the face

Ala: the external outer wall or "wing" of each nostril (plural: alar)

Alar angle:

Alare: The most lateral point on the nasal ala.

Alar curvature (or alar crest) point: The most posterior point in the curved base line of each ala, found in the crease formed by the union of the ala with the cheek.

Auricle: The whole external visible part of the ear.

(nose) Bony framework: The bony framework of the nose comprises the nasal bones, the frontal process of the maxillae and the nasal part of the frontal bone.

(nose) Cartilaginous framework: The cartilaginous framework of the nose comprises the septal, lateral, major and minor cartilages.

Columella: the strip of skin that separates the nares and which runs from the pronasale to the upper lip.

Columella angle: The angle between the line drawn through the midpoint of the nostril aperture and a line drawn perpendicular to the Frankfort horizontal while intersecting subnasale.

Frankfort horizontal plane: A line extending from the most inferior point of the orbital margin to the left tragion. The tragion is the deepest point in the notch superior to the tragus of the auricle.

Glabella: Located on the soft tissue, the most prominent point in the midsagittal plane of the forehead.

Lateral nasal cartilage: A generally triangular plate of cartilage. Its superior margin is attached to the nasal bone and frontal process of the maxilla, and its inferior margin is connected to the greater alar cartilage.

Greater alar cartilage: A plate of cartilage lying below the lateral nasal cartilage. It is curved around the anterior part of the naris. Its posterior end is connected to the frontal process of the maxilla by a tough fibrous membrane containing three or four minor cartilages of the ala.

Nares (Nostrils): Approximately ellipsoidal apertures forming the entrance to the nasal cavity. The singular form of nares is naris (nostril). The nares are separated by the nasal septum.

Naso-labial sulcus or Naso-labial fold: The skin fold or groove that runs from each side of the nose to the corners of the mouth, separating the cheeks from the upper lip.

Naso-labial angle: The angle between the columella and the upper lip, while intersecting subnasale.

Otobasion inferior: The lowest point of attachment of the auricle to the skin of the face.

Otobasion superior: The highest point of attachment of the auricle to the skin of the face.

Pronasale: the most protruded point or tip of the nose, which can be identified in lateral view of the rest of the portion of the head.

Philtrum: the midline groove that runs from lower border of the nasal septum to the top of the lip in the upper lip region.

Pogonion: Located on the soft tissue, the most anterior midpoint of the chin.

Ridge (nasal): The nasal ridge is the midline prominence of the nose, extending from the Sellion to the Pronasale.

Sagittal plane: A vertical plane that passes from anterior (front) to posterior (rear). The midsagittal plane is a sagittal plane that divides the body into right and left halves.

Sellion: Located on the soft tissue, the most concave point overlying the area of the frontonasal suture.

Septal cartilage (nasal): The nasal septal cartilage forms part of the septum and divides the front part of the nasal cavity.

Subalare: The point at the lower margin of the alar base, where the alar base joins with the skin of the superior (upper) lip.

Subnasal point: Located on the soft tissue, the point at which the columella merges with the upper lip in the midsagittal plane.

Supramenton: The point of greatest concavity in the midline of the lower lip between labrale inferius and soft tissue pogonion

### Anatomy of the skull

Frontal bone: The frontal bone includes a large vertical portion, the squama frontalis, corresponding to the region known as the forehead.

Mandible: The mandible forms the lower jaw. The mental protuberance is the bony protuberance of the jaw that forms the chin.

Maxilla: The maxilla forms the upper jaw and is located above the mandible and below the orbits. The frontal process of the maxilla projects upwards by the side of the nose, and forms part of its lateral boundary.

Nasal bones: The nasal bones are two small oblong bones, varying in size and form in different individuals; they are placed side by side at the middle and upper part of the face, and form, by their junction, the "bridge" of the nose.

Nasion: The intersection of the frontal bone and the two nasal bones, a depressed area directly between the eyes and superior to the bridge of the nose.

Occipital bone: The occipital bone is situated at the back and lower part of the cranium. It includes an oval aperture, the foramen magnum, through which the cranial cavity communicates with the vertebral canal. The curved plate behind the foramen magnum is the squama occipitalis.

Orbit: The bony cavity in the skull to contain the eyeball.

Parietal bones: The parietal bones are the bones that, when joined together, form the roof and sides of the cranium.

Temporal bones: The temporal bones are situated on the bases and sides of the skull, and support that part of the face known as the temple.

Zygomatic bones: The face includes two zygomatic bones, located in the upper and lateral parts of the face and forming the prominence of the cheek.

### 5.8.4.2 Anatomy of the respiratory system

Diaphragm: A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

Larynx: The larynx, or voice box houses the vocal folds and connects the inferior part of the pharynx (hypopharynx) with the trachea.

Lungs: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

Nasal cavity: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

Pharynx: The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.8.5 Patient interface

Anti-asphyxia valve (AAV): The component or sub-assembly of a mask system that, by opening to atmosphere in a failsafe manner, reduces the risk of excessive CO2 rebreathing by a patient.

Elbow: An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

Frame: Frame will be taken to mean a mask structure that bears the load of tension between two or more points of connection with a headgear. A mask frame may be a non-airtight load bearing structure in the mask. However, some forms of mask frame may also be air-tight.

Functional dead space: (description to be inserted here)

Headgear: Headgear will be taken to mean a form of positioning and stabilizing structure designed for use on a head. For example the headgear may comprise a collection of one or more struts. ties and stiffeners configured to locate and retain a patient interface in position on a patient's face for delivery of respiratory therapy. Some ties are formed of a soft, flexible, elastic material such as a laminated composite of foam and fabric.

Membrane: Membrane will be taken to mean a typically thin element that has, preferably, substantially no resistance to bending, but has resistance to being stretched.

Plenum chamber: a mask plenum chamber will be taken to mean a portion of a patient interface having walls at least partially enclosing a volume of space, the volume having air therein pressurised above atmospheric pressure in use. A shell may form part of the walls of a mask plenum chamber.

Seal: May be a noun form ("a seal") which refers to a structure, or a verb form ("to seal") which refers to the effect. Two elements may be constructed and/or arranged to 'seal' or to effect 'sealing' therebetween without requiring a separate 'seal' element per se.

Shell: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

Stiffener: A stiffener will be taken to mean a structural component designed to increase the bending resistance of another component in at least one direction.

Strut: A strut will be taken to be a structural component designed to increase the compression resistance of another component in at least one direction.

Swivel (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

Tie (noun): A structure designed to resist tension.

Vent: (noun): A structure that allows a flow of air from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.8.6 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

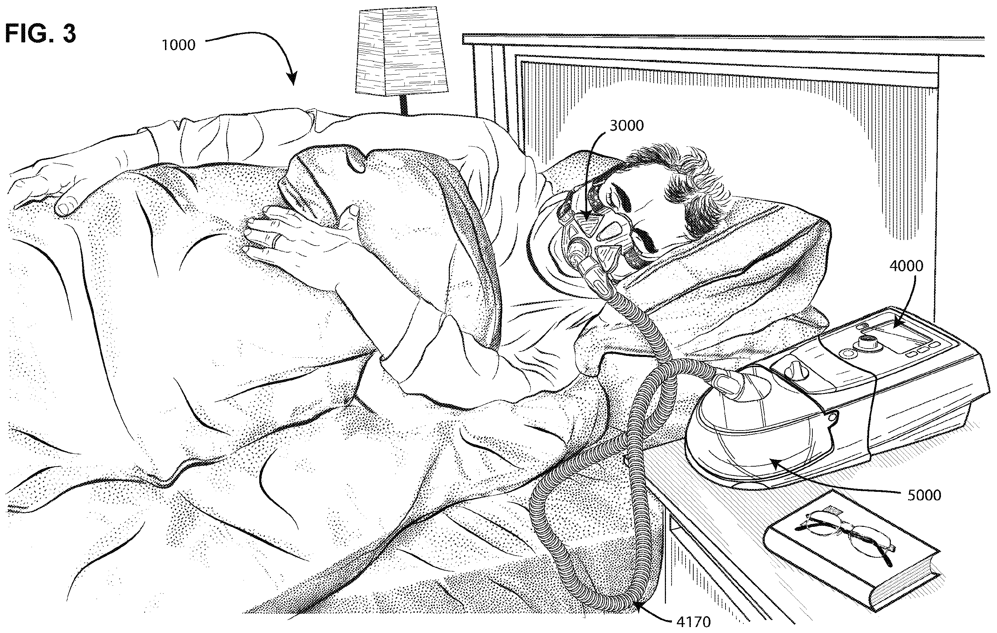

To facilitate describing the shape of the three-dimensional structures and the surfaces, we first consider a cross-section through a surface of the structure at a point, p. See Fig. 3B to Fig. 3F, which illustrate examples of cross-sections at point p on a surface, and the resulting plane curves. Figs. 3B to 3F also illustrate an outward normal vector at p. The outward normal vector at p points away from the surface. In some examples we describe the surface from the point of view of an imaginary small person standing upright on the surface.

### 5.8.6.1 Curvature in one dimension

The curvature of a plane curve at p may be described as having a sign (e.g. positive, negative) and a magnitude (e.g. 1/radius of a circle that just touches the curve at p).

Positive curvature: If the curve at p turns towards the outward normal, the curvature at that point will be taken to be positive (if the imaginary small person leaves the point p they must walk uphill). See Fig. 3B (relatively large positive curvature compared to Fig. 3C) and Fig. 3C (relatively small positive curvature compared to Fig. 3B). Such curves are often referred to as concave.

Zero curvature: If the curve at p is a straight line, the curvature will be taken to be zero (if the imaginary small person leaves the point p, they can walk on a level, neither up nor down). See Fig. 3D.

Negative curvature: If the curve at p turns away from the outward normal, the curvature in that direction at that point will be taken to be negative (if the imaginary small person leaves the point p they must walk downhill). See Fig. 3E (relatively small negative curvature compared to Fig. 3F) and Fig. 3F (relatively large negative curvature compared to Fig. 3E). Such curves are often referred to as convex.

### 5.8.6.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

Principal curvatures and directions: The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at p are the curvatures in the principal directions.

Region of a surface: A connected set of points on a surface. The set of points in a region may have similar characteristics, e.g. curvatures or signs.

Saddle region: A region where at each point, the principal curvatures have opposite signs, that is, one is positive, and the other is negative (depending on the direction to which the imaginary person turns, they may walk uphill or downhill).

Dome region: A region where at each point the principal curvatures have the same sign, e.g. both positive (a "concave dome") or both negative (a "convex dome").

Cylindrical region: A region where one principal curvature is zero (or, for example, zero within manufacturing tolerances) and the other principal curvature is non-zero.

Planar region: A region of a surface where both of the principal curvatures are zero (or, for example, zero within manufacturing tolerances).

Edge of a surface: A boundary or limit of a surface or region.

Path: In certain forms of the present technology, 'path' will be taken to mean a path in the mathematical - topological sense, e.g. a continuous space curve from f(0) to f(1) on a surface. In certain forms of the present technology, a 'path' may be described as a route or course, including e.g. a set of points on a surface. (The path for the imaginary person is where they walk on the surface, and is analogous to a garden path).

Path length: In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

Straight-line distance: The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 5.8.6.3 Space curves

Space curves: Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

Tangent unit vector (or unit tangent vector): For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

Unit normal vector: As the imaginary person moves along the curve, this tangent vector itself changes. The unit vector pointing in the same direction that the tangent vector is changing is called the unit principal normal vector. It is perpendicular to the tangent vector.

Binormal unit vector: The binormal unit vector is perpendicular to both the tangent vector and the principal normal vector. Its direction may be determined by a right-hand rule (see e.g. Fig. 3P), or alternatively by a left-hand rule (Fig. 3O).

Osculating plane: The plane containing the unit tangent vector and the unit principal normal vector. See Figures 3O and 3P.

Torsion of a space curve: The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 5.8.6.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 5.9 OTHER REMARKS

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

The invention is defined by the claims which follow.

### 5.10 REFERENCE SIGNS LIST

| | |
|---|---|
| patient | 1000 |
| bed partner | 1100 |
| patient interface | 3000 |
| seal - forming structure | 3100 |
| seal-forming structure hole | 3102 |
| sealing lip | 3103 |
| sealing lip bypass opening | 3104 |
| cradle cushion module | 3150 |
| pillows cushion module | 3160 |
| nasal pillows | 3165 |
| plenum chamber | 3200 |
| connection lip | 3201 |
| tube connector | 3202 |
| orientation indication protrusion | 3203 |
| receiving hole | 3204 |
| chord | 3210 |
| superior point | 3220 |
| inferior point | 3230 |
| positioning and stabilising structure | 3300 |
| clip | 3301 |
| upper strap | 3302 |
| lower strap | 3303 |
| rear portion | 3304 |
| central section | 3305 |
| conduit connector receiving hole | 3306 |
| strap | 3310 |
| tube | 3350 |
| non - extendable tube section | 3363 |
| sleeve | 3364 |
| fluid connection opening | 3390 |
| vent assembly | 3400 |
| vent cap | 3401 |
| vent hole | 3402 |
| vent body | 3403 |
| vent diffuser material | 3404 |
| vent body hole | 3405 |
| spacer | 3406 |
| orientation indication recess | 3407 |
| connection channel | 3408 |
| attachment structure | 3409 |
| slot | 3410 |
| connection port | 3600 |
| conduit connector and vent assembly | 3605 |
| vent assembly | 3606 |
| outlet portion | 3608 |
| inlet portion | 3609 |
| conduit connector | 3610 |
| outlet end | 3611 |
| inlet end | 3612 |
| bayonet connector | 3613 |
| vent assembly receiving hole | 3614 |
| radial protrusion | 3615 |
| base | 3616 |
| spoke | 3617 |
| base hole | 3618 |
| radial base notch | 3619 |
| post | 3620 |
| axial protrusion | 3621 |
| flap | 3622 |
| flap hole | 3623 |
| cover | 3624 |
| radial cover notch | 3625 |
| axial notch | 3626 |
| cover hole | 3627 |
| central portion | 3628 |
| annular portion | 3629 |
| axial gap | 3630 |
| outer radial gap | 3631 |
| inner radial gap | 3632 |
| body portion | 3640 |
| hole | 3641 |
| tab | 3642 |
| hinge | 3643 |
| tab receiving hole | 3644 |
| flap | 3645 |
| bump | 3646 |
| swivel | 3647 |
| vent ring | 3650 |
| vent holes | 3651 |
| connector ring | 3652 |
| cage | 3653 |
| connector | 3654 |
| membrane | 3655 |
| elbow | 3656 |
| intermediate tube | 3657 |
| connector lip | 3658 |
| vent assembly | 3660 |
| vent body | 3661 |
| first vent holes | 3662 |
| second vent holes | 3663 |
| first side | 3664 |
| second side | 3665 |
| base | 3666 |
| cap | 3667 |
| vent passage | 3668 |
| diffuser member | 3669 |
| ring-shaped protrusion | 3670 |
| membrane | 3671 |
| groove | 3672 |
| membrane hole | 3673 |
| passive vent flow | 3674 |
| active vent flow | 3675 |
| atmospheric vent flow | 3676 |
| diffuser member spacer | 3677 |
| forehead support | 3700 |
| conduit connector | 3800 |
| RPT device | 4000 |
| external housing | 4010 |
| upper portion | 4012 |
| lower portion | 4014 |
| panel | 4015 |
| chassis | 4016 |
| handle | 4018 |
| pneumatic block | 4020 |
| pneumatic components | 4100 |
| air filter | 4110 |
| inlet air filter | 4112 |
| outlet air filter | 4114 |
| muffler | 4120 |
| inlet muffler | 4122 |
| outlet muffler | 4124 |
| pressure generator | 4140 |
| blower | 4142 |
| motor | 4144 |
| anti - spill back valve | 4160 |
| air circuit | 4170 |
| supplementary gas | 4180 |
| electrical components | 4200 |
| PCBA | 4202 |
| electrical power supply | 4210 |
| input devices | 4220 |
| central controller | 4230 |
| transducer | 4270 |
| humidifier | 5000 |
| pressure | 8000 |
| vent flow | 8002 |

## Claims

1. A conduit connector and vent assembly (3605) for connecting an air circuit (4170) to a patient interface (3000) to direct pressurized air into the patient interface (3000) from the air circuit (4170), the conduit connector and vent assembly (3605) comprising:
a conduit connector (3610) comprising:
an inlet portion (3609) having an inlet end (3612) configured to be connected to the air circuit (4170) and having a vent assembly receiving hole (3614) that extends through the inlet portion (3609) in a radial direction; and
an outlet portion (3608) having an outlet end (3611) configured to be connected to the patient interface (3000); and
a vent assembly (3606) positioned in the vent assembly receiving hole (3614), constructed and arranged to allow for washout of exhaled gases to ambient, and comprising:
a base (3616) positioned proximal to an interior of the conduit connector (3610);
a cover (3624) positioned distal to the interior of the conduit connector (3610); and
a flap (3622) positioned between the base (3616) and the cover (3624) and not connected to the base (3616) or the cover (3624) so as to be freely movable between the base (3616) and the cover (3624) during use.

2. The conduit connector and vent assembly (3605) of claim 1, wherein a radial protrusion (3615) extends from the inlet portion (3609) within the vent assembly receiving hole (3614).

3. The conduit connector and vent assembly (3605) of claim 1 or claim 2, wherein the base (3616) includes a radial base notch (3619) configured to receive the radial protrusion (3615) when the base (3616) is positioned within the vent assembly receiving hole (3614).

4. The conduit connector and vent assembly (3605) of one of claims 1 to 3, wherein the cover (3624) includes a radial cover notch configured to receive the radial protrusion (3615) when the cover (3624) is positioned within the vent assembly receiving hole (3614).

5. The conduit connector and vent assembly (3605) of one of claims 1 to 4, wherein the base (3616) includes a post (3620) configured to contact the cover (3624) and maintain a gap between the base (3616) and the cover (3624), the flap (3622) being positioned within the gap, when the vent assembly (3606) is positioned within the vent assembly receiving hole (3614).

6. The conduit connector and vent assembly (3605) of one of claims 1 to 5, wherein the base (3616) includes an axial protrusion (3621) and the cover (3624) includes an axial notch (3626), the axial notch (3626) being configured to receive the axial protrusion (3621) to maintain the gap between the base (3616) and the cover (3624) when the vent assembly (3606) is positioned within the vent assembly receiving hole (3614).

7. The conduit connector and vent assembly (3605) of one of claims 1 to 6, wherein the base (3616) includes a plurality of spokes (3617) forming a plurality of base holes (3618) to allow exhaled gases to pass through the base (3616).

8. The conduit connector and vent assembly (3605) of one of claims 1 to 7, wherein the flap (3622) is configured to rest on the base (3616) until sufficient pressure within the conduit connector (3610) causes the flap (3622) to lift off from the base (3616).

9. The conduit connector and vent assembly (3605) of one of claims 1 to 8, wherein the cover (3624) includes a plurality of cover holes (3627) to allow exhaled gases to pass through the cover (3624) to ambient.

10. The conduit connector and vent assembly (3605) of claim 9, wherein the cover (3624) includes an annular portion (3629) and a central portion (3628), the cover holes (3627) being positioned between the annular portion (3629) and the central portion (3628).

11. The conduit connector and vent assembly (3605) of claim 10, wherein the flap (3622) is configured to completely block the cover holes (3627) when the flap contacts the annular portion (3629) and the central portion (3628),
wherein the flap (3622) is configured to allow exhaled gases to pass through the cover (3624) to ambient when the flap contacts only one of the annular portion (3629) or the central portion (3628), and
wherein the annular portion (3629) and the central portion (3628) are offset relative to one another in an axial direction of the cover (3624).

12. The conduit connector and vent assembly (3605) of one of claims 1 to 11, wherein the flap (3622) is constructed from a flexible material,
wherein the flexible material is silicone, and
wherein each of the base (3616) and the cover (3624) is constructed from a rigid material.

13. The conduit connector and vent assembly (3605) of one of claims 1 to 12, wherein the inlet end (3612) include a bayonet connector (3613) such that when the air circuit (4170) is connected to the conduit connector (3610), the air circuit (4170) and the conduit connector (3610) are not rotatable relative to one another.

14. A patient interface (3000) comprising:
a plenum chamber pressurisable to a therapeutic pressure of at least 4 cmH20 above ambient air pressure by a flow of air at the therapeutic pressure for breathing by a patient, the plenum chamber (3200) further comprising two plenum chamber connectors (3202), each of the plenum chamber connectors (3202) being positioned on a corresponding lateral side of the plenum chamber (3200) and configured to receive the flow of air at the therapeutic pressure;
a seal-forming structure (3100) connected to the plenum chamber (3200), the seal-forming structure (3100) being constructed and arranged to seal with a region of the patient's face that at least partly surrounds an entrance to the patient's airways, the seal-forming structure (3100) having a hole therein to deliver the flow of air at the therapeutic pressure to at least the patient's nares during use, the seal-forming structure (3100) being constructed and arranged to maintain the therapeutic pressure within the plenum chamber (3200) throughout the patient's respiratory cycle in use;
a positioning and stabilising structure (3300) configured to hold the seal-forming structure (3100) in a therapeutically effective position on the patient's head, the positioning and stabilising structure (3300) further comprising two conduits (3350) and a central section (3305) between the conduits (3350), the central section (3305) having a conduit connector receiving hole (3306), each of the conduits (3350) being configured to be positioned on a corresponding lateral side of the patient's head in use and being configured to connect to a corresponding one of the plenum chamber connectors (3202); and
the conduit connector and vent assembly (3605) of one of claims 1 to 13 connected to the central section (3305) at the conduit connector receiving hole (3306),
wherein the patient interface (3000) is configured to leave the patient's mouth uncovered, or the seal-forming structure (3100) is configured to seal around the patient's nose and mouth and the patient interface (3000) is configured to allow the patient to breath from ambient in the absence of the flow of air at the therapeutic pressure.

15. The patient interface (3000) of claim 14, wherein the conduit connector and vent assembly (3605) is rotatably and releasably connected to the central section (3305), and
wherein the plenum chamber (3200) comprises a passive vent (3400) configured to allow exhaled gases to pass to ambient independent of therapeutic pressure and throughout the patient's respiratory cycle.

## Patentansprüche

1. Leitungsverbinder- und Entlüftungsanordnung (3605) zum Verbinden einer Luftleitung (4170) mit einer Patientenschnittstelle (3000), um Druckluft aus der Luftleitung (4170) in die Patientenschnittstelle (3000) zu leiten, wobei die Leitungsverbinder- und Entlüftungsanordnung (3605) aufweist:
einen Leitungsverbinder (3610), aufweisend:
einen Einlassabschnitt (3609) mit einem Einlassende (3612), das dazu konfiguriert ist, mit der Luftleitung (4170) verbunden zu werden, und mit einer Entlüftungsanordnungs-Aufnahmeöffnung (3614), die sich durch den Einlassabschnitt (3609) in einer radialen Richtung erstreckt; und einen Auslassabschnitt (3608) mit einem Auslassende (3611), das dazu konfiguriert ist, mit der Patientenschnittstelle (3000) verbunden zu werden; und
eine in der Entlüftungsanordnungs-Aufnahmeöffnung (3614) positionierte Entlüftungsanordung (3606), die zum Ermöglichen des Auswaschens von ausgeatmeten Gasen in die Umgebung aufgebaut und angeordnet ist und die aufweist:
eine Basis (3616), die proximal zu einem Inneren des Leitungsverbinders (3610) positioniert ist; eine Abdeckung (3624), die distal zu dem Inneren des Leitungsverbinders (3610) positioniert ist; und
eine Klappe (3622), die zwischen der Basis (3616) und der Abdeckung (3624) positioniert und nicht mit der Basis (3616) oder der Abdeckung (3624) verbunden ist, sodass sie im Gebrauch frei zwischen der Basis (3616) und der Abdeckung (3624) beweglich ist.

2. Leitungsverbinder- und Entlüftungsanordnung (3605) nach Anspruch 1, wobei sich ein radialer Vorsprung (3615) von dem Einlassabschnitt (3609) in der Entlüftungsanordnungs-Aufnahmeöffnung (3614) erstreckt.

3. Leitungsverbinder- und Entlüftungsanordnung (3605) nach Anspruch 1 oder Anspruch 2, wobei die Basis (3616) eine radiale Basiskerbe (3619) aufweist, die zum Aufnehmen des radialen Vorsprungs (3615) konfiguriert ist, wenn die Basis (3616) in der Entlüftungsanordnungs-Aufnahmeöffnung (3614) positioniert ist.

4. Leitungsverbinder- und Entlüftungsanordnung (3605) nach einem der Ansprüche 1 bis 3, wobei die Abdeckung (3624) eine radiale Abdeckungskerbe aufweist, die zum Aufnehmen des radialen Vorsprungs (3615) konfiguriert ist, wenn die Abdeckung (3624) in der Entlüftungsanordnungs-Aufnahmeöffnung (3614) positioniert ist.

5. Leitungsverbinder- und Entlüftungsanordnung (3605) nach einem der Ansprüche 1 bis 4, wobei die Basis (3616) eine Strebe (3620) aufweist, die zum Berühren der Abdeckung (3624) und zum Aufrechterhalten eines Spalts zwischen der Basis (3616) und der Abdeckung (3624) konfiguriert ist, wobei die Klappe (3622) in dem Spalt positioniert ist, wenn die Entlüftungsanordnung (3606) in der Entlüftungsanordnungs-Aufnahmeöffnung (3614) positioniert ist.

6. Leitungsverbinder- und Entlüftungsanordnung (3605) nach einem der Ansprüche 1 bis 5, wobei die Basis (3616) einen axialen Vorsprung (3621) und die Abdeckung (3624) eine axiale Kerbe (3626) aufweist, wobei die axiale Kerbe (3626) zum Aufnehmen des axialen Vorsprungs (3621) konfiguriert ist, um den Spalt zwischen der Basis (3616) und der Abdeckung (3624) aufrechtzuerhalten, wenn die Entlüftungsanordnung (3606) in der Entlüftungsanordnungs-Aufnahmeöffnung (3614) positioniert ist.

7. Leitungsverbinder- und Entlüftungsanordnung (3605) nach einem der Ansprüche 1 bis 6, wobei die Basis (3616) mehrere Speichen (3617) aufweist, die mehrere Basisöffnungen (3618) bilden, damit ausgeatmete Gase durch die Basis (3616) strömen können.

8. Leitungsverbinder- und Entlüftungsanordnung (3605) nach einem der Ansprüche 1 bis 7, wobei die Klappe (3622) zum Aufliegen auf der Basis (3616) konfiguriert ist, bis ein ausreichender Druck in dem Leitungsverbinder (3610) bewirkt, dass sich die Klappe (3622) von der Basis (3616) abhebt.

9. Leitungsverbinder- und Entlüftungsanordnung (3605) nach einem der Ansprüche 1 bis 8, wobei die Abdeckung (3624) mehrere Abdeckungsöffnungen (3627) aufweist, damit ausgeatmete Gase durch die Abdeckung (3624) in die Umgebung strömen können.

10. Leitungsverbinder- und Entlüftungsanordnung (3605) nach Anspruch 9, wobei die Abdeckung (3624) einen ringförmigen Abschnitt (3629) und einen mittleren Abschnitt (3628) aufweist, wobei die Abdeckungsöffnungen (3627) zwischen dem ringförmigen Abschnitt (3629) und dem mittleren Abschnitt (3628) positioniert sind.

11. Leitungsverbinder- und Entlüftungsanordnung (3605) nach Anspruch 10, wobei die Klappe (3622) zum vollständen Blockieren der Abdeckungsöffnungen (3627) konfiguriert ist, wenn die Klappe den ringförmigen Abschnitt (3629) und den mittleren Abschnitt (3628) berührt,
wobei die Klappe (3622) dazu konfiguriert ist, dass ausgeatmete Gase durch die Abdeckung (3624) in die Umgebung strömen können, wenn die Klappe nur entweder den ringförmigen Abschnitt (3629) oder den mittleren Abschnitt (3628) berührt, und
wobei der ringförmige Abschnitt (3629) und der mittlere Abschnitt (3628) in einer axialen Richtung der Abdeckung (3624) relativ zueinander versetzt sind.

12. Leitungsverbinder- und Entlüftungsanordnung (3605) nach einem der Ansprüche 1 bis 11, wobei die Klappe (3622) aus einem flexiblen Material hergestellt ist,
wobei das flexible Material Silikon ist und
wobei sowohl die Basis (3616) als auch die Abdeckung (3624) aus einem starren Material hergestellt sind.

13. Leitungsverbinder- und Entlüftungsanordnung (3605) nach einem der Ansprüche 1 bis 12, wobei das Einlassende (3612) einen Bajonettverschluss (3613) aufweist, so dass die Luftleitung (4170) und der Leitungsverbinder (3610) nicht relativ zueinander drehbar sind, wenn die Luftleitung (4170) mit dem Leitungsverbinder (3610) verbunden ist.

14. Patientenschnittstelle (3000), aufweisend:
eine Luftkammer, die durch einen Luftstrom mit dem therapeutischen Druck zur Atmung durch einen Patienten auf einen therapeutischen Druck von mindestens 4 cmH2O über dem Umgebungsluftdruck bringbar ist, wobei die Luftkammer (3200) ferner zwei Luftkammerverbinder (3202) aufweist, wobei jeder der Luftkammerverbinder (3202) auf einer entsprechenden lateralen Seite der Luftkammer (3200) positioniert ist und zum Aufnehmen des Luftstroms mit dem therapeutischen Druck konfiguriert ist;
eine dichtungsbildende Struktur (3100), die mit der Luftkammer (3200) verbunden ist, wobei die dichtungsbildende Struktur (3100) zum Bilden einer Dichtung mit einem Bereich des Gesichts des Patienten, der zumindest teilweise einen Eingang zu den Atemwegen des Patienten umgibt, aufgebaut und angeordnet ist, wobei die dichtungsbildende Struktur (3100) eine Öffnung derart aufweist, dass der Luftstrom mit dem therapeutischen Druck im Gebrauch mindestens den Nasenlöchern des Patienten zugeführt wird, wobei die dichtungsbildende Struktur (3100) im Gebrauch zum Aufrechterhalten des therapeutischen Druck in der Luftkammer (3200) während des gesamten Atemzyklus des Patienten aufgebaut und angeordnet ist;
eine Positionierungs- und Stabilisierungsstruktur (3300), die zum Halten der dichtungsbildenden Struktur (3100) in einer therapeutisch wirksamen Position am Kopf des Patienten konfiguriert ist, wobei die Positionierungs- und Stabilisierungsstruktur (3300) ferner zwei Leitungen (3350) und
einen mittleren Abschnitt (3305) zwischen den Leitungen (3350) aufweist, wobei der mittlere Abschnitt (3305) eine Leitungsverbinderaufnahmeöffnung (3306) aufweist, wobei jede der Leitungen (3350) dazu konfiguriert ist, im Gebrauch auf einer entsprechenden lateralen Seite des Kopfes des Patienten positioniert zu sein und dazu konfiguriert ist, mit einem entsprechenden der Luftkammerverbinder (3202) verbunden zu sein; und
die Leitungsverbinder- und Entlüftungsanordnung (3605) nach einem der Ansprüche 1 bis 13, die mit dem mittleren Abschnitt (3305) an der Leitungsverbinderaufnahmeöffnung (3306) verbunden ist,
wobei die Patientenschnittstelle (3000) dazu konfiguriert ist, den Mund des Patienten unbedeckt zu lassen, oder die dichtungsbildende Struktur (3100) dazu konfiguriert ist, den Bereich um die Nase und den Mund des Patienten herum abzudichten, und die Patientenschnittstelle (3000) dazu konfiguriert ist, dem Patienten zu ermöglichen, bei Abwesenheit des Luftstroms mit dem therapeutischen Druck aus der Umgebung zu atmen.

15. Patientenschnittstelle (3000) nach Anspruch 14, wobei die Leitungsverbinder- und Entlüftungsanordnung (3605) drehbar und lösbar mit dem mittleren Abschnitt (3305) verbunden ist, und wobei die Luftkammer (3200) eine passive Entlüftung (3400) aufweist, die dazu konfiguriert ist, dass ausgeatmete Gase unabhängig vom therapeutischen Druck und während des gesamten Atmungszyklus des Patienten in die Umgebung strömen können.

## Revendications

1. Ensemble de raccord de conduit et d'évent (3605) destiné à raccorder un circuit d'air (4170) à une interface patient (3000) afin d'acheminer de l'air sous pression depuis le circuit d'air (4170) vers l'interface patient (3000), l'ensemble de raccord de conduit et d'évent (3605) comprenant :
un raccord de conduit (3610) comprenant :
une portion d'entrée (3609) ayant une extrémité d'entrée (3612) configurée pour être raccordée au circuit d'air (4170) et ayant un trou de réception d'ensemble d'évent (3614) qui s'étend à travers la portion d'entrée (3609) dans une direction radiale ; et
une portion de sortie (3608) ayant une extrémité de sortie (3611) configurée pour être raccordée à l'interface patient (3000) ; et
un ensemble d'évent (3606) positionné dans le trou de réception d'ensemble d'évent (3614), conçu et agencé pour permettre l'évacuation des gaz expirés vers l'environnement, et comprenant :
une base (3616) positionnée à proximité d'un espace intérieur du raccord de conduit (3610) ;
un couvercle (3624) positionné distalement par rapport à l'espace intérieur du raccord de conduit (3610) ; et
un volet (3622) positionné entre la base (3616) et le couvercle (3624) et non relié à la base (3616) ou au couvercle (3624) de manière à pouvoir se déplacer librement entre la base (3616) et le couvercle (3624) pendant l'utilisation.

2. Ensemble de raccord de conduit et d'évent (3605) selon la revendication 1,
dans lequel une saillie radiale (3615) s'étend depuis la portion d'entrée (3609) à l'intérieur du trou de réception d'ensemble d'évent (3614).

3. Ensemble de raccord de conduit et d'évent (3605) selon la revendication 1 ou la revendication 2,
dans lequel la base (3616) comprend une encoche de base radiale (3619) configurée pour recevoir la saillie radiale (3615) lorsque la base (3616) est positionnée à l'intérieur du trou de réception d'ensemble d'évent (3614).

4. Ensemble de raccord de conduit et d'évent (3605) selon l'une des revendications 1 à 3,
dans lequel le couvercle (3624) comprend une encoche de couvercle radiale configurée pour recevoir la saillie radiale (3615) lorsque le couvercle (3624) est positionné à l'intérieur du trou de réception d'ensemble d'évent (3614).

5. Ensemble de raccord de conduit et d'évent (3605) selon l'une des revendications 1 à 4,
dans lequel la base (3616) comprend un montant (3620) configuré pour entrer en contact avec le couvercle (3624) et pour maintenir un interstice entre la base (3616) et le couvercle (3624), le volet (3622) étant positionné dans ledit interstice lorsque l'ensemble d'évent (3606) est positionné à l'intérieur du trou de réception d'ensemble d'évent (3614).

6. Ensemble de raccord de conduit et d'évent (3605) selon l'une des revendications 1 à 5,
dans lequel la base (3616) comprend une saillie axiale (3621) et le couvercle (3624) comprend une encoche axiale (3626), l'encoche axiale (3626) étant configurée pour recevoir la saillie axiale (3621) afin de maintenir l'interstice entre la base (3616) et le couvercle (3624) lorsque l'ensemble d'évent (3606) est positionné à l'intérieur du trou de réception d'ensemble d'évent (3614).

7. Ensemble de raccord de conduit et d'évent (3605) selon l'une des revendications 1 à 6,
dans lequel la base (3616) comprend une pluralité de rayons (3617) formant une pluralité de trous de base (3618) pour permettre aux gaz expirés de passer à travers la base (3616).

8. Ensemble de raccord de conduit et d'évent (3605) selon l'une des revendications 1 à 7,
dans lequel le volet (3622) est configuré pour reposer sur la base (3616) jusqu'à ce qu'une pression suffisante à l'intérieur du raccord de conduit (3610) provoque le soulèvement du volet (3622) de la base (3616).

9. Ensemble de raccord de conduit et d'évent (3605) selon l'une des revendications 1 à 8,
dans lequel le couvercle (3624) comprend une pluralité de trous de couvercle (3627) pour permettre aux gaz expirés de passer à travers le couvercle (3624) vers l'environnement.

10. Ensemble de raccord de conduit et d'évent (3605) selon la revendication 9,
dans lequel le couvercle (3624) comprend une portion annulaire (3629) et une portion centrale (3628), les trous de couvercle (3627) étant positionnés entre la portion annulaire (3629) et la portion centrale (3628).

11. Ensemble de raccord de conduit et d'évent (3605) selon la revendication 10,
dans lequel le volet (3622) est configuré pour bloquer complètement les trous de couvercle (3627) lorsque le volet entre en contact avec la portion annulaire (3629) et la portion centrale (3628),
le volet (3622) est configuré pour permettre aux gaz expirés de passer à travers le couvercle (3624) vers l'environnement lorsque le volet est en contact uniquement avec la portion annulaire (3629) ou la portion centrale (3628), et
la portion annulaire (3629) et la portion centrale (3628) sont décalées l'une par rapport à l'autre dans une direction axiale du couvercle (3624).

12. Ensemble de raccord de conduit et d'évent (3605) selon l'une des revendications 1 à 11,
dans lequel le volet (3622) est fabriqué à partir d'un matériau flexible,
le matériau flexible est du silicone, et
la base (3616) et le couvercle (3624) sont tous deux fabriqués à partir d'un matériau rigide.

13. Ensemble de raccord de conduit et d'évent (3605) selon l'une des revendications 1 à 12,
dans lequel l'extrémité d'entrée (3612) comprend un raccord à baïonnette (3613) de telle sorte que lorsque le circuit d'air (4170) est relié au raccord de conduit (3610), le circuit d'air (4170) et le raccord de conduit (3610) ne peuvent pas tourner l'un par rapport à l'autre.

14. Interface patient (3000) comprenant :
une chambre de distribution pouvant être pressurisée à une pression thérapeutique d'au moins 4 cmH2O au-dessus de la pression d'air ambiant par un flux d'air à la pression thérapeutique pour la respiration d'un patient,
la chambre de distribution (3200) comprenant en outre deux raccords de chambre de distribution (3202), chacun des raccords de chambre de distribution (3202) étant positionné sur un côté latéral correspondant de la chambre de distribution (3200) et étant configuré pour recevoir le flux d'air à la pression thérapeutique ;
une structure formant joint d'étanchéité (3100) reliée à la chambre de distribution (3200), la structure formant joint d'étanchéité (3100) étant conçue et agencée pour former un joint d'étanchéité avec une région du visage du patient qui entoure au moins partiellement une entrée des voies respiratoires du patient, la structure formant joint d'étanchéité (3100) présentant un trou pour délivrer le flux d'air à la pression thérapeutique au moins aux narines du patient pendant l'utilisation, la structure formant joint d'étanchéité (3100) étant conçue et agencée pour maintenir la pression thérapeutique à l'intérieur de la chambre de distribution (3200) tout au long du cycle respiratoire du patient pendant l'utilisation ;
une structure de positionnement et de stabilisation (3300) configurée pour maintenir la structure formant joint d'étanchéité (3100) dans une position thérapeutiquement efficace sur la tête du patient, la structure de positionnement et de stabilisation (3300) comprenant en outre deux conduits (3350) et une section centrale (3305) entre les conduits (3350), la section centrale (3305) présentant un trou de réception de raccord de conduit (3306), chacun des conduits (3350) étant configuré pour être positionné sur un côté latéral correspondant de la tête du patient pendant l'utilisation et étant configuré pour être relié à un raccord correspondant parmi les raccords de chambre de distribution (3202) ; et
l'ensemble de raccord de conduit et d'évent (3605) selon l'une des revendications 1 à 13, relié à la section centrale (3305) au niveau du trou de réception de raccord de conduit (3306),
dans laquelle l'interface patient (3000) est configurée pour laisser la bouche du patient découverte, ou la structure formant joint d'étanchéité (3100) est configurée pour assurer l'étanchéité autour du nez et de la bouche du patient, et l'interface patient (3000) est configurée pour permettre au patient de respirer l'air ambiant en l'absence de flux d'air à la pression thérapeutique.

15. Interface patient (3000) selon la revendication 14,
dans laquelle l'ensemble de raccord de conduit et d'évent (3605) est relié de manière rotative et amovible à la section centrale (3305), et
la chambre de distribution (3200) comprend un évent passif (3400) configuré pour permettre aux gaz expirés de passer vers l'environnement indépendamment de la pression thérapeutique et tout au long du cycle respiratoire du patient.
